# EUROPEAN PATENT SPECIFICATION

(11) **EP 0 440 362 B1**
(45) Date of publication and mention of the grant of the patent: **03.04.1996**
(21) Application number: 91300481.8
(22) Date of filing: 22.01.1991
(51) Int. Cl.: C07C 255/33, C07C 255/37, C07C 253/00, C07C 251/40, C07C 249/08, A61K 7/46, C11B 9/00, C11D 13/08

(54) **2,2-Dimethyl-1-nitrilo or 2,2-dimethyl-1-hydroxylamino-3-(alkyl phenyl)substituted propanes or 2-methyl-1-nitrilo-or 2-methyl-1-hydroxylamino-3-(methoxyphenyl) propane, organoleptic uses thereof and processes for preparing same**
2-2-Dimethyl-1-nitrilo oder 2,2-Dimethyl-1-hydroxylamino-3-(alkylphenyl)-substituierte Propane oder 2-Methyl-1-nitrilo- oder 2-Methyl-1-hydroxylamino-3-(methoxyphenyl)propan, ihre organoleptische Verwendung und Verfahren zu ihrer Herstellung
Propanes substitués par 2-2-diméthyl-1-cyano ou 2,2-diméthyl-1-hydroxylamino-3-(alkylphényl) ou 2-méthyl-1-cyano- ou 2-méthyl-1-hydroxylamino-3-(méthoxyphényl)propane, leur utilisation organoleptique et leur procédé de préparation

(30) Priority: 02.02.1990 US 474545; 25.04.1990 US 514514
(43) Date of publication of application: 07.08.1991
(73) Proprietor: INTERNATIONAL FLAVORS & FRAGRANCES INC., New York New York 10019 (US)
(72) Inventor: Narula, Anubhav P.S., Monmouth, New Jersey 07730 (US); de Virgilio, John J., Monmouth, New Jersey 07728 (US); Fox, Eleanor, New York, New York 10027 (US)
(74) Representative: Brown, John David

(56) References cited:
- DE-A- 3 139 358
- DE-A- 3 639 158
- CHEMICAL ABSTRACT, vol. 80, no. 19, May 13, 1974, Columbus, Ohio, USA A.D. GREBENYUK et al. "Reaction of methacrylonitrile with anisole in the presence of aluminum chloride" page 383, abstract-No. 108 143q &Zh. Org. Khim 1974,10(1), 88-91

## Description

The present invention relates to a substituted propane defined according to the structure: 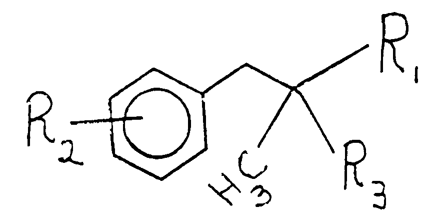
wherein R₁ is cyanide having the structure:

⁅C≡N]

or hydroxyimino methyl having the structure: 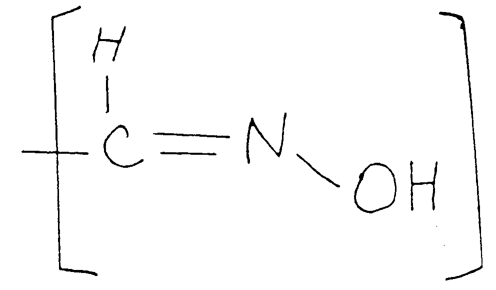
R₂ is ethyl or methoxy and R₃ is hydrogen or methyl, but wherein R₃ is not hydrogen when R₂ is p-methoxy
Chemical Abstracts, Vol 80, No. 19, 13 May 1974, 108143q, Columbus, Ohio, U.S.A. (A.D. GREBENYUK ET AL) discloses p - MeOC₆H₄CH₂CHMeCN and o - MeOC₆H₄CH₂CHMeCN
D E - A - 3639158 (BASF AG) discloses the following compound: 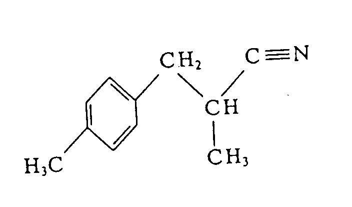
for use in augmenting the aroma of perfume compositions and detergents.

According to the present invention, there is provided a substituted propane defined according to the structure: 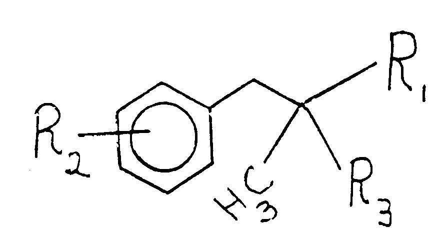
wherein R₁ is cyanide having the structure:

⁅C≡N]

or hydroxyimino methyl having the structure: 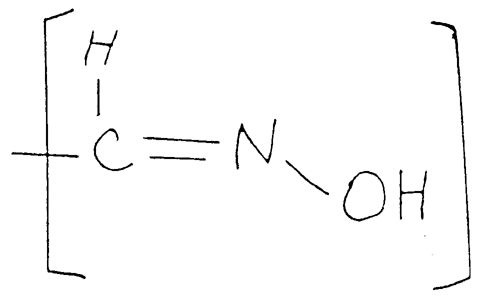
R₂ is ethyl or methoxy and R₃ is hydrogen or methyl, but wherein R₃ is not hydrogen when R₂ is p-methoxy.

According to another aspect of the present invention, there is provided at least one 2,2-dimethyl-1-nitrilo or hydroxyimino-3-(alkyl phenyl)-substituted propane defined according to the structure: 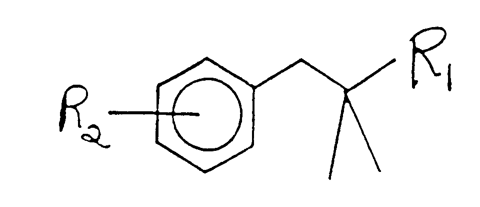
wherein R₁ is a nitrogen-containing moiety selected from the group consisting of:
(i) cyanide having the structure:

   ⁅C≡N]

   and
(ii) hydroxyimino methyl having the structure:

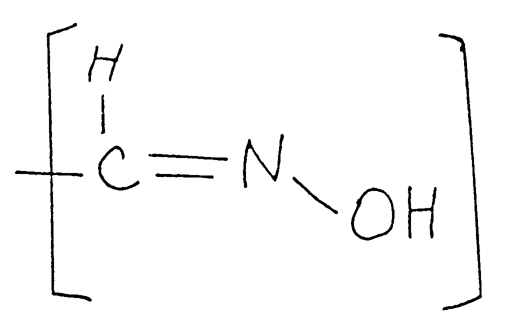

and wherein R₂ is C₁-C₅ lower alkyl:
According to a further aspect of the present invention, there is provided a mixture including a mixture of 2,2-dimethyl-1-nitrilo or hydroxyimino-3-(alkyl phenyl)-substituted propanes having the structure: 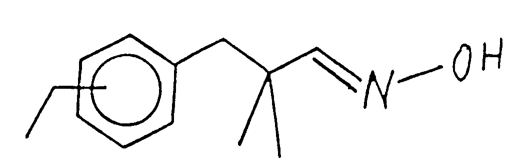
prepared according to the process comprising the steps of reacting a mixture of compounds having the structure: 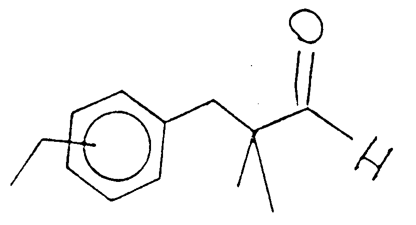
with a hydroxylamine salt having the structure: 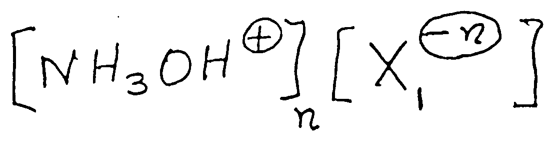
in the presence of base wherein n is an integer of from 1 up to 3 and x₁⁻ⁿ is an anion having a valence of -n.

According to another aspect of the present invention, there is provided a mixture of compounds including a mixture of 2,2-dimethyl-1-nitrilo or hydroxyimino-3-(alkyl phenyl)-substituted propanes having the structure: 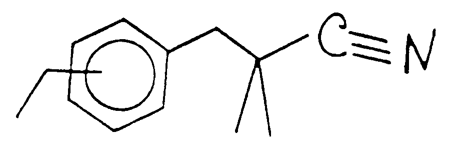
prepared according to the process of:
(i) first reacting a mixture of compounds having the structure: 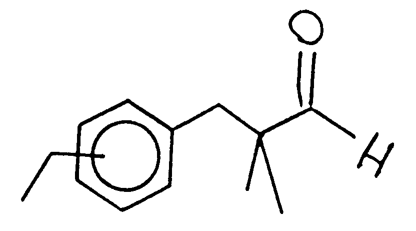 with a hydroxylamine salt having the structure: 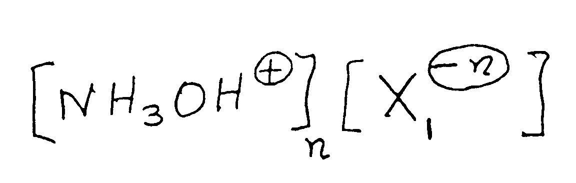 in the presence of base whereby a mixture of compounds containing the mixture defined according to the structure: 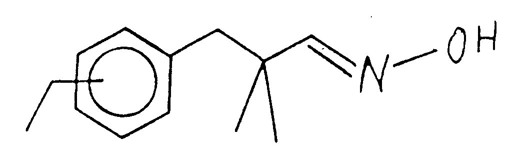 is obtained;
and then
(ii) reacting the mixture containing the mixture of compounds having the structure: 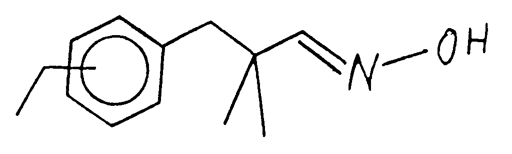 with a dehydrating agent to produce a mixture of compounds containing the mixture defined according to the structure:

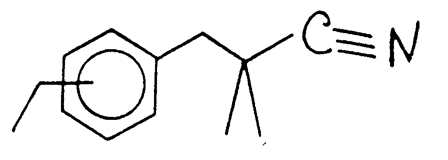

According to a further aspect of the present invention, there is provided a 2-methyl-1-hydroxyimino-3-(methoxyphenyl)-substituted propane having the structure: 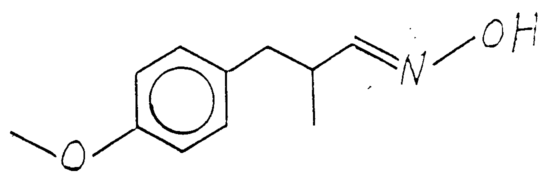
According to another aspect of the present invention, there is provided a mixture of nitriles having the structures : 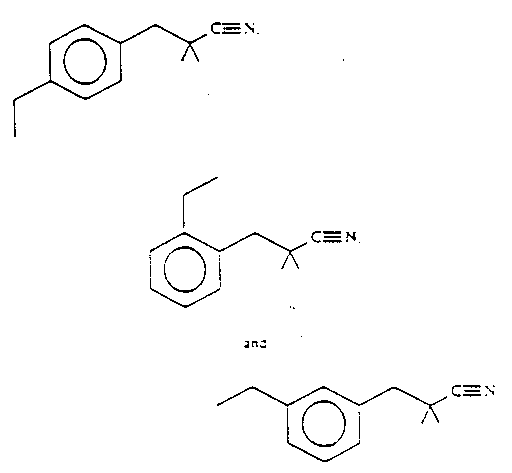
According to a further aspect of the present invention, there is provided a mixture of oximes having the structures : 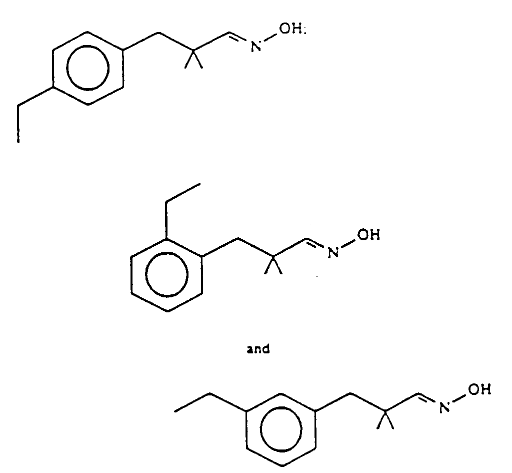
The substituted propanes of our invention produced according to the processes of our invention are capable of augmenting or enhancing sweet, herbal, green, anisic, floral, jasmine, vanilla and animalic aromas with floral, dry, citrus and ozoney undertones and anisic topnotes in perfume compositiions, colognes and perfumed articles including soaps, bleaches, anionic, cationic, nonionic or zwitterionic detergents, fabric softener articles and perfumed articles.

The substituted propanes of our invention may be produced using as starting materials aldehydes defined according to the generic structure: 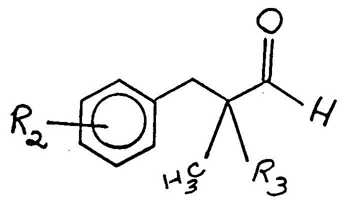
(which structure represents "ortho", "meta" or "para" isomers); and wherein R₂ is ethyl or methoxy and R₃ is hydrogen or methyl, but wherein R₃ is not hydrogen when R₂ is p-methoxy

The mixture of aldehydes defined according to the generic structure: 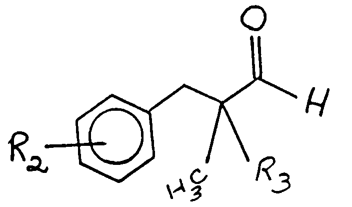
may be reacted with a hydroxylamine salt defined according to the structure: 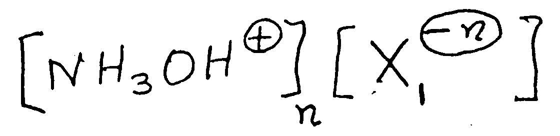
in the presence of base according to the reaction: 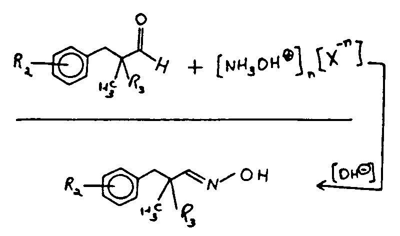
The hydroxylamine salt represented by the structure: 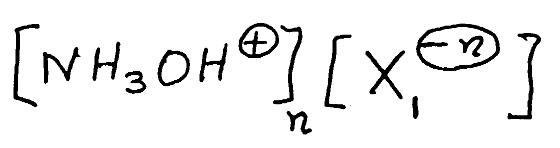
may be a sulfate, bisulfate, phosphate, diacid phosphate, monoacid phosphate or sulfite defined, respectively, according to the formulae:

SO₄⁼ ; HSO₄⁻ : PO₄^{≡} ; H₂PO₄⁻ : HPO₄⁼ and SO₃⁼

wherein X₁ represents the anion such as sulfate, bisulfate, chloride, bromide, phosphate and the like. The letter n is an integer of from 1 up to 3 and the term "-n" represents the valence of the anion.

The resulting mixture of compounds defined according to the generic structure: 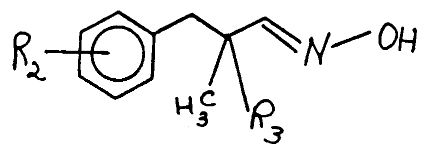
may be fractionally distilled from the reaction mass and used "as-is" for its organoleptic properties.

In the alternative, the resulting mixture of compounds having the structure: 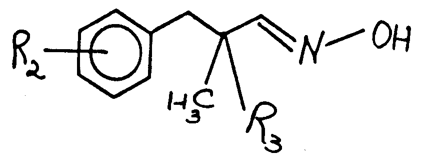
may be further reacted with a dehydrating agent according to the reaction: 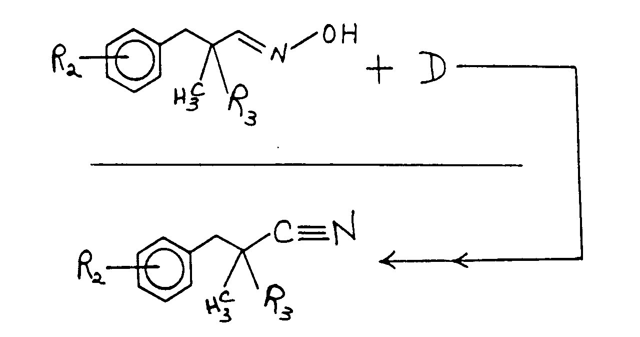
to form the mixture of compounds defined according to the generic structure: 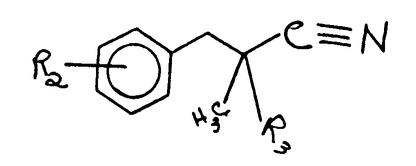
wherein R₂ and R₃ are defined supra. Examples of dehydrating agents useful in this reaction are as follows:
(i) mixtures of copper sulfate and acetic anhydride;
(ii) phosphorous oxychloride;
(iii) phosphorous trichloride;
(iv) phosphorous pentoxide;
(v) thionyl chloride; and
(vi) acetyl chloride.

In the alternative, a substituted benzylhalide may be reacted with an alkyl cyanide directly in the presence of a basic catalyst such as sodamide, potassium hydroxide, diethylamine and the like according to the reaction: 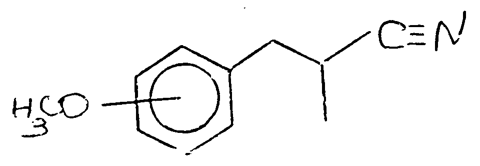
With reference to the reaction: 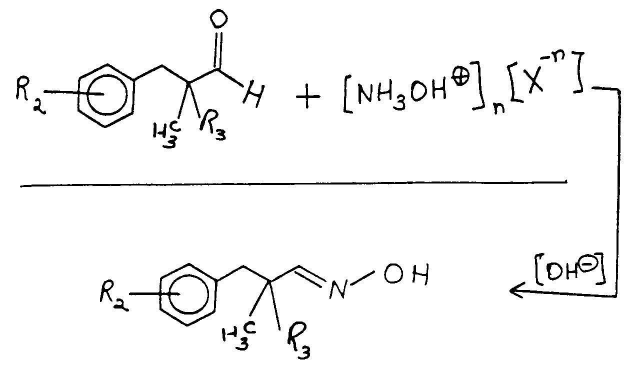
the reaction is carried out at a temperature in the range of from about 50 up to about 70°C. At such a temperature, the reaction time is approximately 1-1.5 hours.

The mole ratio hydroxylamine salt having the structure: 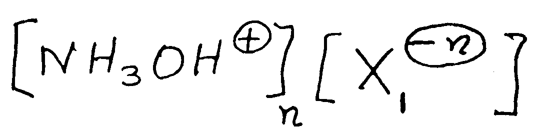
to aldehyde having the structure: 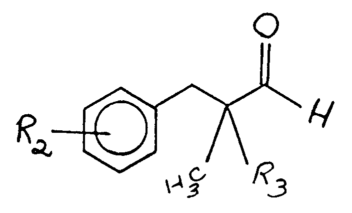
may vary from about 0.5:1.5 up to 1.5:0.5 with a preferred mole ratio of aldehyde:hydroxylamine salt being about 1.5:1.

At the end of the reaction, the reaction mass is washed with a material such as saturated sodium chloride; solvent extracted; filtered and fractionally distilled.

With reference to the reaction: 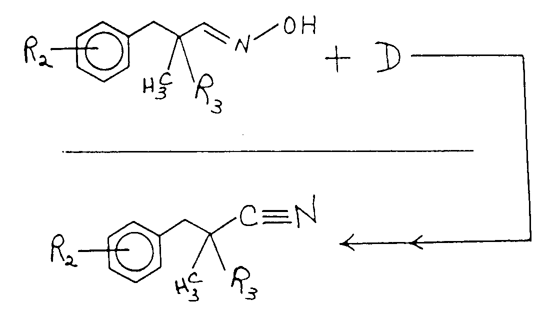
when using the preferred dehydration reagent, the mixture of acetic anhydride and copper sulfate, the reaction preferably takes place at a temperature in the range of from about 100°C up to about 120°C over a period of 1.5-2 hours. The preferred mole ratio of acetic anhydride:hydroxyimino mixture defined according to the structure: 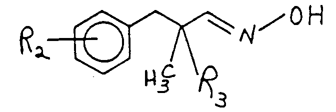
may vary from about 1:1 up to 1.5:1 acetic anhydride:hydroxyimino derivative having the structure: 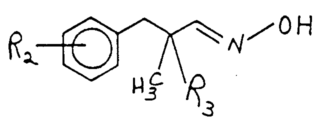
with the quantity of copper sulfate being a catalytic quantity (in an amount of from about 0.2 up to about 0.8% by weight of the reaction mass).

At the end of the reaction, the reaction mass is quenched by adding water dropwise. The resulting product is then washed with weak base, e.g., sodium bicarbonate followed by saturated sodium chloride. The resulting product is then filtered and vacuum distilled to yield product which is organoleptically useful, e.g., in perfumes, perfumed articles and stable perfumed bleaches.

The following tables set forth exemplary reaction products and their organoleptic properties:

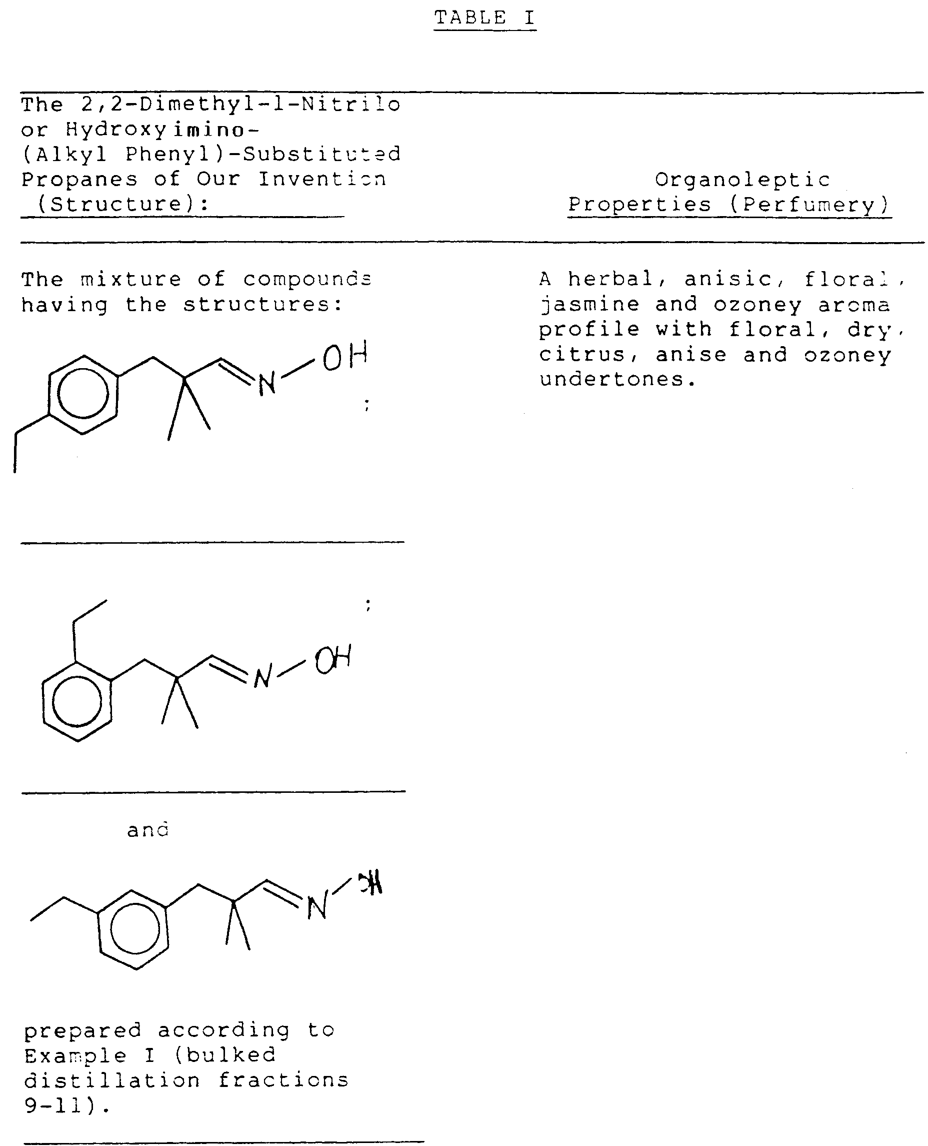

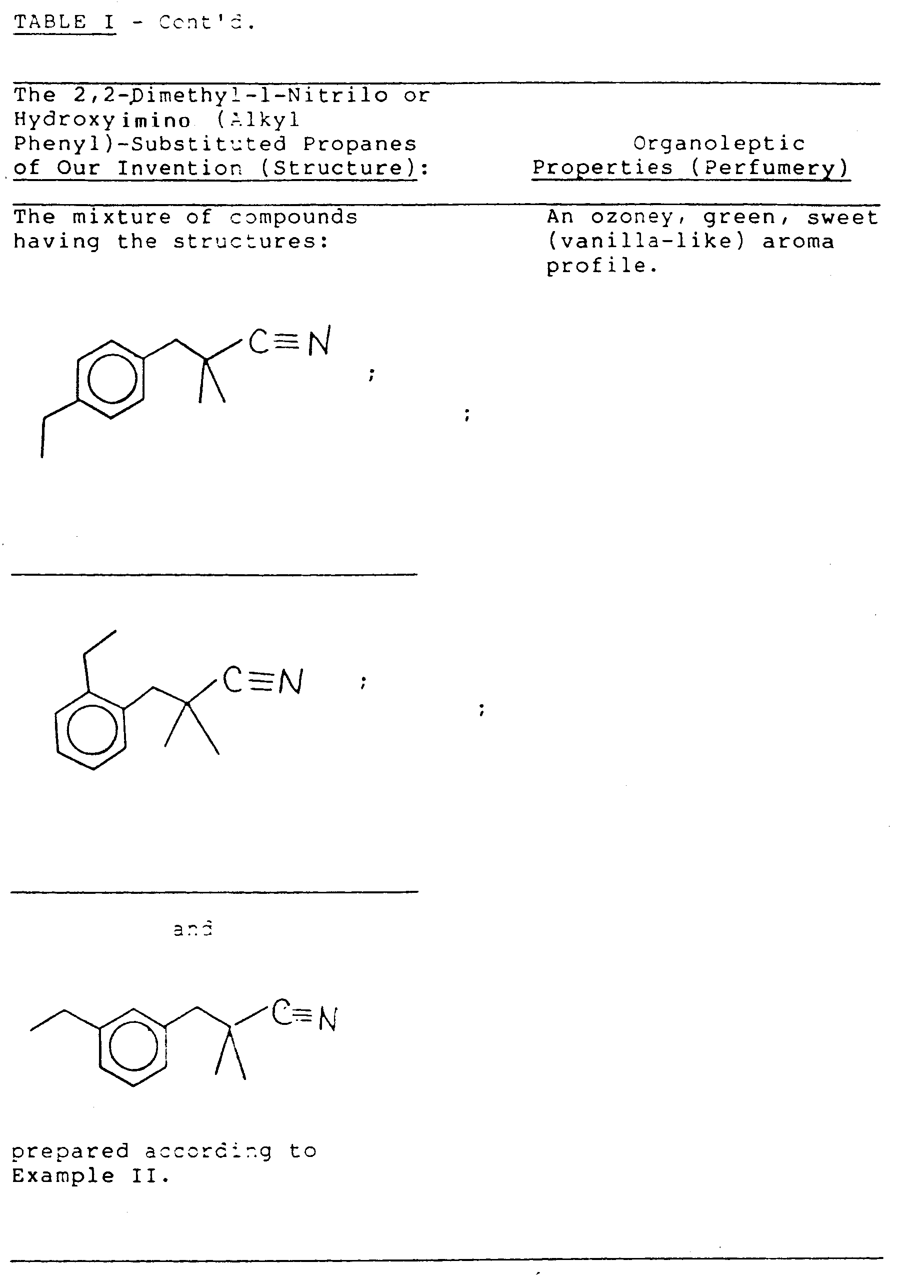

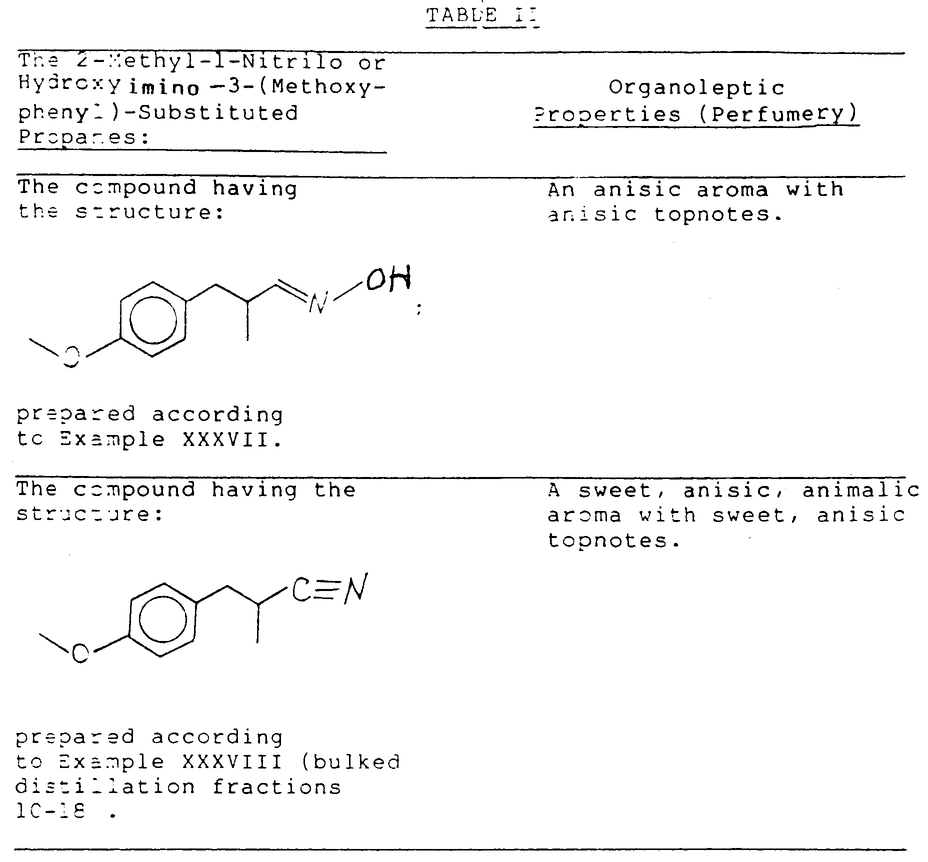

The substituted propanes of our invention and one or more auxiliary perfume ingredients including, for example, hydrocarbons, alcohols, ketones, aldehydes, nitriles (other than the nitriles of our invention), esters, lactones, ethers, hydrocarbons, synthetic essential oils and natural essential oils may be admixed so that the combined odors of the individual components produce a pleasant and desired fragrance, particularly and preferably in the "wintergreen/pine fragrance" area. Such perfume compositions usually contain (a) the main note or the "bouquet" or foundation stone of the composition; (b) modifiers which round off and accompany the main note; (c) fixatives which include odorous substances which lend a particular note to the perfume throughout all stages of evaporation and substances which retard evaporation; and (d) topnotes which are usually low boiling fresh smelling materials.

In perfume compositions, it is the individual components which contribute to their particular olfactory characteristics, however the overall sensory effect of the perfume composition will be at least the sum total of the effects of each of the ingredients. Thus, one or more of the substituted propanes of our invention can be used to alter, modify or enhance the aroma characteristics of a perfume composition, for example, by utilizing or moderating the olfactory reaction contributed by another ingredient in the composition.

The amount of substituted propanes of our invention which will be effective in perfume compositions as well as in perfumed articles and colognes depends upon many factors, including the other ingredients, their amounts and the effects which are desired. It has been found that perfume compositions containing as little as 0.005% of one or more of the substituted propanes of our invention or even less (e.g., 0.002%) can be used to impart herbal, green, anisic, floral, jasmine and sweet (vanilla-like) aromas with floral, dry, citrus, anise and ozoney undertones to soaps, cosmetics, detergents (including anionic, cationic, nonionic or zwitterionic solid or liquid detergents) or other products. The amount employed can range up to 70% of the fragrance components and will depend upon considerations of cost, nature of the end product, the effect desired on the finished product and the particular fragrance sought.

The substituted propanes of our invention are useful (taken alone or together with other ingredients in perfume compositions), in detergents and soaps, space odorants and deodorants, perfumes, colognes, toilet water, bath preparations such as lacquers, brilliantines, pomades and shampoos; cosmetic preparations such as creams, deodorants, hand lotions and face powders and the like. As little as 0.7% of the substituted propanes of our invention will suffice to impart an intense and substantive herbal, sweet, green, anisic, floral, jasmine, (vanilla-like) and animalic aroma with floral, dry, citrus, anise and ozoney undertones and anisic topnotes to wintergreen/pine perfume formulations. Generally, no more than 5% of the substituted propanes of our invention based on the ultimate end product is required to be used "as is" or in the perfume composition.

Furthermore, as little as 0.25% of one or more of the substituted propanes of our invention will suffice to impart such aroma to perfumed articles per se, whether in the presence of other perfume materials or whether used by themselves. Thus, the range of use of the substituted propanes of our invention in perfumed articles may vary from about 0.25% up to about 5% by weight based on the total weight of the perfumed article.

In addition, the perfume composition or fragrance composition of our invention can contain a vehicle, or carrier for the substituted propanes of our invention. The vehicle can be a liquid such as a non-toxic alcohol, e.g., ethanol, a non-toxic glycol, e.g., propylene glycol or the like. The carrier can also be an absorbent solid, such as a gum (e.g., gum arabic) or components for encapsulating the composition by means of coacervation (such as gelatin).

It will thus be apparent that the substituted propanes of our invention can be utilized to alter, modify or enhance the aroma of perfume compositions, colognes or perfumed articles.

Furthermore, several processes may be used in order to produce a thickened, highly viscous hypochlorite bleaching or sterilizing solution whereby the desired aroma profiles are imparted to the articles treated with said hypochlorite solutions.

Thus, for example, the substituted propanes of our invention may be premixed with the diphenyl oxide derivative or diphenyl oxide derivative-amine oxide solubilizer-stabilizer (having the structures, respectively: 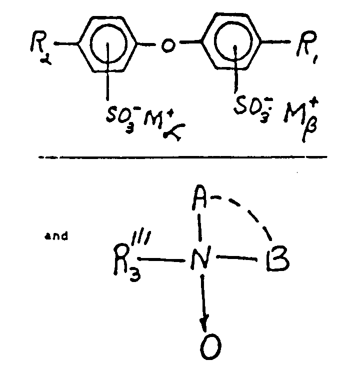
and the resulting substituted propanes of our invention or a substituted propane premix is then mixed with the hypochlorite bleaching or sterilizing solution with stirring. Immediately after such addition, an aqueous alkali metal hydroxide solution is added to the mixture to bring the pH to the range of 11-14.0. A pH of less than 11 is not desired since it is difficult to achieve a single phase stable system at low pH's. A pH higher than 14.0 will also create a system which (1) is unnecessarily corrosive; (2) will narrow the range of perfume oils useable (in conjunction with the substituted propanes) of the system and (3) will limit the particular ingredients useable in such perfume oils in conjunction with the substituted propanes. On the other hand, if for example, the substituted propanes are used alone or further in combination with (i) diisoamylene epoxides; (ii) diisoamylenes as described in application for United States Letters Patents, Serial No. 188,576 filed on October 9, 1980; now U.S. Letters Patent No. 4,303,555 issued on December 1, 1981 or (iii) acyl diisoamylene derivatives described in application for United States Letters Patent, Serial No. 184,132 filed on September 4, 1980, now U.S. Letters Patent No. 4,321,255 issued on March 23, 1982 and/or (iv) ketal derivatives of acyl diisoamylene derivatives described in application for United States Letters Patent, Serial No. 212,993 filed on December 4, 1980, now U.S. Letters Patent No. 4,315,952 issued on February 16, 1982, a pH of about 14.0 and even slightly higher (e.g., 14.1) is acceptable.

The aqueous alkali metal hydroxide can be added to the aqueous alkali metal hypochlorite solution before adding the diphenyl oxide derivatives (taken alone or in conjunction with the amine oxide) or the substituted propanes or mixture of substituted propanes with other materials such as diisoamylene epoxides. Indeed, the ingredients: the substituted propanes; the alkali metal hydroxide and the diphenyl oxide derivative or diphenyl oxide derivative-amine oxide composition (having the structures, respectively 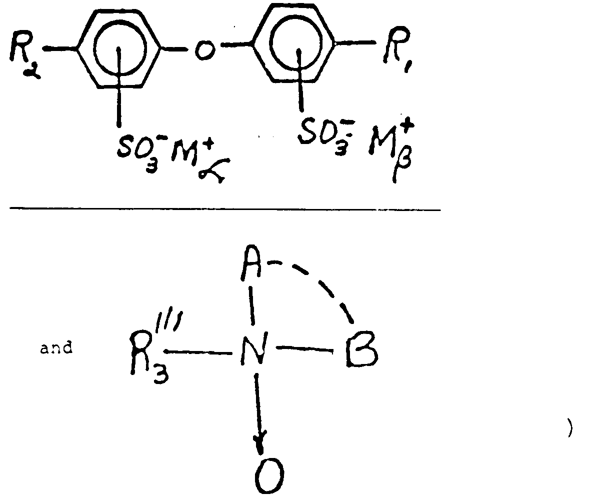
may be added or admixed in any order which is convenient to the formulator.

The alkali metal hypochlorites preferred in the practice of our invention are: sodium hypochlorite, potassium hypochlorite and lithium hypochlorite or mixtures of same. The alkali metal hypochlorites preferred in the practice of this invention are: lithium hydroxide, potassium hydroxide and sodium hydroxide, or, if desired, mixtures of such hydroxides.

The temperature at which the composition of our invention remains both substantially stable and commercially useful for the purposes set forth herein (that is, remains as a clear single aqueous or gel phase) and retains (1) the desired properties inherent in the known bleaching and sterilizing uses of aqueous alkali metal hypochlorite liquid or gel solutions, and (2) the properties imparted thereto as a result of the use of the substituted propanes which impart to articles previously subjected to the aqueous alkali metal hypochlorite gel or liquid solutions a desired aroma profile, varies from approximately -6.67°C (20°F) up to approximately 48.89°C (120°F). At temperatures below -6.67°C (20°F) a two-phase system usually occurs and at temperatures higher than 48.89°C (120°F) the bleaching or sterilizing efficiency of the compositions of our invention is diminished at an excessive rate.

When it is desired to (1) initially form the C₁₀-C₁₂ straight chain or branched chain diphenyl oxide alkali metal sulfonate or diphenyl oxide derivative-amine oxide premix; (2) then combine the resulting premix with an alkali metal hypochlorite solution; (3) then add the thickening agent and then (4) adjust the pH of the resulting solution to the range of 11-14.0, then the temperature of mixing ranges may be as follows:

| | | |
|---|---|---|
| (a) | Formation of the diphenyl oxide derivative or diphenyl oxide amine oxide-2,2-dimethyl-1-nitrilo or 2,2-dimethyl-1-hydroxylamino-3-(alkyl phenyl)-substituted propane premix | -6.67°C - 65.56°C (20°F) -(150°F) |
| (b) | Mixing the premix with aqueous alkali metal hypochlorite solution followed by thickening agent | -6.67°C - 48.89°C (20°F) -(120°F) |
| (c) | Adjustment of pH of the solution to the range of 11-14.0 using aqueous alkali metal hydroxide solution | -6.67°C - 48.89°C (20°F) -(120°F.) |

In any event, whenever a mixing unit operation involves the aqueous alkali metal hypochlorite solution, the temperature of mixing is limited to the range of -6.67°C - 48.89°C (20°F-120°F). Where the mixing unit operation involves the mixing of substituted propanes, the upper bound of the temperature range is limited by the stability of the substituted propanes or other perfume ingredient mixed with the substituted propanes useable in the practice of our invention; and the lower bound of said temperature range is limited by the least temperature where a single liquid phase or gel phase including the substituted propanes or other ingredients admixed therewith will exist. Where a unit mixing operation of the process of our invention involves the mixing of one or more diphenyl oxide derivatives having the generic structure: 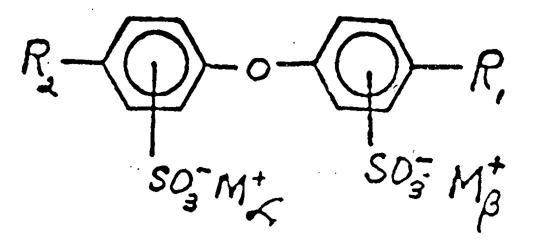
taken alone or taken together with one or more amine oxides having the generic structure: 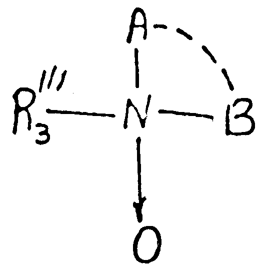
with other materials, the upper bound of the temperature range is the decomposition point of any one of the diphenyl oxide derivatives or amine oxide components and the lower bound is the least temperature where a single liquid phase or gel phase, including the diphenyl oxide derivatives or diphenyl oxide-amine oxide mixture will exist.

Preferred diphenyl oxide derivative compositions from a practical standpoint useful in the practice of our invention are compounds having the structure: 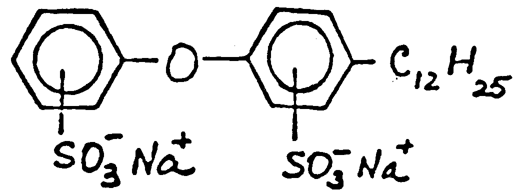
where the C₁₂H₂₅ moiety represents one or a series of different branched chains; compounds defined according to the structure: 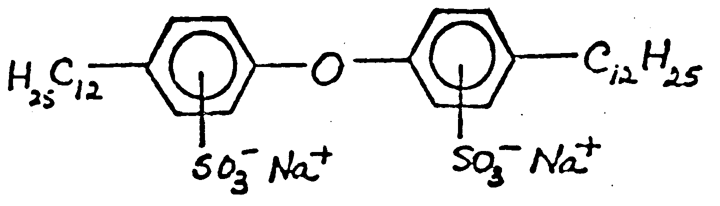
where the C₁₂H₂₅ moiety represents one or a series of different branched chains: compounds defined according to the structure: 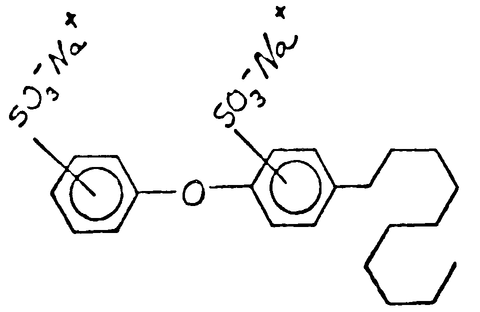
and compounds defined according to the structure: 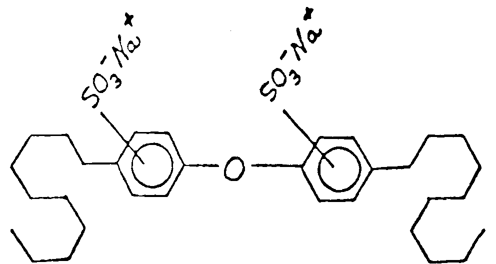
otherwise known as DOWFAX® 2A1 in the case where one or R₁ or R₂ represents branched C₁₂H₂₅ alkyl chains and the other of R₁ or R₂ represents hydrogen, or DOWFAX®3B2 in the case where one of R₁ or R₂ represents straight C₁₀ alkyl chain and the other of R₁ or R₂ represents hydrogen (DOWFAX® being a registered trademark of the Dow Chemical Company of Midland, Michigan).

When used in conjunction with the diphenyl oxide derivatives preferred amine oxide compositions, from a practical standpoint, useful in the practice of our invention are the commercially available (1) dimethyl "cocoamine" oxide (a mixture which is dominated by dimethyl-C₁₂-C₁₆ straight chain alkyl amine oxides; more particularly a mixture containing approximately 70% C₁₂ straight chain alkyl amine oxides, approximately 25% of straight chain C₁₄ alkyl amine oxides and approximately 4% straight chain C₁₆ alkyl amine oxides) and (2) N-cocomorpholine oxide, a mixture dominated by straight chain C₁₂-C₁₆ alkyl morpholine oxides (specifically containing approximately 70% straight chain C₁₂ alkyl morpholine oxide, approximately 25% straight chain C₁₄ alkyl morpholine oxide, and approximately 4% straight chain C₁₆ alkyl morpholine oxide). Commercial examples of such amine oxide compositions are: AROMOX® DMC-W and AROMOX® DMMC-W which are 30% aqueous dimethyl cocoamine oxide solutions and AROMOX® NCMDW which is a 40% aqueous N-cocomorpholine oxide solution each of which is produced by the Armac Division of AKZO of Chicago, Illinois. These materials are described in Brochure 68011, published by Armour Industrial Chemicals, P.O. Box 1805, Chicago, Illinois 60690. Other preferred amine oxides are n-undecyl dimethyl amine oxide and n-tridecyl dimethyl amine oxide.

The percentage of hypochlorite ion in the compositions of our invention may vary from about 1% up to about 20% for the desired effects to be produced using the diphenyl oxide substituted propanes covered by our invention. The usual percent of alkali metal hypochlorite in solution is about 5%, the percentage of sodium hypochlorite in such mixtures as CLOROX® the registered trademark of the Clorox Corporation.

The perfume oil used in conjunction with the substituted propanes which, in turn, is used in conjunction with the aqueous alkali metal hypochlorite solution must have such properties as to be able (1) to be compatible with the substituted propanes; (2) to impart to the resulting or "aqueous alkali metal hypochlorite" liquid or gel solution a pleasant aroma which harmonizes with the aroma of the substituted propanes; (3) to effect a substantial diminution or elimination of the disagreeable "hypochlorite" aroma which is imparted to surfaces (e.g., bleached laundry or the hands of the user which are in direct contact with the hypochlorite solution) on which known aqueous alkali metal hypochlorite solutions have been used; and (4) to impart to the surfaces with which such aqueous alkali metal hypochlorite solutions are in contact, a pleasant long lasting stable aroma. Examples of ingredients compatible with substituted propanes and suitable for the aforementioned purposes, that is, useable in conjunction with the disclosed hypochlorites, amine oxide derivatives and diphenyl oxide derivatives are as follows:
1. Cedryl alkyl ethers covered by U.S. Patent No. 3,373,208 such as cedryl methyl ether;
2. Isochroman musks covered by U.S. Patent No. 3,360,530 and 3,591,528 such as 6-oxa-1,1,3,3,8-pentamethyl-2,3,5,6,7,8-hexahydro-1H-benz(f)indene;
3. Polycyclic ethers covered by U.S. Patent No. 3,281,432, such as octahydro-1,3a,6-trimethyl-1H-1,6a,ethanopentaleno-(1,2-C) furan;
4. Polycyclic ketones such as hexahydro-1,1,5,5-tetramethyl-2H-2,4a-methanonaphthalen-8-(5H)one;
5. Diisoamylenes described according to application for United States Letters Patent, Serial No. 188,576 filed on September 18, 1980;
6. Acyl diisoamylene derivatives described according to application for United States Letters Patent, Serial No. 184,132 filed on September 4, 1980, and ketal derivatives thereof described according to application for United States Letters Patent, Serial No. 212,993 filed on December 4, 1980; and
7. Diisoamylene epoxide derivatives prepared according to application for United States Letters Patent, Serial No. 231,773 filed on February 27, 1981.

It will be understood that a number of materials cannot be used in conjunction with the substituted propanes of our invention, such as: 1,5,9-trimethyl-12-acetyl cyclo-dodecatriene-1,5,8 and 1,5,9-trimethyl-12-cyclododecadiene-1,8 covered by British Patent No. 1,204,409, because they are easily oxidized in hypochloride bleach.

A basic feature of our invention concerns the fact that the only detergent group needed or desirable in the composition containing the substituted propanes of our invention is the class of diphenyl oxide derivatives defined according to the structure: 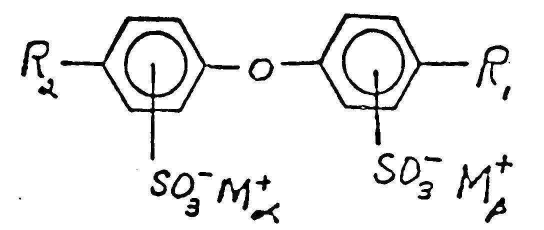
wherein R₁, R₂, M_{α} and M_{β} are defined, supra, taken alone or in conjunction with the class of morpholino and/or dimethyl C₁₁-C₁₃ straight chain alkyl amine oxides defined according to the structure: 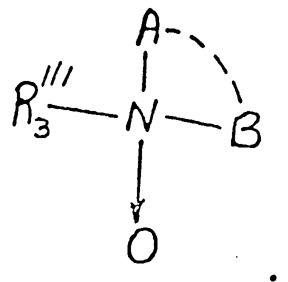
More specifically, such detergents as sodium decyl ether sulfate, sodium myristyl ether sulfate, sodium lauryl ether sulfate and lithium lauryl ether sulfate are neither desired nor are they required. Furthermore, the well known hydrotropes employed in prior art compositions such as the well known family of clarifying agents comprising the alkali metal or alkali earth metal salts of mono- and polyalkylated benzene or naphthalene sulfonates such as sodium xylene or magnesium toluene sulfonate are again neither desired nor are they required in the compositions intended to be encompassed by the instant invention.

Another basic feature of our invention concerns the fact that when it is desired to have a gel phase composition, thickener agents may be employed in conjunction with the system; hypochlorite bleach-substituted propanes-diphenyl oxide derivative or diphenyl oxide derivative-amine oxide derivative (having the general structure 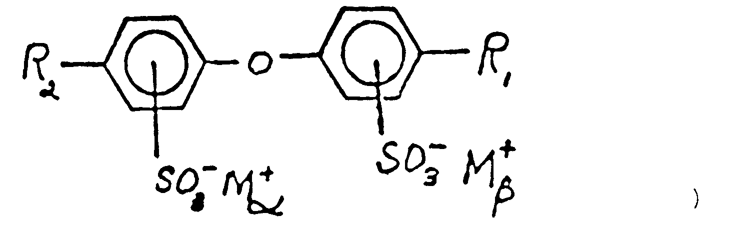
and having the structure:. 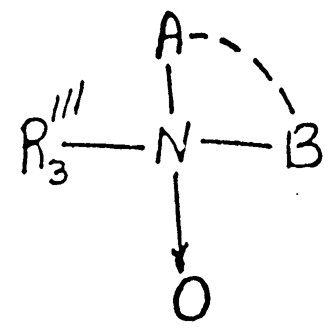
Still another basic feature of our invention concerns the fact that the gel phase compositions including thickener agents are employed with the "premix" system: substituted propanes-diphenyl oxide derivative or diphenyl oxide derivative-amine oxide.

Thus, sodium palmitate, sodium stearate, sodium laurate, potassium palmitate, potassium stearate, potassium laurate, lithium palmitate, lithium stearate and/or lithium laurate or combinations of the foregoing may be added to the compositions of matter containing the substituted propanes of our invention to provide a thickened gel-type hypochlorite bleach which, in addition to being in a semi-solid state, is obviously, advantageously and unexpectedly stable over long periods of time. Percentages of thickening agents such as sodium palmitate, sodium stearate, sodium laurate, potassium laurate, lithium palmitate, lithium stearate or lithium laurate or combinations of these which may be used in the thickened compositions containing the substituted propanes of our invention are from 1% by weight up to 12% by weight of the thickener based on the overall weight of hypochlorite bleach-diphenyl oxide derivative (or diphenyl oxide derivative-amine oxide)-substituted propanes composition. When it is merely desired to have a thickened "premix" the percentage of thickening agent may vary from about 5% up to about 40% by weight of thickener based on overall weight of "premix".

The following Examples I, II and XXXVII serve to illustrate processes for producing the substituted propanes of our invention. Examples III-XXXVI and Examples following XXXVIII, in general, serve to illustrate organoleptic utilities of the substituted propanes of our invention, and of the substituted propane prepared according to Example XXXVIII.

In general, the following examples serve to illustrate specific embodiments of our invention. It will be understood that these examples are illustrative and that the invention is to be considered restricted thereto only as indicated in the appended claims.

All parts and percentages given herewith are by weight unless otherwise specified.

### EXAMPLE I

### PREPARATION OF FLORALOZONE OXIME

### Reaction:

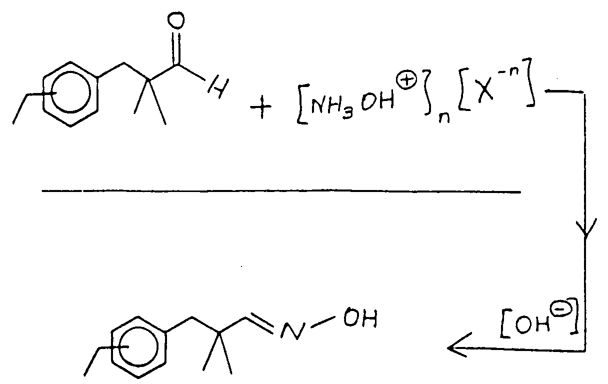

(wherein X is SO₄= and wherein n is 2).

Into a 2 liter reaction vessel equipped with stirrer, thermometer, heating mantle and addition funnel is placed 1200 ml water and 354 grams (2.14 moles) of hydroxylamine sulfate.

The hydroxylamine sulfate-water mixture is stirred until homogeneous while maintaining the temperature at 18°C. Over a period of 15 minutes while stirring the reaction mass and maintaining same at a termperature of 17°C, 534 grams (3 moles) of the mixture of aldehydes having the structure; 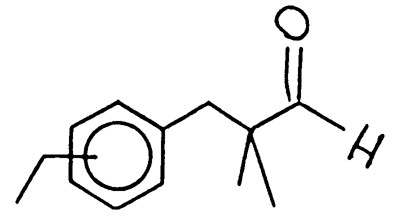
is added to the reaction mass.

394 Grams (4.92 moles) of sodium hydroxide is then added to the reaction mass over a period of 40 minutes while maintaining the reaction temperature in the range of 54-58°C.

The reaction mass is then allowed to cool to 40°C and the organic phase is separated from the aqueous phase.

The organic phase is washed with six 1000 ml portions of aqueous sodium chloride solution until neutral. 250 ml of ethyl acetate is added to cause even separation.

The reaction mass is filtered through anhydrous magnesium sulfate to yield 441 grams (2.28 moles) of the mixture of compounds having the structures:

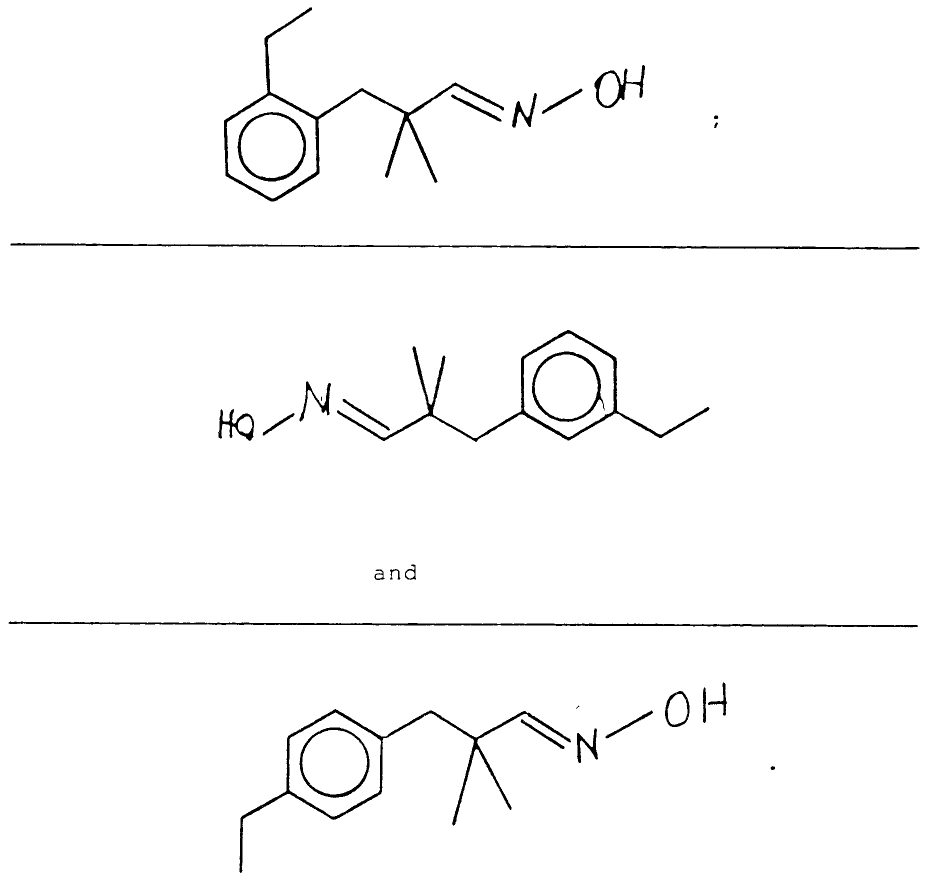

The reaction mass is then fractionally distilled yielding the following fractions:

| Fraction No. | Vapor Temp. (°C) | Liquid Temp. (°C) | Vacuum (mm/Hg.) Pa Pressure | Reflux Ratio |
|---|---|---|---|---|
| 1 | 63/90 | 130/130 | (2.2/2.21) 293.26/294.6 | 19:1/19:1 |
| 2 | 95 | 130 | (2.0) 266.6 | 9:1 |
| 3 | 97 | 135 | (2.0) 266.6 | 9:1 |
| 4 | 103 | 135 | (2.0) 266.6 | 9:1 |
| 5 | 120 | 140 | (2.4) 319.92 | 9:1 |
| 6 | 124 | 143 | (2.4) 319.92 | 4:1 |
| 7 | 128 | 143 | (3.0) 399.9 | 4:1 |
| 8 | 130 | 140 | (2.8) 373.24 | 4:1 |
| 9 | 126 | 140 | (2.2) 293.26 | 4:1 |
| 10 | 126 | 140 | (2.2) 293.26 | 4:1 |
| 11 | 130 | 142 | (2.6) 346.58 | 4:1 |
| 12 | 132 | 143 | (3.0) 399.9 | 4:1 |
| 13 | 133 | 144 | (3.6) 479.88 | 4:1 |
| 14 | 135 | 146 | (3.9) 519.87 | 4:1 |
| 15 | 137 | 153 | (4.6) 613.18 | 4:1 |
| 16 | 133 | 163 | (4.2) 559.86 | 4:1 |
| 17 | 133 | 200 | (6.0) 799.8 | 4:1. |

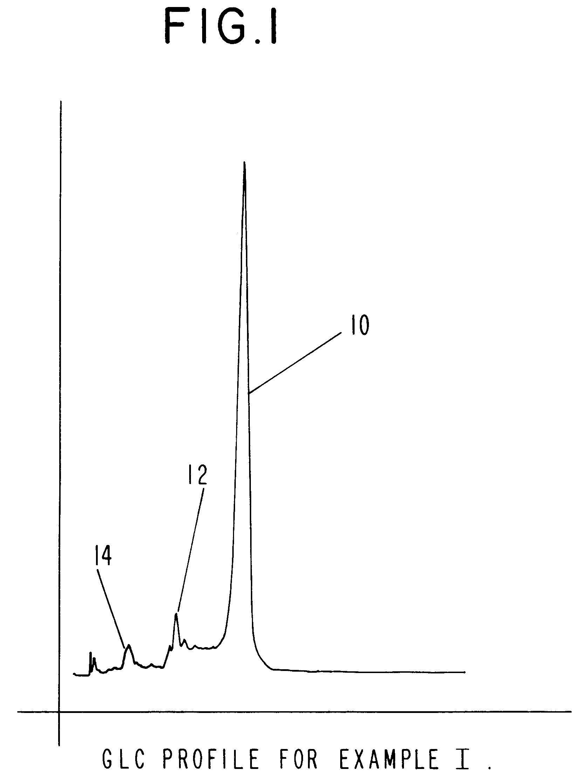

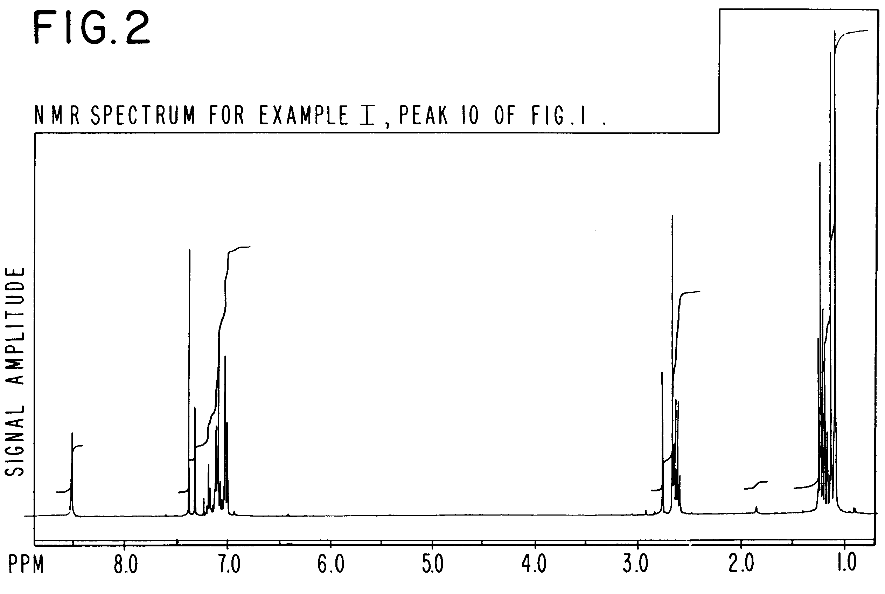

Figure 1 is the GLC profile for the reaction product.

The peak indicated by reference numeral 10 is the peak for the mixture of compounds having the structures: 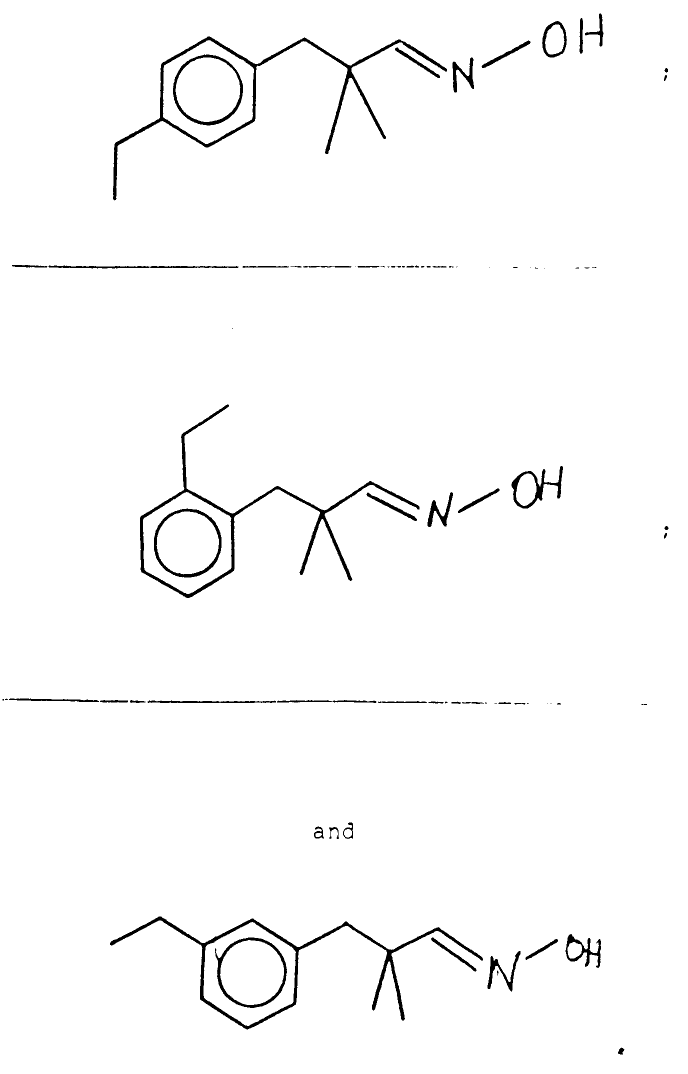
The peak indicated by reference numeral 12 is the peak for the nitriles produced during the reaction having the structures: 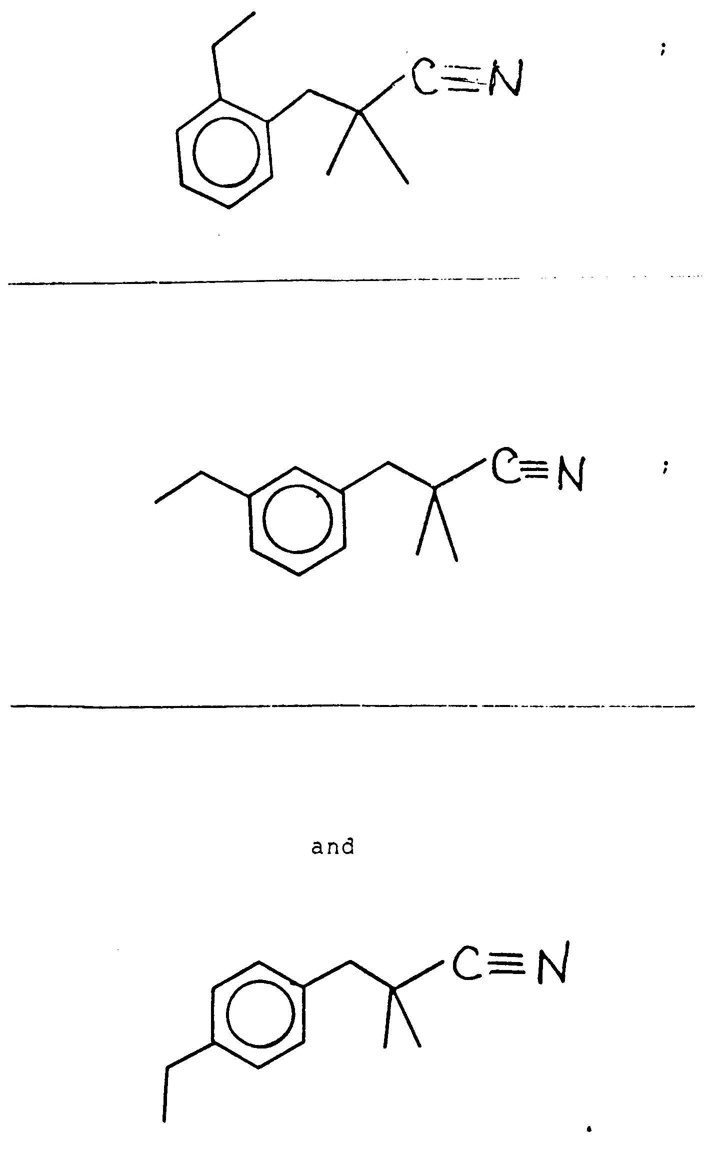
The peak indicated by reference numeral 14 is the peak for the compounds having the structures:

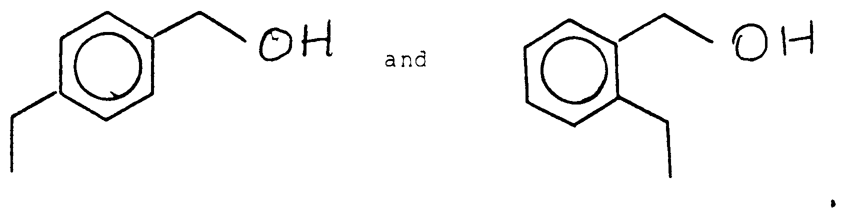

Figure 2 is the NMR spectrum for the peak indicated by reference numeral 10 of the GLC profile of Figure 1 for the compounds having the structures:

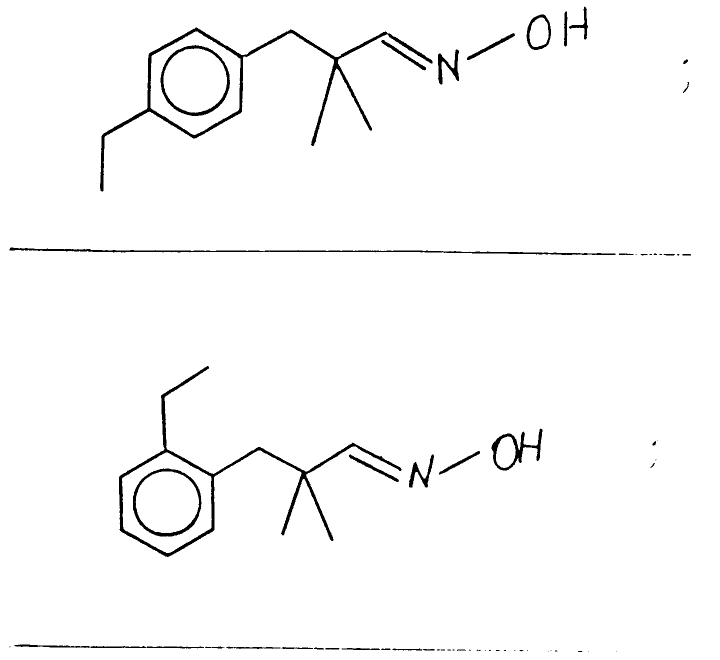

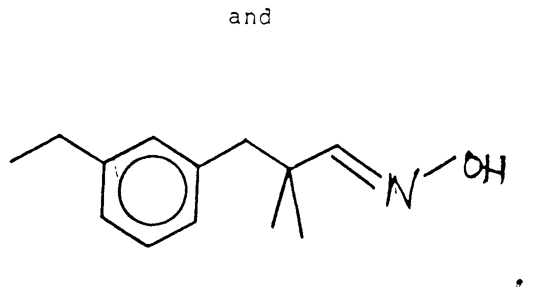

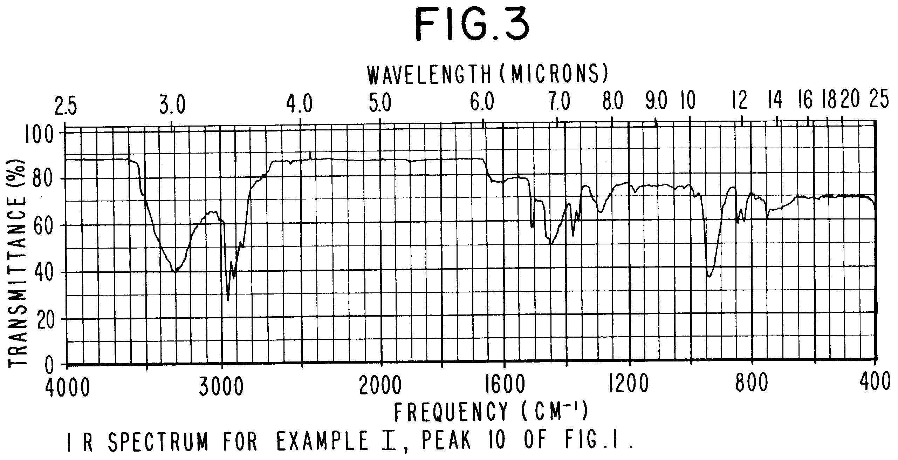

Figure 3 is the infrared-red spectrum for the peak indicated by reference numeral 10 of Figure 1 for the compounds having the structures:

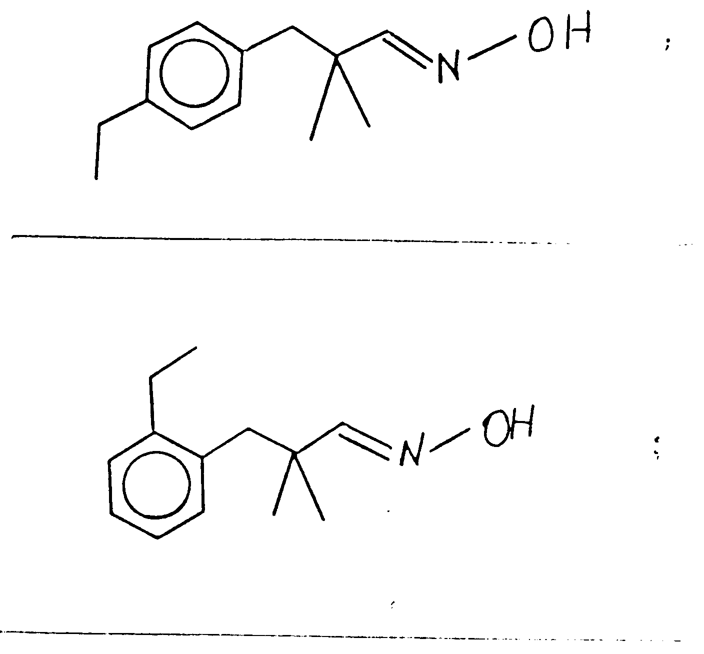

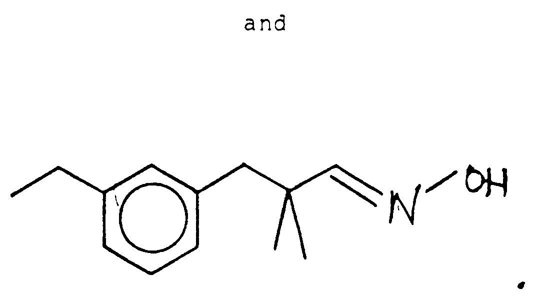

Fractions 9-11 of the foregoing distillation are bulked and the resulting product has a herbal, anisic, floral, jasmine and ozoney aroma with floral, dry, citrus, anise and ozoney undertones.

### EXAMPLE II

### PREPARATION OF FLORALOZONE NITRILE

### Reaction:

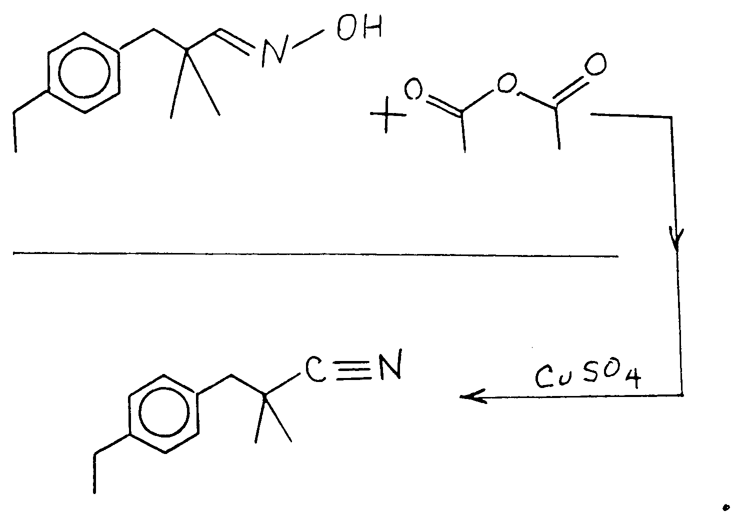

(wherein X is SO₄= and wherein n is 2).

Into a 2 liter reaction vessel equipped with stirrer, thermometer, heating mantle and addition funnel is placed 1200 ml water and 354 grams (2.14 moles) of hydroxylamine sulfate. 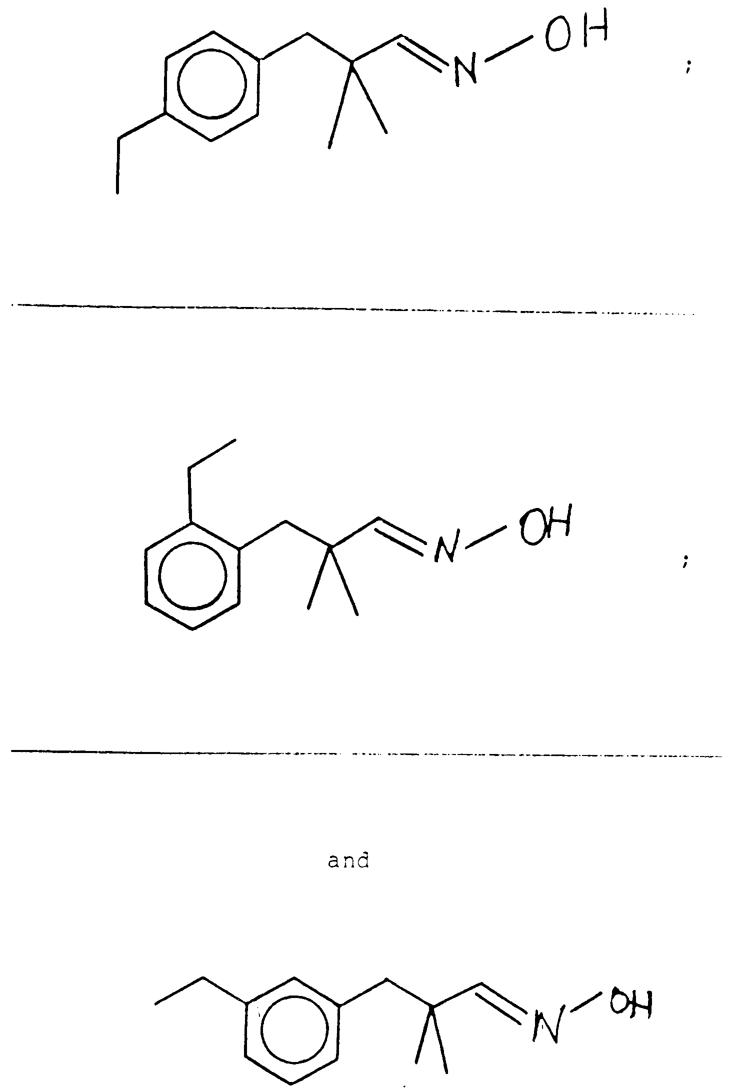
are added. At the end of the two hour period, the reaction mass is quenched by adding 500 ml water dropwise at 100°C. The reaction mass is then cooled as water is added. The reaction mass is allowed to settle overnight and the organic layer is separated from the aqueous layer. The organic layer is washed with two 300 ml volumes of saturated 10% sodium bicarbonate solution followed by two 300 ml portions of saturated sodium chlorite. The reaction mass is then filtered through anhydrous magnesium sulfate and distilled to yield 282 grams of product using an 18" x 1" Goodloe column and yielding the following fractions:

| Fraction No. | Vapor Temp. (°C) | Liquid Temp. (°C) | Vacuum (mm/Hg.) Pa Pressure | Reflux Ratio |
|---|---|---|---|---|
| 1 | 120/124 | 123/125 | (3.4/3.12) 453.22/415.9 | 4:1/ |
| 2 | 124 | 125 | (3.4) 453.22 | 1:1 |
| 3 | 123 | 126 | (3.44) 458.55 | 1:1 |
| 4 | 124 | 126 | (3.48) 463.88 | 1:1 |
| 5 | 123 | 126 | (3.42) 455.89 | 1:1 |
| 6 | 125 | 126 | (3.42) 455.89 | 1:1 |
| 7 | 106 | 119 | (1.57) 209.28 | 1:1 |
| 8 | 93 | 115 | (0.75) 99.98 | 1:1 |
| 9 | | | | |
| 10 | 93 | 117 | (0.77) 102.64 | 1:1 |
| 11 | 90 | 155 | (0.745) 99.31 | 1:1 |
| 12 | 90 | 187 | (0.800) 106.64 | 1:1. |

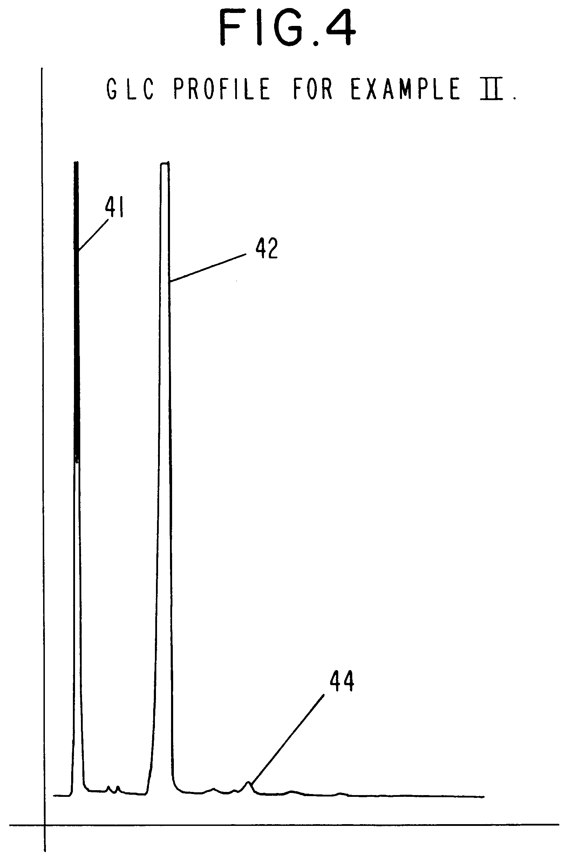

Figure 4 is the GLC profile for the crude reaction product. The peak indicated by reference numeral 42 is the peak for the mixture of compounds defined according to the structures: 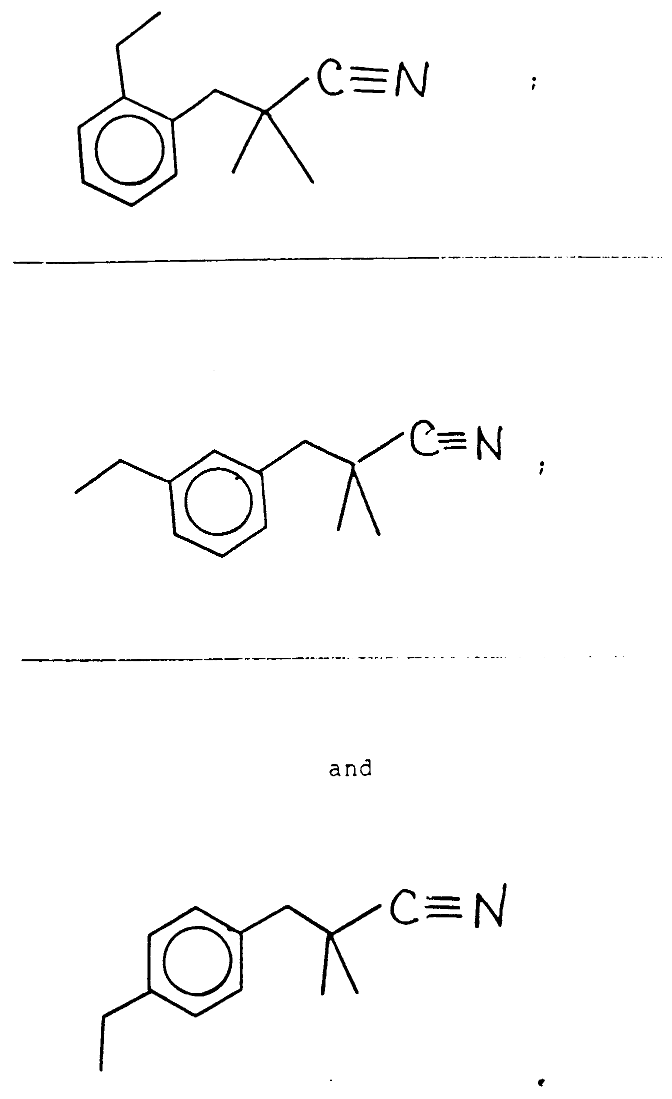
The peak indicated by reference numeral 41 is the peak for the reaction solvent, the acetic anhydride. The peak indicated by reference numeral 44 is the peak for the compounds having the structures:

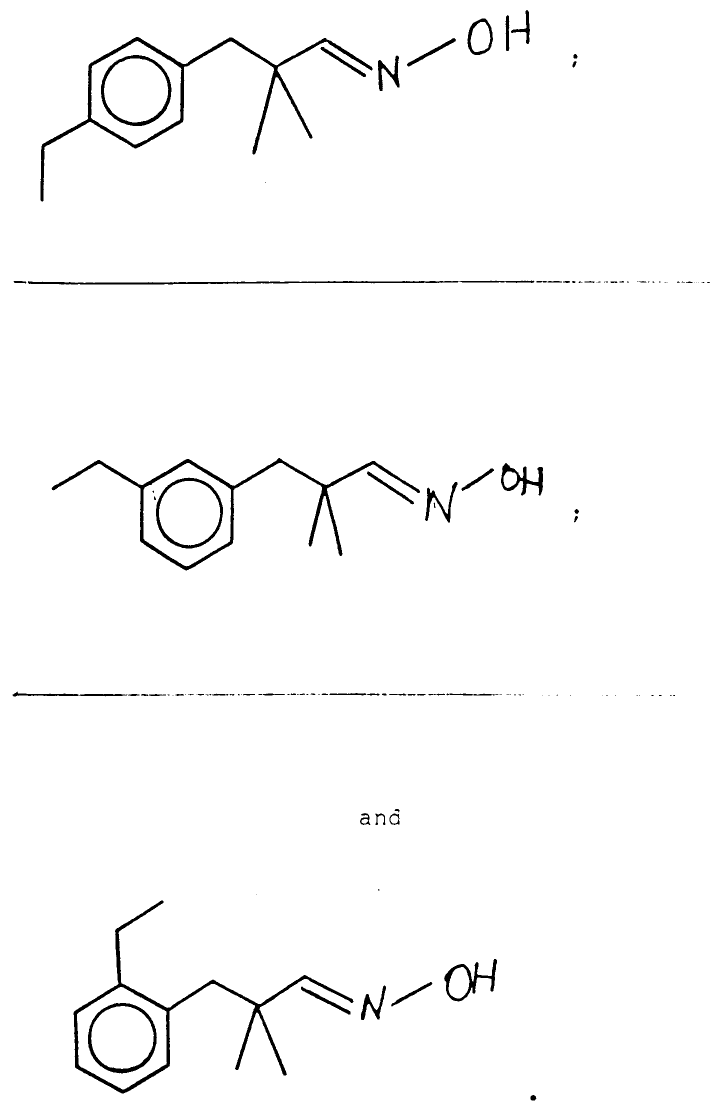

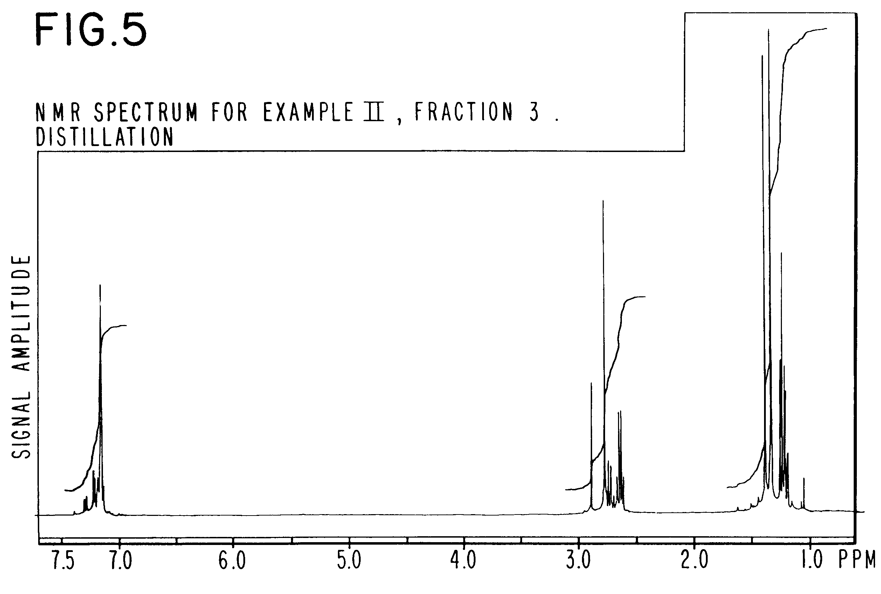

Figure 5 is the NMR spectrum for distillation fraction 3 of the reaction product of Example II containing the compounds having the structures:

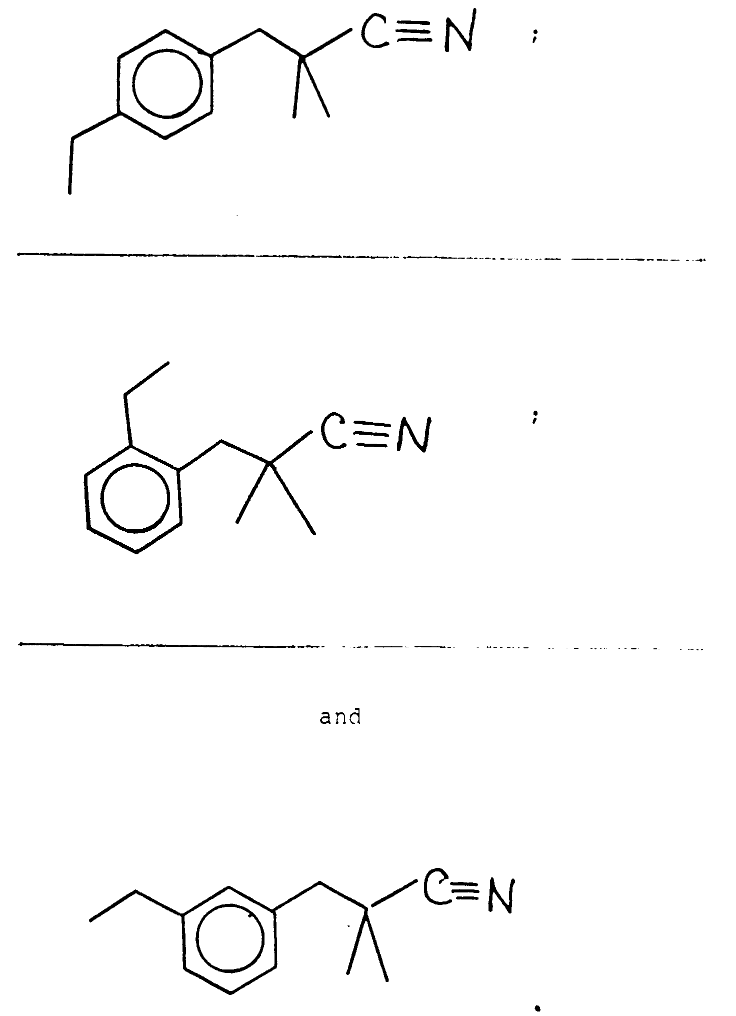

Figure 6 is the infra-red spectrum for the mixture of compounds having the structures: 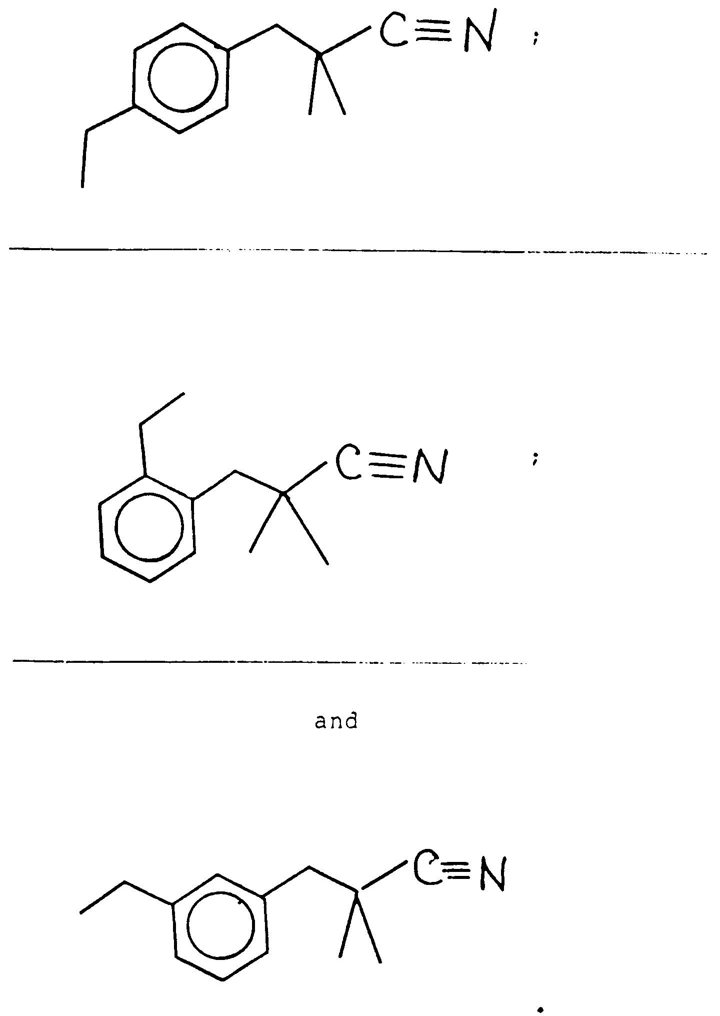
The peak indicated by reference numeral 62 is the peak for the nitrile moiety having the structure:

⁅C≡N].

### EXAMPLE III

The following Chypre formulations are prepared:

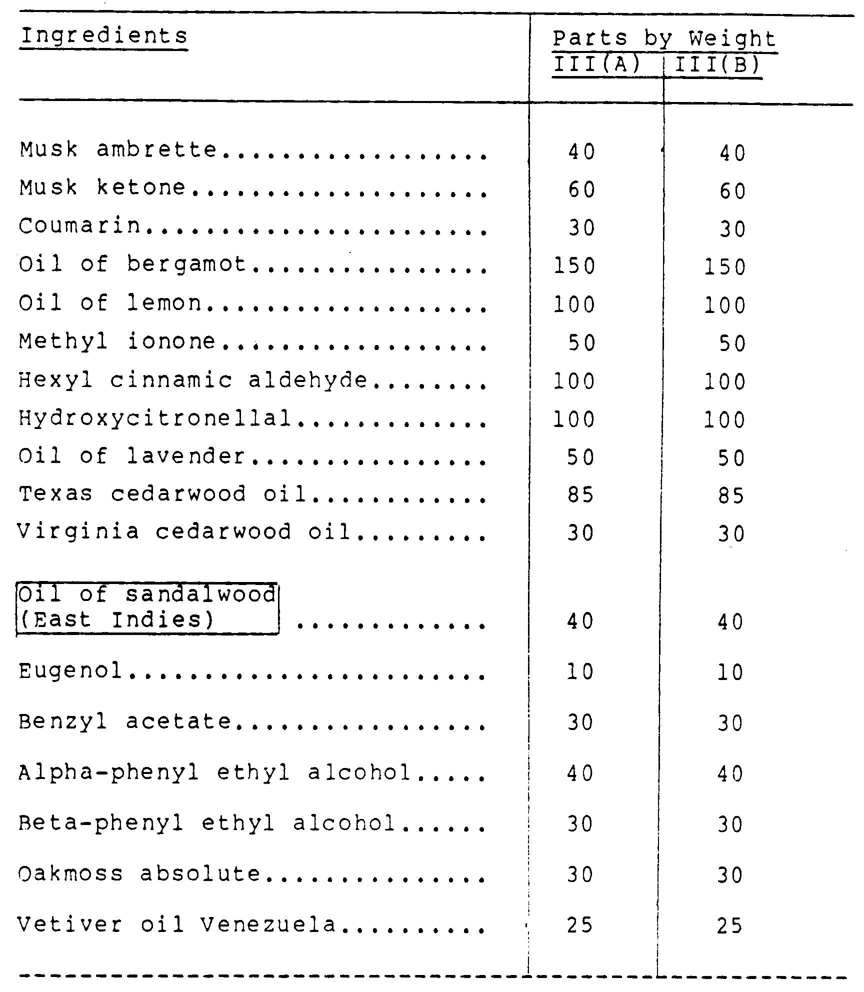

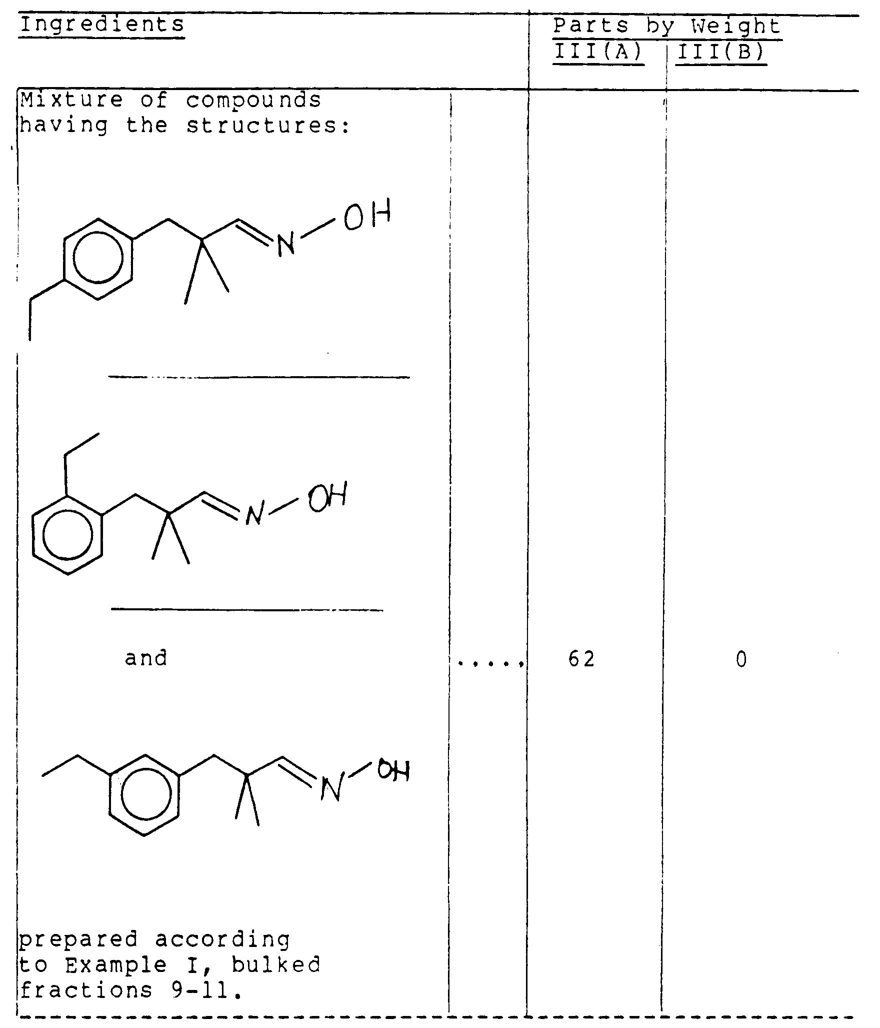

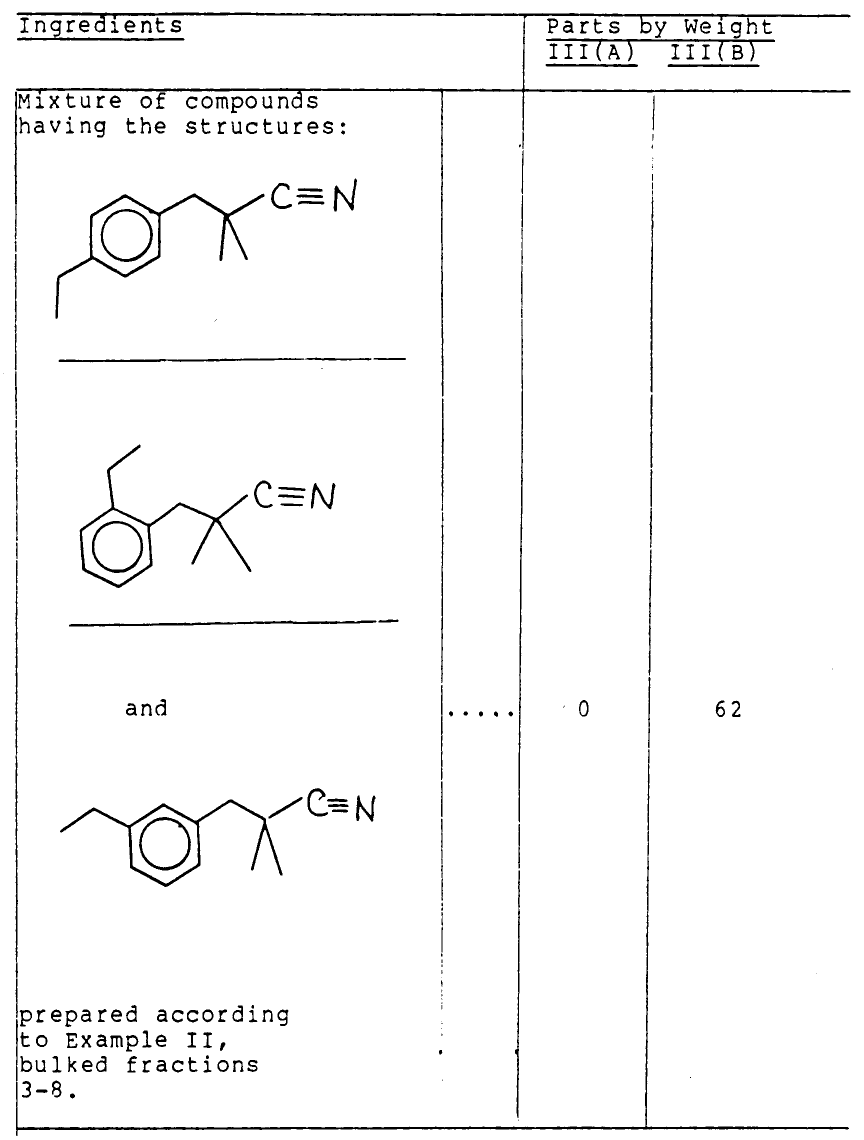

The mixture of compounds having the structures: 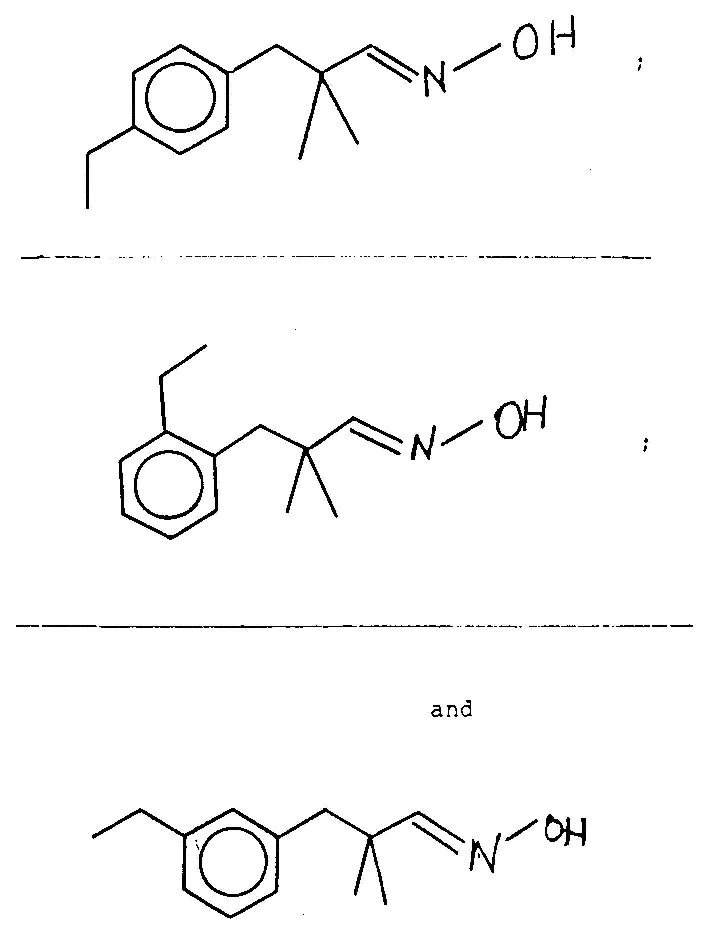
prepared according to Example I imparts to this Chypre formulation herbal, anisic, floral, jasmine and ozoney topnotes with floral, dry, citrus, anise and ozoney undertones. Accordingly, the formulation of Example III(A) can be described as having a "Chypre aroma with herbal, anisic, floral, jasmine and ozoney topnotes and floral, dry, citrus, anise and ozoney" undertones.

The mixture of compounds having the structures: 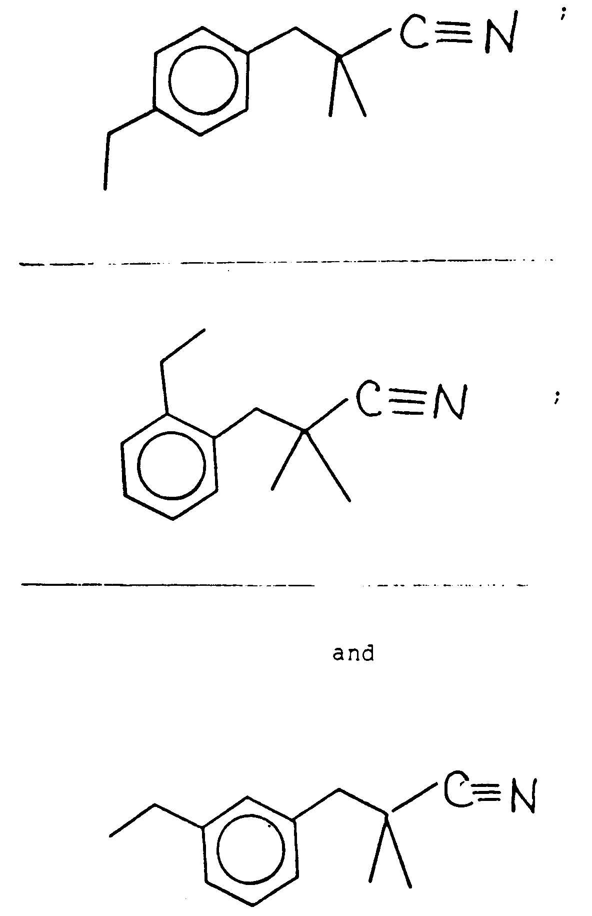
prepared according to Example II, imparts to this Chypre formulation ozoney, green and sweet vanilla-like undertones. Accordingly, the Chypre formulation of Example III(B) can be described as having a "Chypre aroma with ozoney, green and sweet vanilla" undertones.

### EXAMPLE IV

### PREPARATION OF COSMETIC POWDER COMPOSITIONS

Cosmetic powder compositions are prepared by mixing in a ball mill 100 grams of talcum powder with 0.25 grams of each of the substances set forth in Table III below. Each of the cosmetic powder compositions has an excellent aroma as described in Table III below:

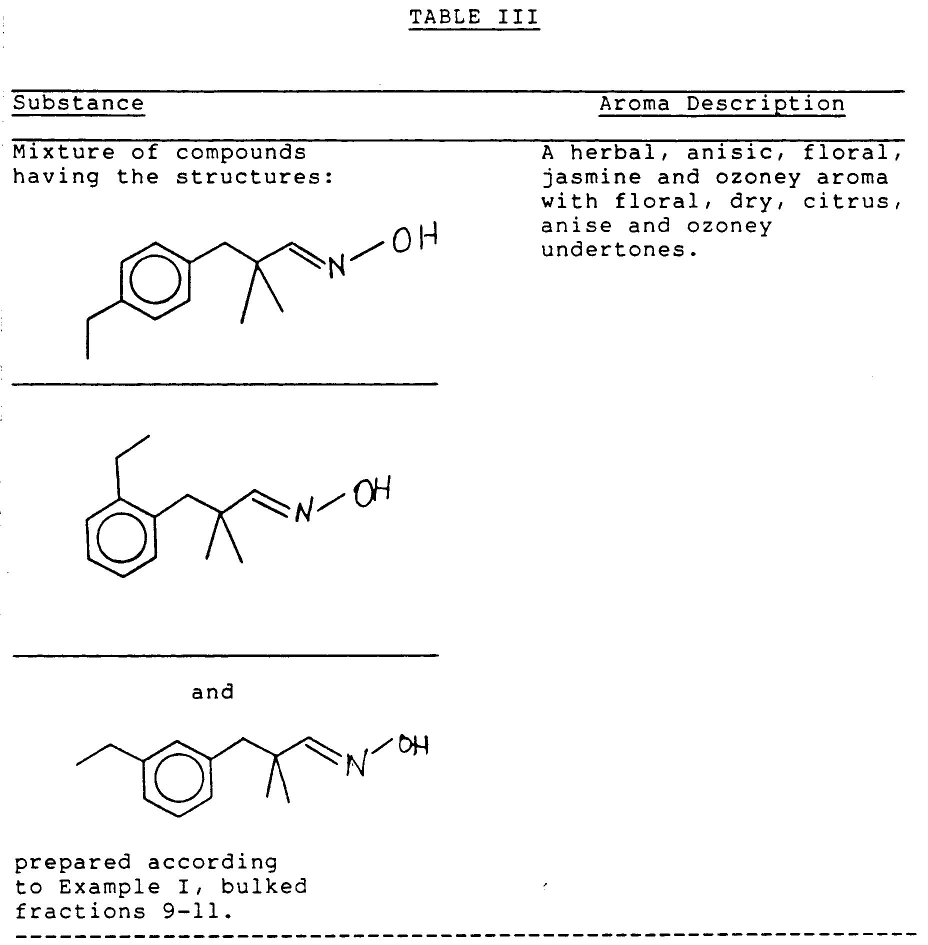

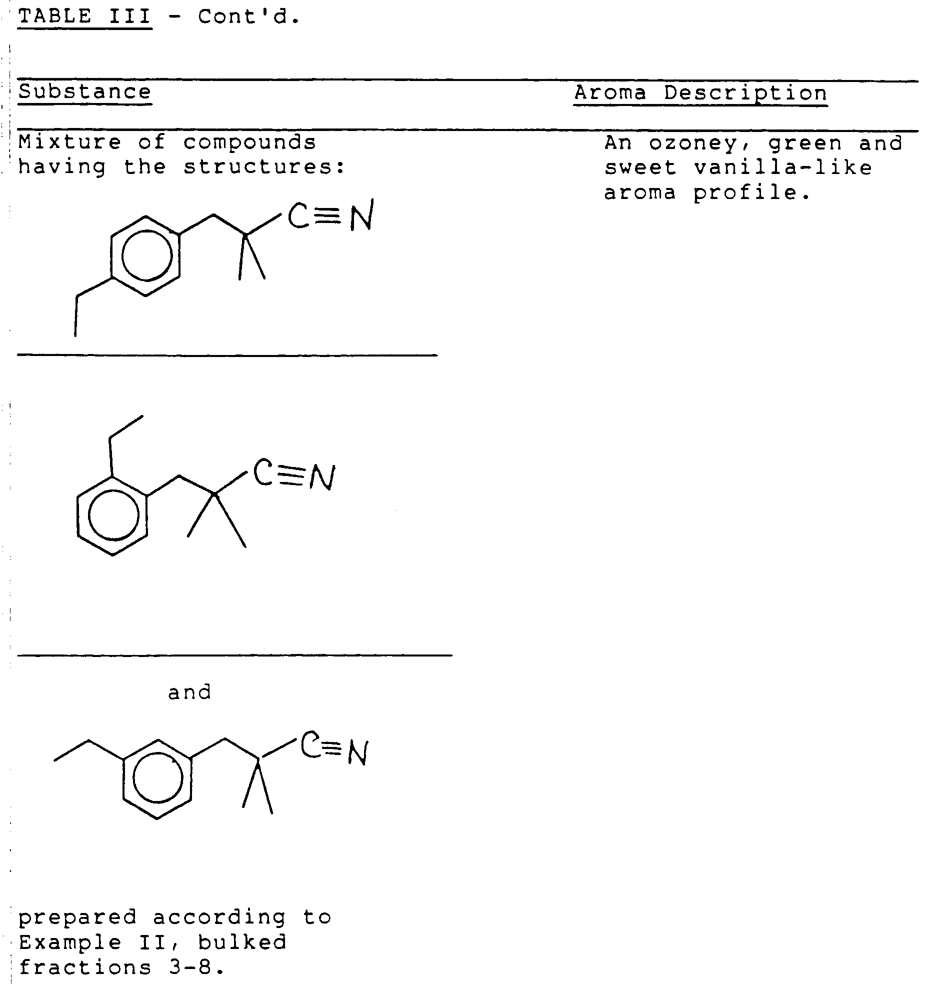

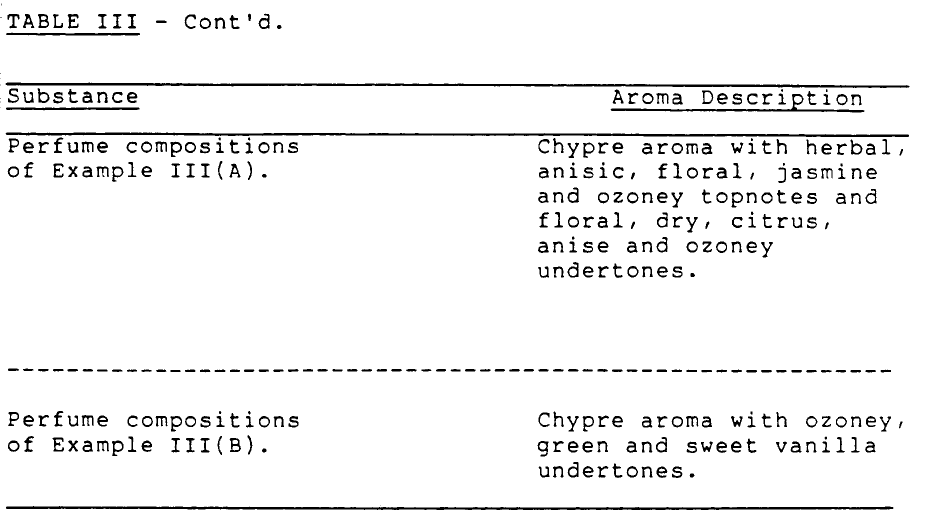

### EXAMPLE V

### PERFUMED LIQUID DETERGENTS

Concentrated liquid detergents (Lysine salt of no-dodecylbenzene sulfonic acid as more specifically described in U. S. Patent No. 3,948,818 issued on April 6, 1976) with aroma nuances as set forth in Table III of Example IV are prepared containing 0.10%, 0.15%, 0.20%, 0.25%, 0.30% and 0.35% of the substance set forth in Table III of Example IV. They are prepared by adding and homogeneously mixing the appropriate quantity of substance set forth in Table III of Example IV in the liquid detergent. The detergents all possess excellent aromas as set forth in Table III of Example IV, the intensity increasing with greater concentrations of substance as set forth in Table III of Example IV.

### EXAMPLE VI

### PREPARATION OF COLOGNES AND HANDKERCHIEF PERFUMES

Compositions as set forth in Table III of Example IV are incorporated into colognes at concentrations of 2.0%, 2.5%, 3.0%, 3.5%, 4.0%, 4.5% and 5.0% in 80%, 85%, 90% and 95% aqueous food grade ethanol solutions; and into handkerchief perfumes at concentrations of 15%, 20%, 25% and 30% (in 80%, 85%, 90% and 95% aqueous food grade ethanol solutions). Distinctive and definitive fragrances as set forth in Table III of Example IV are imparted to the colognes and to the handkerchief perfumes at all levels indicated.

### EXAMPLE VII

### PREPARATION OF SOAP COMPOSITIONS

One hundred grams of soap chips [per sample](IVORY®, produced by the Procter & Gamble Company of Cincinnati, Ohio), are each mixed with one gram samples of substances as set forth in Table III of Example IV until homogeneous compositions are obtained. In each of the cases, the homogeneous compositions are heated under 810.4kPa (8 atmospheres pressure) at 180°C for a period of three hours and the resulting liquids are placed into soap molds. The resulting soap cakes, on cooling, manifest aromas as set forth in Table III of Example IV.

### EXAMPLE VIII

### PREPARATION OF SOLID DETERGENT COMPOSITIONS

Detergents are prepared using the following ingredients according to Example I of Canadian Patent No. 1,007,948:

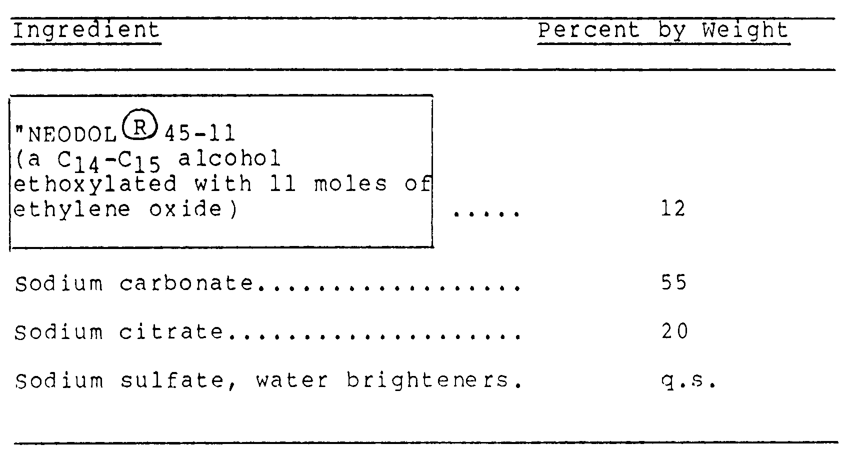

This detergent is a phosphate-free detergent. Samples of 100 grams each of this detergent are admixed with 0.10, 0.15, 0.20 and 0.25 grams of each of the substances as set forth in Table III of Example IV. Each of the detergent samples has an excellent aroma as indicated in Table III of Example IV.

### EXAMPLE IX

Utilizing the procedure of Example I at column 15 of U.S. Patent No. 3,632,396, non-woven cloth substrates useful as drier-added fabric softening articles of manufacture are prepared wherein the substrate, the substrate coating and the outer coating and the perfuming material are as follows:
1. A water "dissolvable" paper ("Dissolvo Paper");
2. Adogen 448 (m.p. about 60°C (140°F)) as the substrate coating; and
3. An outer coating having the following formulation (m.p. about 65.56°C (150°F)):
   - 57% -: C₂₀₋₂₂ _{HAPS}
   - 22% -: isopropyl alcohol
   - 20% -: antistatic agent
   - 1% -: of one of the substances as set forth in Table III of Example IV.

Fabric softening compositions prepared according to Example I at column 15 of U.S. Patent No. 3,632,396 having aroma characteristics as set forth in Table III of Example IV consist of a substrate coating having a weight of about 3 grams per 645.16 cm² (100 square inches) of substrate; a first coating on the substrate coating consisting of about 1.85 grams per 645.16 cm² (100 square inches) of substrate; and an outer coating coated on the first coating consisting of about 1.4 grams per 645.16 cm² (100 square inches) of substrate. One of the substances of Table III of Example IV is admixed in each case with the outer coating mixture, thereby providing a total aromatized outer coating weight ratio to substrate of about 0.5:1 by weight of the substrate. The aroma characteristics are imparted in a pleasant manner to the head space in a dryer on operation thereof in each case using said dryer-added fabric softener non-woven fabrics and these aroma characteristics are described in Table III of Example IV.

### EXAMPLE X

### HAIR SPRAY FORMULATIONS

The following hair spray formulation is prepared by first dissolving PVP/VA E-735 copolymer manufactured by the GAF Corporation of 140 west 51st Street, New York, New York, in 91.62 grams of 95% food grade ethanol, 8.0 grams of the polymer is dissolved in the alcohol. The following ingredients are added to the PVP/VA alcoholic solution:

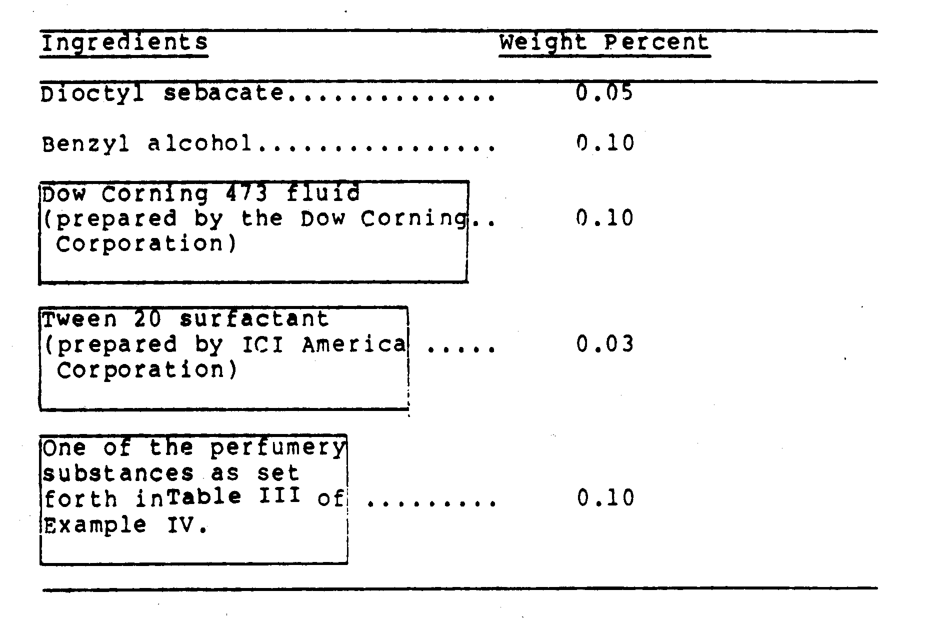

The perfuming substances as set forth in Table III of Example IV add aroma characteristics as set forth in Table III of Example IV which are rather intense and aesthetically pleasing to the users of the soft-feel, good-hold pump hair sprays.

### EXAMPLE XI

### CONDITIONING SHAMPOOS

Monamid CMA (prepared by the Mona Industries Company)(3.0 weight percent) is melted with 2.0 weight percent coconut fatty acid (prepared by Procter & Gamble Company of Cincinnati, Ohio); 1.0 weight percent ethylene glycol distearate (prepared by the Armak Corporation) and triethanolamine (a product of Union Carbide Corporation)(1.4 weight percent). The resulting melt is admixed with Stepanol WAT produced by the Stepan Chemical Company (35.0 weight percent). The resulting mixture is heated to 60°C and mixed until a clear solution is obtained (at 60°C).

GAFQUAT® 755N polymer (manufactured by GAF Corporation of 140 West 51st Street, New York, New York )(5.0 weight percent) is admixed with 0.1 weight percent sodium sulfite and 1.4 weight percent polyethylene glycol 6000 distearate produced by Armak Corporation.

The resulting material is then mixed and cooled to 45°C and 0.3 weight percent of perfuming substance as set forth in Table III of Example IV is added to the mixture. The resulting mixture is cooled to 40°C and blending is carried out for an additional one hour in each case. At the end of this blending period, the resulting material has a pleasant fragrance as indicated in Table III of Example IV.

### EXAMPLE XII

Four drops of each of the substances set forth in Table III of Example IV, supra, is added separately to two grams of AROMOX® DMC-W to produce a clear premix. The clear premix is added to 200 grams of CLOROX® with stirring resulting in a clear stable, single phase solution. Sufficient 1 M aqueous NaOH is added to bring the pH of the mixture up to 12.8. The solution remains substantially stable 48.89°C (120°F) for a period of seven days. When the 5% aqueous sodium hypochlorite solution is used as a laundry bleach, the resulting laundry, on dry-out in an atmosphere of 65% relative humidity yields substantially no characteristic "hypochlorite" odor, but does have a faint pleasant aroma as set forth in Table III of Example IV. Furthermore, no such characteristic "hypochlorite" aroma is retained on the hands of the individual handling such laundry in both the wet and the dry states.

### EXAMPLE XIII

AROMOX® DMMC-W in various quantities is mixed with 0.1 grams of one of the substances set forth in Table III of Example IV, supra. The resulting premixes are then added to 200 grams of an aqueous 5% sodium hypochlorite solution. Sufficient 12.5 M aqueous NaOH is added to bring the pH of the mixture up to 13. The following results are obtained:

| Percentage AROMOX® DMMC- W | Clarity of hypochlorite solution after addition of premix |
|---|---|
| 0.23% | Clear after three days |
| 0.15% | Clear after three days |
| 0.08% | Initially slightly turbid; two phases exist after three days. |

When the 5% aqueous sodium hypochlorite solution is used as a laundry bleach, the resulting laundry on dry-out, in an atmosphere of 65% relative humidity, yields substantially no characteristic "hypochlorite" odor, but does have a faint, pleasant aroma as set forth in Table III of Example IV. Furthermore, no such characteristic "hypochlorite" aroma is retained on the hands of the individual handling such laundry in both the wet and dry states.

### EXAMPLE XIV

Two grams of AROMOX® DMMC-W is admixed with eight drops of one of the substances set forth in Table III of Example IV, supra. The premix is then added with stirring to 200 grams of a 7% aqueous solution of lithium hypochlorite. Sufficient 3 M aqueous LiOH is added to bring the pH of the solution to 13.4. The mixture is then heated to 48.89°C (120°F) and maintained at that temperature with stirring for a period of one week. The resulting solution remains clear in a single phase. When used as a laundry bleach, the resulting bleached laundry, on dry-out in an atmosphere of 50% relative humidity retains a "clean" warm aroma as set forth in Table III of Example IV, supra; whereas without the use of the substance set forth in Table III of Example IV, supra, the bleached laundry has a faint characteristic disagreeable "hypochlorite" aroma.

### EXAMPLE XV

Two grams of AROMOX® DMMC-W is admixed with eight drops of one of the substances of Table III of Example IV, supra. This premix is then added, with stirring to 200 grams of a mixture containing 4.5% aqueous sodium hypochorite. Sufficient 4 M aqueous LiOH is added to bring the pH of the solution to 13.4. The mixture is then heated to 48.89°C (120°F) and maintained at that temperature for a period of one week. The resulting solution remains clear in a single phase. When used as a laundry bleach, the resulting bleached laundry on dry-out in an atmosphere of 50% relative humidity retains a "clean fresh" warm aroma as set forth in Table III of Example IV, supra; whereas without the use of the substance set forth in Table III of Example IV, supra, the bleached laundry has a faint characteristic disagreeable "hypochlorite" aroma.

### EXAMPLE XVI

Two grams of AROMOX® DMMC-W is admixed with eight drops of one of the substances as set forth in Table III of Example IV, supra. This premix is then added with stirring to 200 grams of a mixture containing 4.5% aqueous sodium hypochlorite and 4.5% aqueous lithium hypochlorite. Sufficient 2 M aqueous NaOH is added to bring the pH of the solution to 13.4. The mixture is then heated to 43.33°C (110°F) and maintained at that temperature with stirring for a period of 2 weeks. The resulting solution remains clear as a single phase when used as a laundry bleach. The resulting laundry bleach, on dry-out in an atmosphere of 50% relative humidity, retains an aroma as set forth in Table III of Example IV, supra, whereas without the use of the substance set forth in Table III of Example IV, supra, the bleached laundry has a faint characteristic disagreeable "hypochlorite" aroma.

### EXAMPLE XVII

Four drops of one of the substances set forth in Table III of Example IV, supra, is added to 1.5 grams of AROMOX® to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 48.89°C (120°F) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant warm, long-lasting aroma as set forth in Table III of Example IV, supra. Furthermore, no such characteristic "hypochlorite" aroma is retained on the hands of the individual handling such laundry in both the wet and dry states.

### EXAMPLE XVIII

Four drops of one of the substances set forth in Table III of Example IV, supra, is added to 1 gram n-undecyl dimethyl amine oxide to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 48.89°C (120°F) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant warm aroma as set forth in Table III of Example IV, supra. Furthermore, no such characteristic "hypochlorite" aroma is retained on the hands of the individual handling such laundry in both the wet and the dry states.

### EXAMPLE XIX

Four drops of one of the substances as set forth in Table III of Example IV, supra are added to 1 gram of n-dododecyl dimethyl amine oxide to produce a clear premix. The clear premix is added to 200 grams of CLOROX® with stirring resulting in a clear, stable single phase solution. Sufficient 1 M aqueous NaOH is added to bring the pH of the mixture up to 12.8. The solution remains substantially stable at 48.89°C (120°F) for a period of 7 days. When the 5% aqueous sodium hypochlorite solution is used as a laundry bleach, the resulting laundry on dry-out in an atmosphere of 65% relative humidity yields substantially no characteristic "hypochlorite" aroma, but does have a warm, pleasant, long-lasting aroma as set forth in Table III of Example IV, supra. Furthermore, no such characteristic "hypochlorite" aroma is retained on the hands of the individual handling such laundry in both the wet and dry states.

### EXAMPLE XX

One gram of n-tridecyl dimethyl amine oxide is admixed with eight drops of one of the substances as set forth in Table III of Example IV, supra. This premix is then added with stirring to 200 grams of a 7% aqueous solution of lithium hypochlorite. Sufficient 3 M aqueous LiOH is added to bring the pH of the solution to 13.4. The mixture is then heated to 48.89°C (120°F) and maintained at that temperature with stirring for a period of one week. The resulting solution remains clear in a single phase. When used as a laundry bleach, the resulting bleached laundry on dry-out in an atmosphere of 50% relative humidity retains a warm, fresh aroma described in Table III of Example IV, supra, whereas without the use of one of the substances of Table III of Example IV, supra, the bleached laundry has a faint characteristic disagreeable "hypochlorite" aroma.

### EXAMPLE XXI

Four drops of the mixture of compounds having the structures: 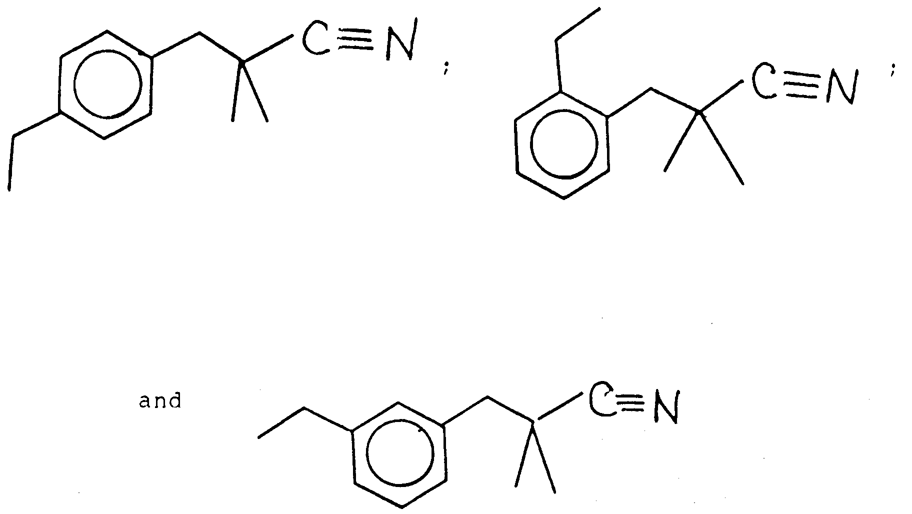
prepared according to Example II (bulked fractions 3-8) is added to 2 grams of AROMOX® DMC-W to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a strong fresh ozoney, green and sweet vanilla aroma profile. Furthermore, no such characteristic "hypochlorite" aroma is retained on the hands of the individual handling such laundry in both the wet and the dry states.

### EXAMPLE XXII

AROMOX® DMMC-W in various quantities is mixed with 0.1 grams of the mixture of compounds having the structures: 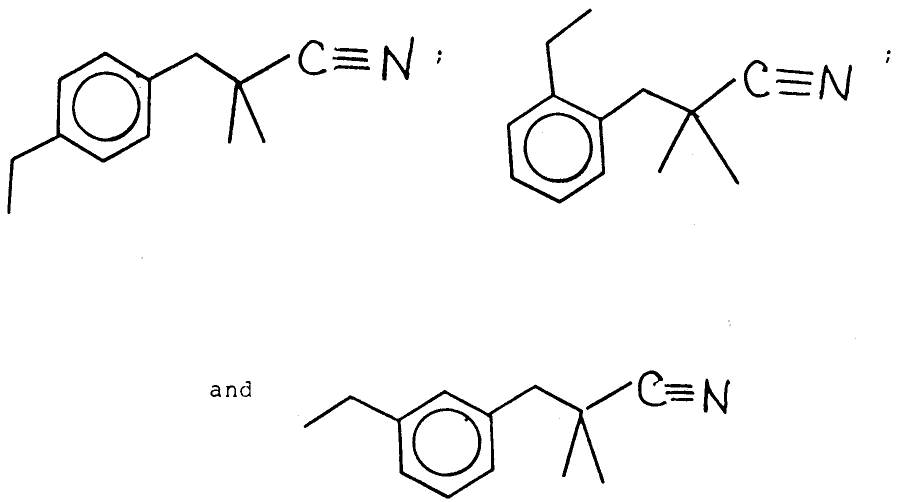
prepared according to Example II (bulked fractions 3-8). The resulting premix is then added to 200 grams of an aqueous 5% sodium hypochlorite solution. Sufficient 12.5 M aqueous NaOH is added to bring the pH of the mixture up to 13. The following results are obtained:

| Percentage AROMOX® DMMC-W | Clarity of Hypochlorite Solution After Addition of Premix |
|---|---|
| 0.23% | Clear after three days. |
| 0.15% | Clear after three days. |
| 0.08% | Initially slightly turbid; two phases exist after three days. |

When the 5% aqueous sodium hypochlorite solutions are used as laundry bleaches, the resulting laundry batches on dry-out In an atmosphere of 65% relative humidity yields substantially no characteristic "hypochlorite" odor, but does have a strong fresh ozoney, green and sweet vanilla aroma. Furthermore, no such characteristic "hypochlorite" aroma is retained on the hands of the individual handling such laundry batches in both the wet and the dry states.

### EXAMPLE XXIII

Two grams of AROMOX® DMMC-W are admixed with eight drops of the mixture of compounds having the structures: 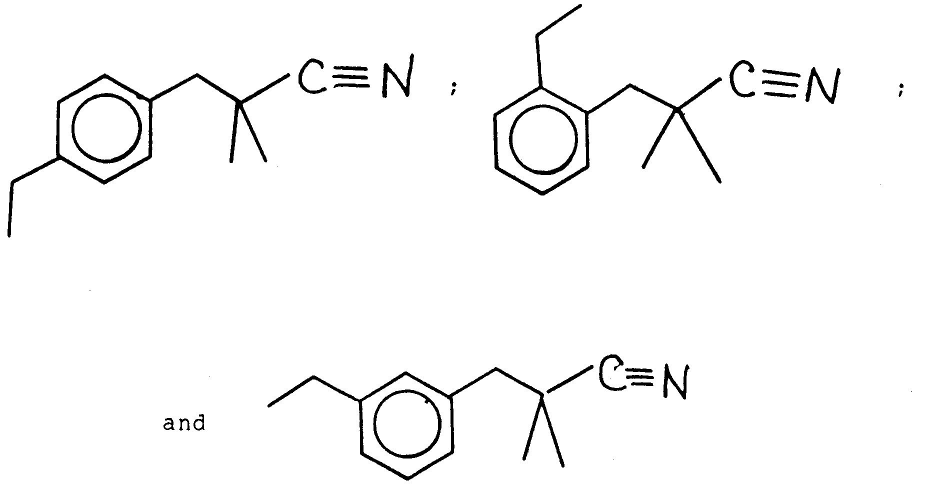
prepared according to Example II (bulked fractions 3-8). The premix is then added with stirring to 200 grams of a 7% aqueous solution of lithium hypochlorite. Sufficient 3 M aqueous LiOH is added to bring the pH of the solution to 13.4. The mixtures are then heated to 48.89°C (120°F) and maintained at that temperature with stirring for a period of one week. The resulting solution remains clear in a single phase. When used as laundry bleaches, the resulting bleached laundry batches on dry-out in an atmosphere of 50% relative humidity retain a strong fresh ozoney, green, sweet, vanilla profile whereas without the use of the the mixture of compounds having the structures: 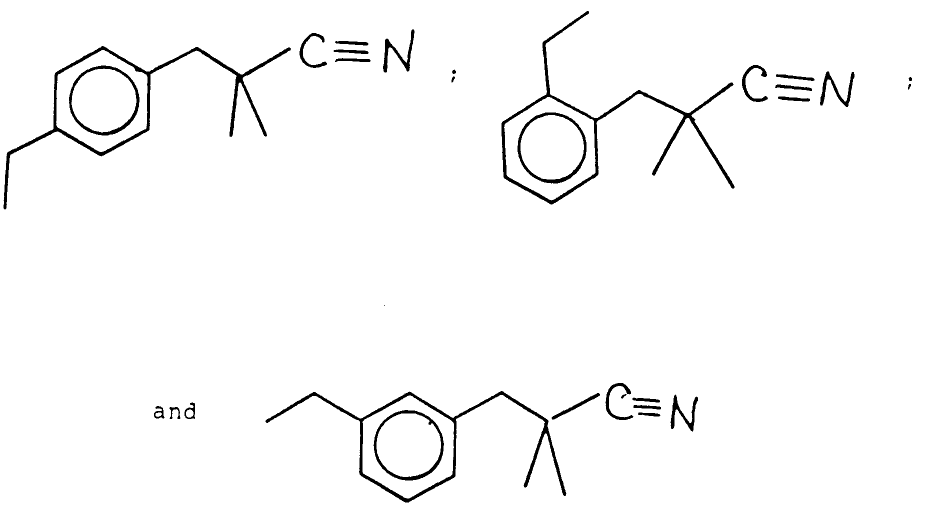
prepared according to Example II (bulked fractions 3-8), the bleached laundry batches have a faint characteristic disagreeable "hypochlorite" aroma.

### EXAMPLE XXIV

Two grams of AROMOX® DMMC-W are admixed with eight drops of the mixture of compounds having the structures: 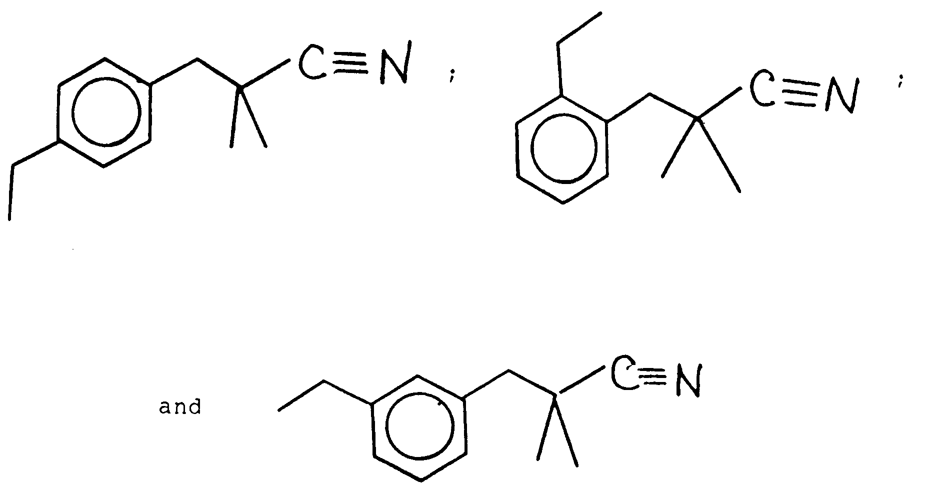
prepared according to Example II (bulked fractions 3-8). The premix is then added with stirring to 200 grams of a mixture containing 4.5% aqueous sodium hypochlorite and 4.5% aqueous lithium hypochlorite. Sufficient 4 M aqueous LiOH is added to bring the pH of the solution to 13.4. The mixture is then heated to 48.89°C (120°F) and maintained at that temperature for a period of one week. The resulting solution remains clear in a single phase. When used as a laundry bleach, the resulting bleached laundry batches on dry-out in an atmosphere of 50% relative humidity retain a strong fresh ozoney, green and sweet vanilla aroma profile whereas without the use of the mixture of compounds having the structures: 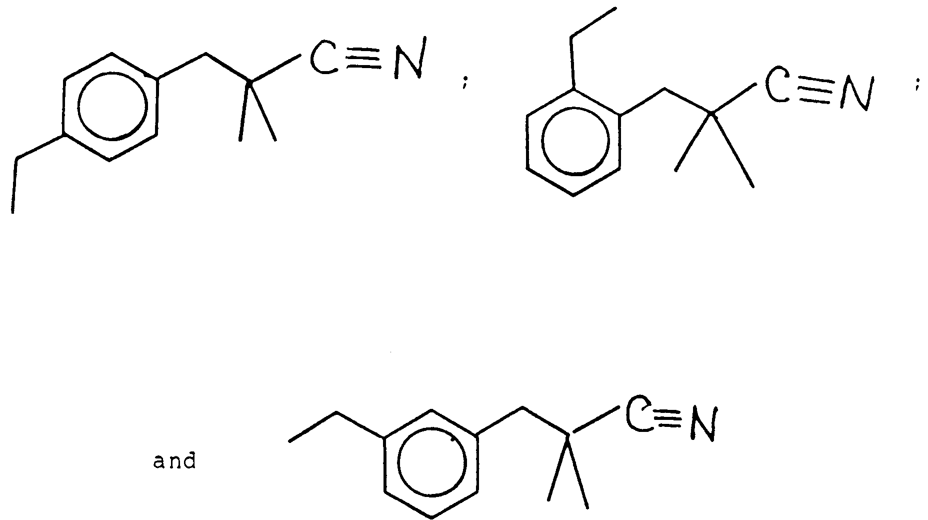
prepared according to Example II (bulked fractions 3-8), the bleached laundry batches have faint characteristic disagreeable "hypochlorite" aroma.

### EXAMPLE XXV

Two grams of AROMOX® DMMC-W are admixed with eight drops of either (a) the mixture of compounds having the structures: 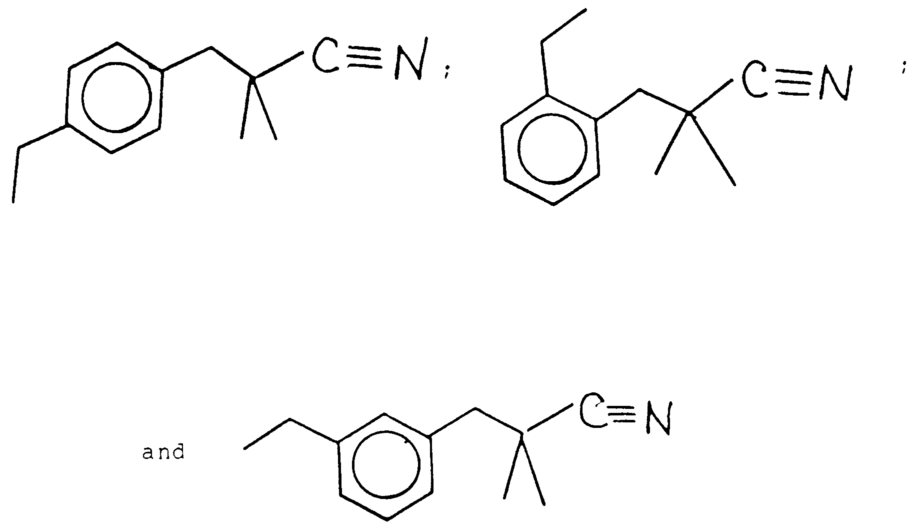
prepared according to Example II (bulked fractions 3-8); or (b) a 50-50 mixture of the mixture of compounds having the structures: 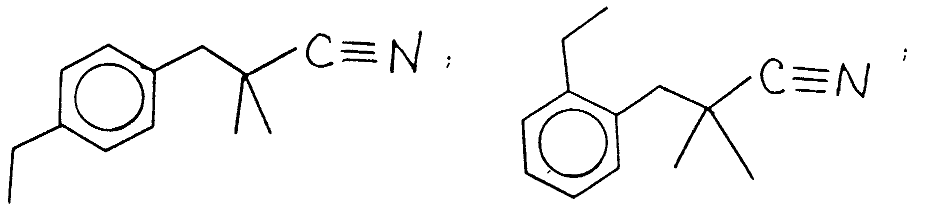 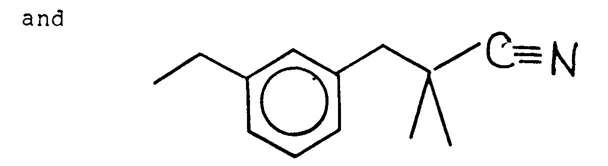
prepared according to Example II, bulked fractions 3-8 and diisoamylene epoxide produced according to Example II of application for United States Letters Patent 4,335,009 issued June 15, 1982 the disclosure of which is incorporated herein by reference. These premixes are then added with stirring to 200 grams of a mixture containing 4% aqueous sodium hypochlorite and 4% aqueous lithium hypochlorite. Sufficient 2 M aqueous NaOH is added to bring the pH of the solutions to 13.4. The mixtures are then heated to 43.33°C (110°F) and maintained at that temperature with stirring for a period of two weeks. The resulting solutions remain clear as a single phase when used as laundry bleaches. The resulting bleached laundry batches on dry-out in an atmosphere of 50% relative humidity have a strong fresh ozoney, green and sweet vanilla aroma (when using the mixture of compounds having the structures: 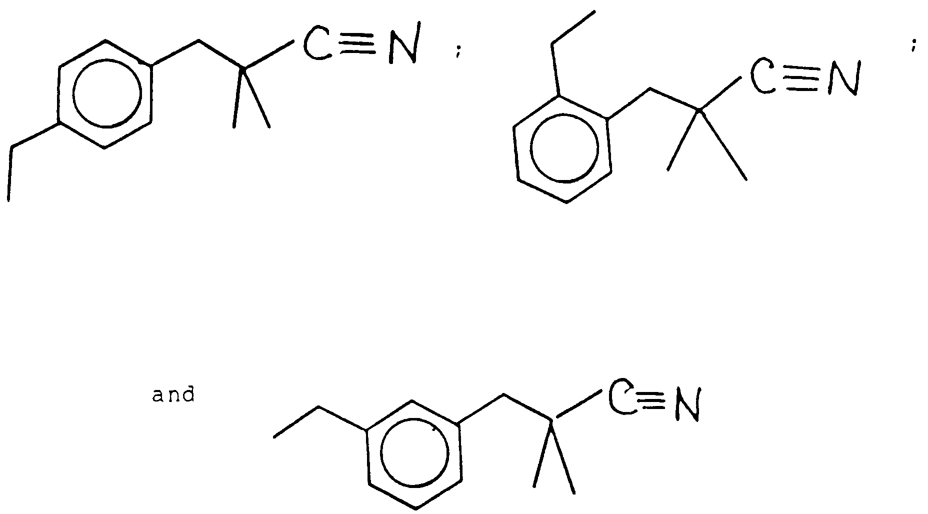
prepared according to Example II, bulked fractions 3-8 have a strong fresh ozoney, green and sweet vanilla aroma when using the mixture of the diisoamylene epoxide and the mixture of compounds having the structures: 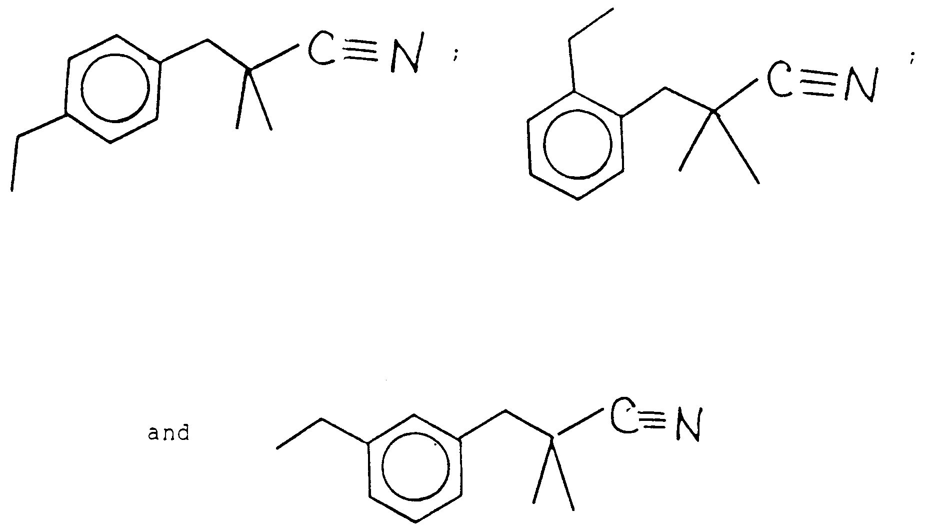
prepared according to Example II, bulked fractions 3-8; whereas without the use of the mixture of compounds having the structures: 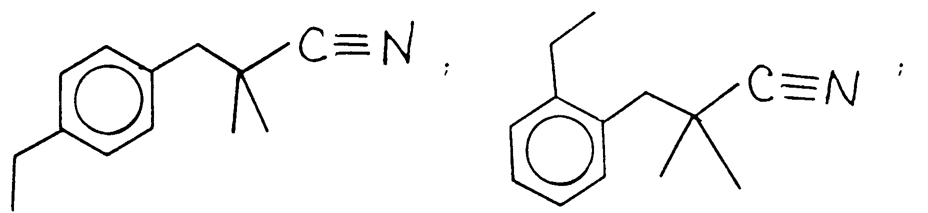 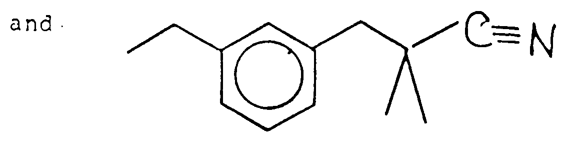
prepared according to Example II, bulked fractions 3-8 containing compositions of matter the bleached laundry batches have faint characteristic disagreeable "hypochlorite" aromas.

### EXAMPLE XXVI

Four drops of the mixture of compounds having the structures: 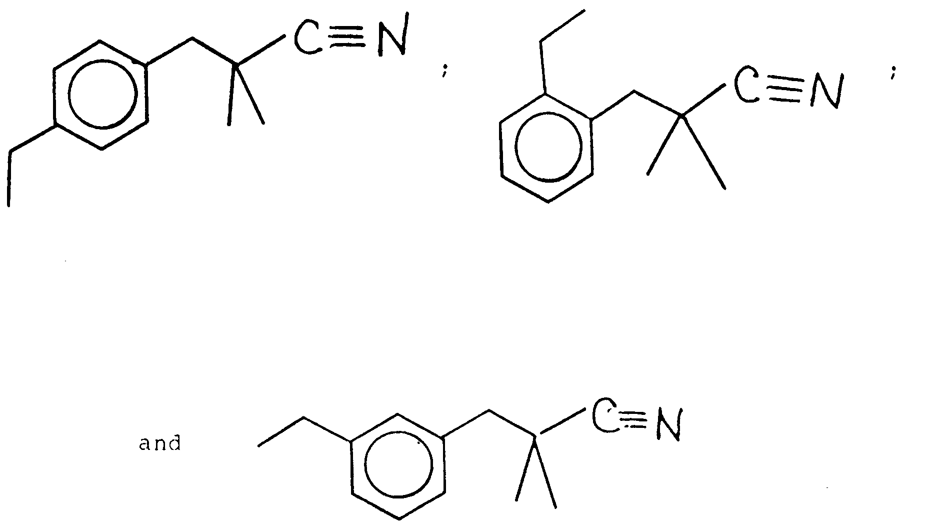
prepared according to Example II, bulked fractions 3-8 are added to 1.5 grams of AROMOX® NCMD-W to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a strong fresh ozoney, green and sweet vanilla aroma. Furthermore, no such characteristic "hypochlorite" aroma is retained on the hands of the individual handling such laundry in both the wet and the dry states.

### EXAMPLE XXVII

Four drops of the mixture of compounds having the structures: 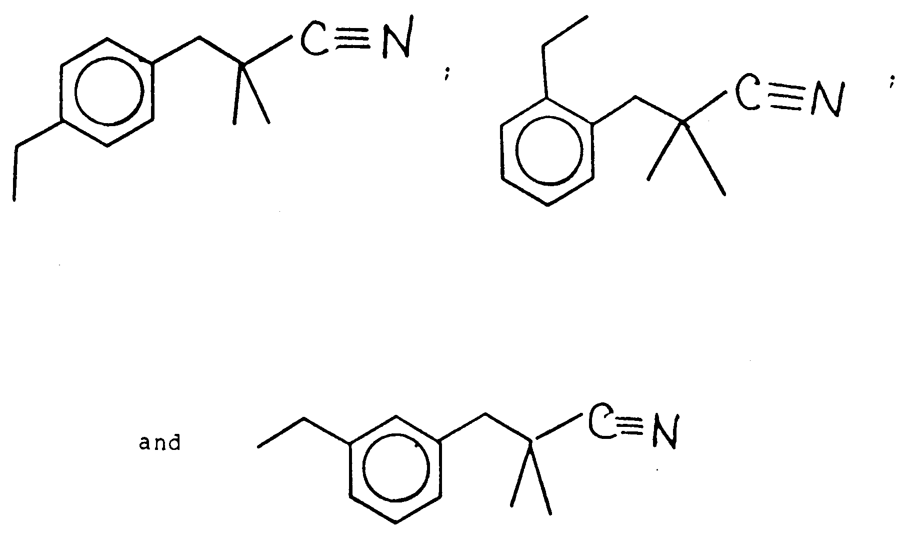
prepared according to Example II, bulked fractions 3-8 is added to 1 gram of n-undecyl dimethyl amine oxide to produce a clear premix. The clear premix is added to 200 grams of CLOROX® with stirring resulting in a clear stable single phase solution. Sufficient 1 M aqueous NaOH is added to bring the pH of the mixture to 12.8. The solution remains sustantially stable at 48.89°C (120°F) for a period of seven days. When the 5% aqueous sodium hypochlorite solution is used as a laundry bleach the resulting laundry on dry-out in an atmosphere of 65% relative humidity yields substantially no characteristic "hypochlorite" odor but does have a strong fresh ozoney, green and sweet vanilla aroma. Furthermore, no such characteristic "hypochlorite" aroma is retained on the hands of the individual handling such laundry in both the wet and the dry states.

### EXAMPLE XXIII

One drop of n-tridecyl dimethyl amine oxide is admixed with eight drops of a 50:50 mixture of the diisoamylene epoxide prepared according to Example I(B) of application for United States Letters Patent 4,335,009 issued June 15, 1982 and the mixture of compounds having the structures: 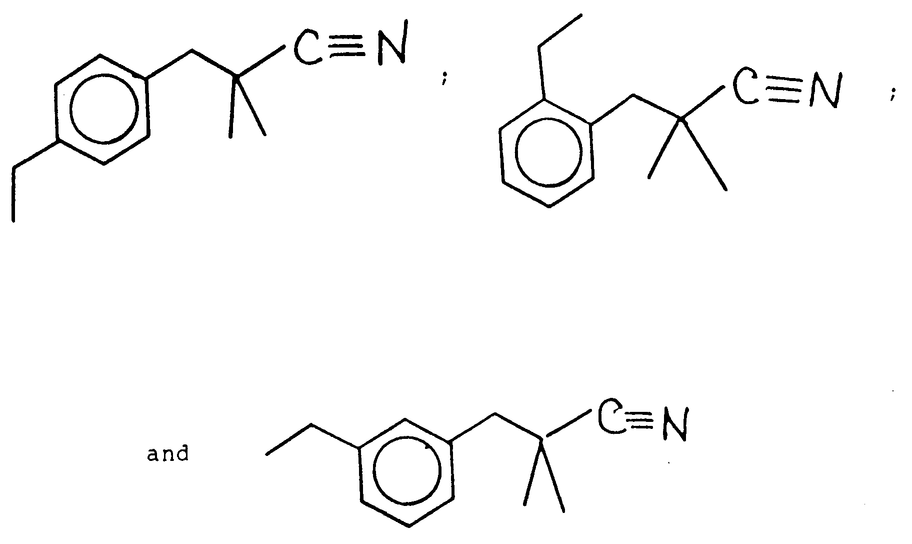
prepared according to Example II, bulked fractions 3-8, supra. This premix is then added with stirring to 200 grams of a 7% aqueous solution of lithium hypochlorite. Sufficient 3 M aqueous LiOH is added to bring the pH of the solution to 13.4. The mixture is then heated to 48.89°C (120°F) and maintained at that temperature with stirring for a period of one week. The resulting solution remains clear in a single phase. When used as a laundry bleach, the resulting bleached laundry on dry-out in an atmosphere of 50% relative humidity does have a strong fresh ozoney, green and sweet vanilla aroma; whereas without the use of the mixture of diisoamylene epoxide and the mixture of compounds having the structures: 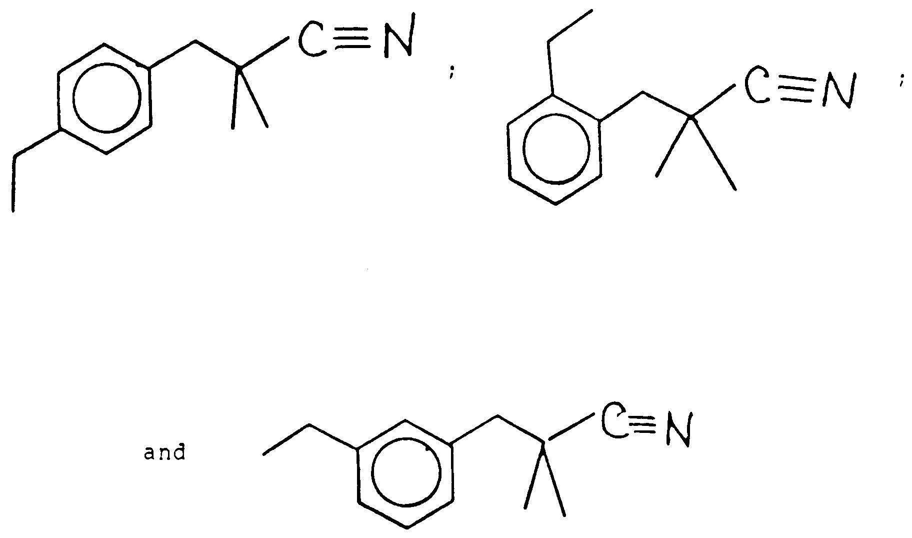
prepared according to Example II, bulked fractions 3-8, the bleached laundry has a faint characteristic disagreeable "hypochlorite" aroma.

### EXAMPLE XXIX

AROMOX® DMMC-W in various quantities is mixed with 0.1 gram of a 25:75 weight:weight mixture of diisoamylene epoxide:the mixture of compounds having the structures: 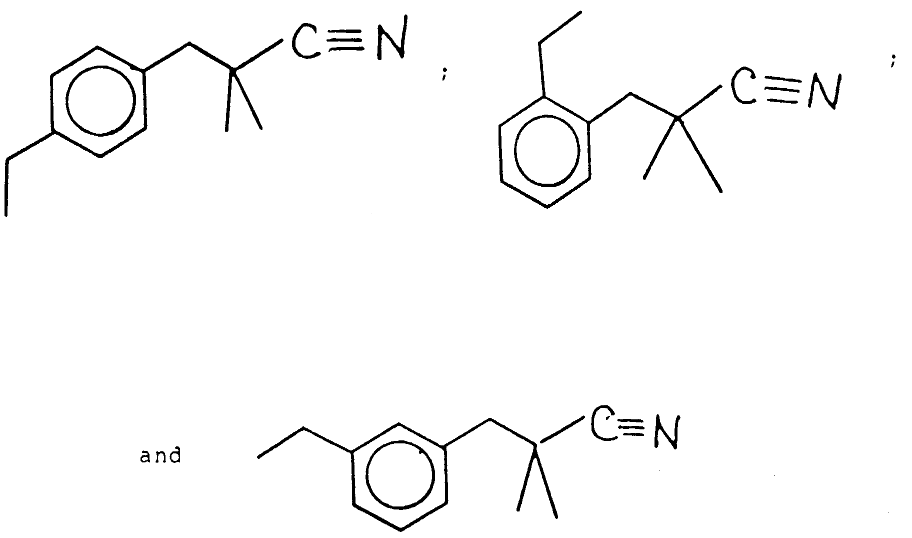
prepared according to Example II, bulked fractions 3-8. The resulting premixes are then added to 200 grams of an aqueous 5% sodium hypochlorite solution. Sufficient 12.5 M aqueous NaOH is added to bring the pH of the mixture up to 13. The following results are obtained:

| Percentage AROMOX® DMMC-W | Clarity of Hypochlorite Solution After Addition of Premix |
|---|---|
| 0.23% | Clear after three days. |
| 0.15% | Clear after three days. |
| 0.08% | Initially slightly turbid; two phases exist after three days. |

When used as laundry bleaches, the resulting bleached laundries on dry-out in an atmosphere of 50% relative humidity in each of the three cases above but does have a strong fresh ozoney, green and sweet vanilla aroma" whereas without the use of the composition of matter set forth above containing diisoamylene epoxide and the mixture of compounds having the structures: 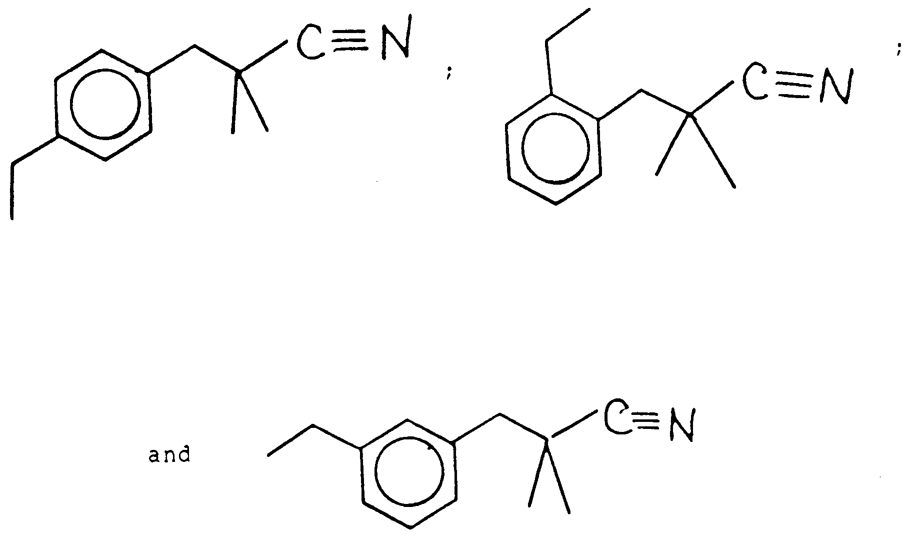
prepared according to Example II, bulked fractions 3-8, the bleached laundry has the same characteristic disagreeable "hypochlorite" aroma.

### EXAMPLE XXX

DOWFAX® 2A1 (see Note 1, infra) in various quantities, as set forth below, is mixed with 0.1 grams of a 50:50 mixture of (a) one of the diisoamylene epoxide compositions prepared according to Example II of application for United States Letters Patent 4,335,009 issued June 15, 1982 and (b) the mixture of compounds having the structures: 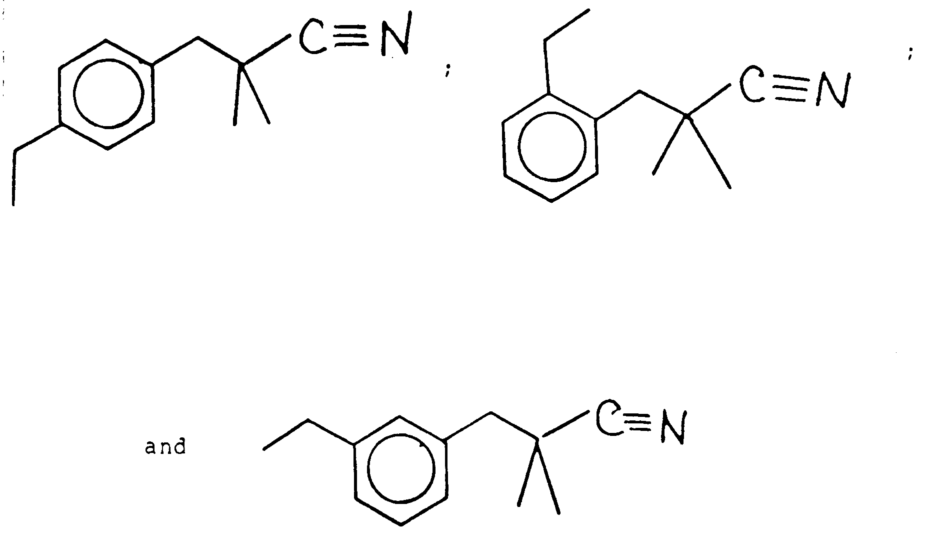
prepared according to Example II, bulked fractions 3-8. The resulting premixes are then added to 200 grams of an aqueous 7% sodium hypochlorite solution. Sufficient 12.5 M aqueous sodium hydroxide is added to bring the pH of the mixture up to 13.5. The following results are obtained:

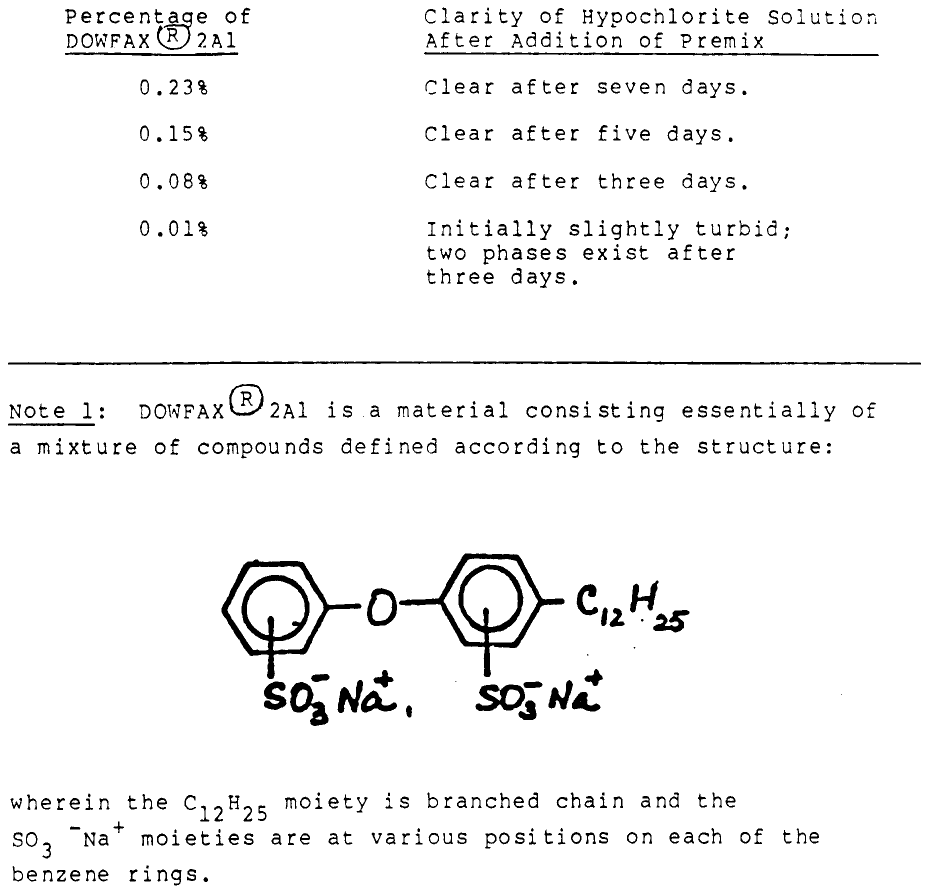

### EXAMPLE XXXI

DOWFAX® 3B2 (see Note 2, infra) in various quantities as set forth below, is mixed with 0.1 gram of the mixture of compounds having the structures: 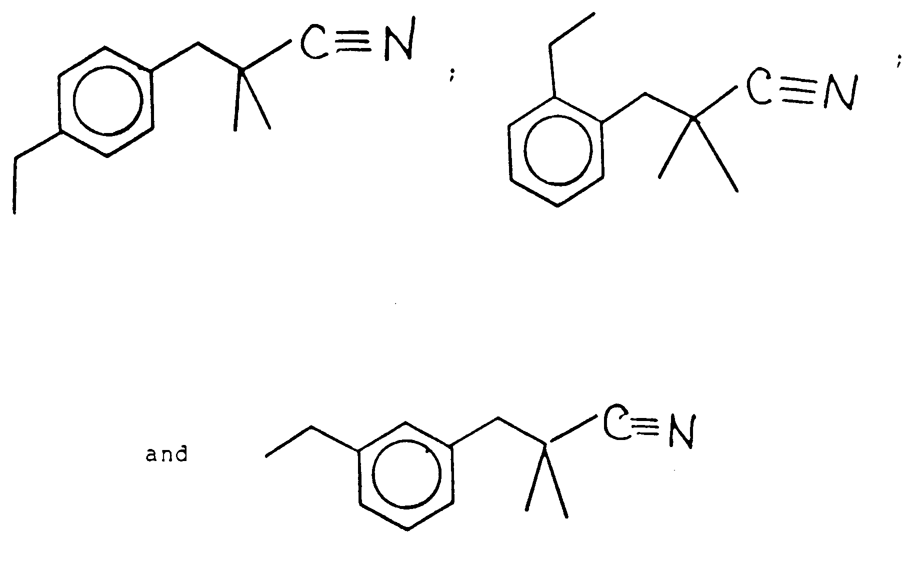
Note 2: DOWFAX® 3B2 is a mixture of compounds essentially defined according to the structure: 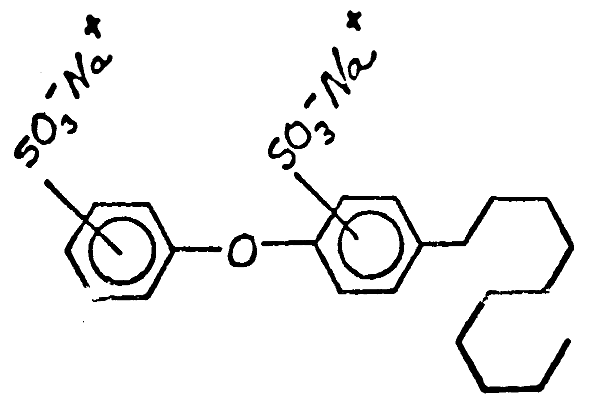
wherein the SO₃ ⁻Na⁺ moieties are at various positions on the phenyl moieties. DOWFAX® 3B2 is a registered trademark of the Dow Chemical Company of Midland, Michigan.

In the following examples, AROMOX® DMC-W and AROMOX® DMMC-W are 30% aqueous solutions of dimethyl cocoamine oxide; and AROMOX® NCMDW is a 40% aqueous solution of N-cocomorphloline oxide produced by Armac Division of Akzo of Chicago, Illinois prepared according to Example I(B). The resulting premixes are then added to 200 grams of an aqueous 7% sodium hypochlorite solution. Sufficient 12.5 M aqueous NaOH is added to bring the pH of the mixture up to 13.5. The following results are obtained:

| Percentage of DOWFAX® 3B2 | Clarity of Hypochlorite Solution After Addition of Premix |
|---|---|
| 0.23% | Clear after seven days. |
| 0.15% | Clear after five days. |
| 0.08% | Clear after three days. |
| 0.01% | Clear after three days. Initially slightly turbid; two phases exist after three days. |

When the 7% aqueous sodium hypochlorite solutions are used as laundry bleaches, the resulting laundry batches on dry-out in an atmosphere of 65% relative humidity yield substantially no characteristic "hypochlorite" odors but but does have a strong fresh ozoney, green and sweet vanilla aroma. Furthermore, no such characteristic "hypochlorite" aromas are retained on the hands of the individuals handling such laundry batches in both the wet and the dry states.

Four drops of a 25:75 weight/weight mixture of diisoamylene epoxide prepared according to Example II of application for United States Letters Patent 4,335,009 issued June 15, 1982 and the mixture of compounds having the structures: 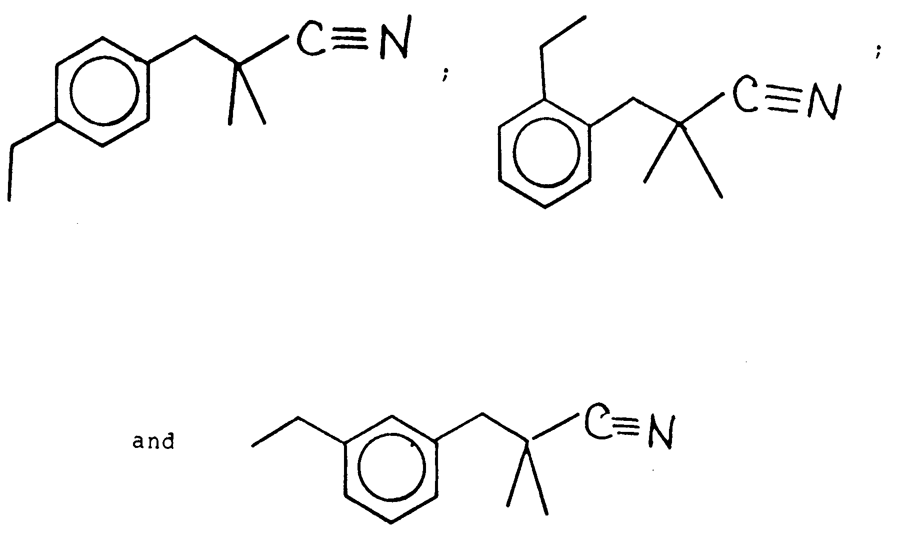
prepared according to Example II, bulked fractions 3-8, supra, is added to 2 grams of DOWFAX® 3B2 and 0.5 grams of AROMOX® DMC-W to produce a clear premix. The clear premix is added to 200 grams of CLOROX® with stirring resulting in a clear stable single phase solution. Sufficient 1 M aqueous NaOH is added to bring the pH of the mixture of 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a strong fresh ozoney, green and sweet vanilla aroma. Furthermore, no such characteristic "hypochlorite" aroma is retained on the hands of the individual handling such laundry in both the wet and the dry states.

### EXAMPLE XXXIII

One gram of DOWFAX® 3B2; one gram of DOWFAX® 2A1 and 0.25 grams of AROMOX® DMMC-W is admixed with eight drops of the mixture of compounds having the structures: 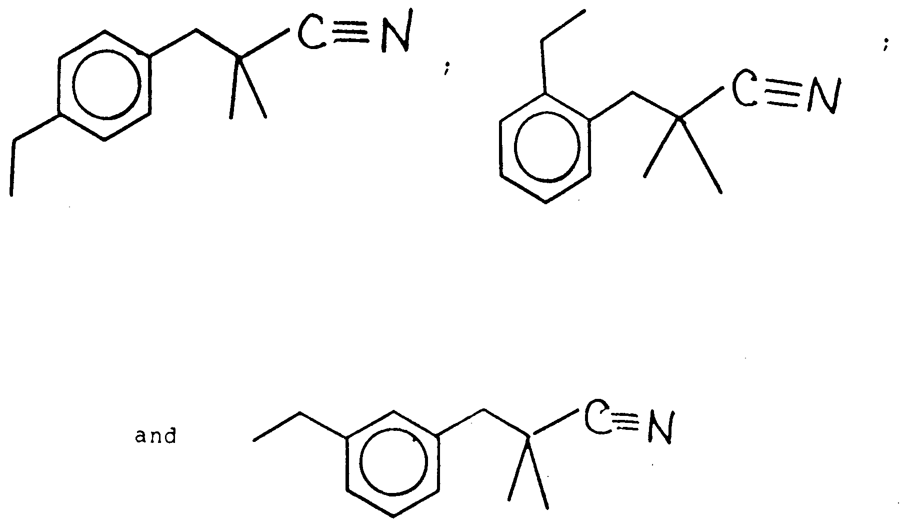
prepared according to Example II, bulked fractions 3-8. This premix is then added, with stirring to 200 grams of a mixture containing 4.5% aqueous sodium hypochlorite and 4.5% aqueous lithium hypochlorite. Sufficient 4 M aqueous LiOH is added to bring the pH of the solution to 13.4. The mixture is then heated to 48.89°C (120°F) and maintained at that temperature for a period of one week. The resulting solution remains clear in a single phase. When used as a laundry bleach, the resulting bleached laundry on dry-out in an atmosphere of 50% relative humidity but does have a strong fresh ozoney, green and sweet vanilla aroma; whereas without the use of the mixture of compounds having the structures: 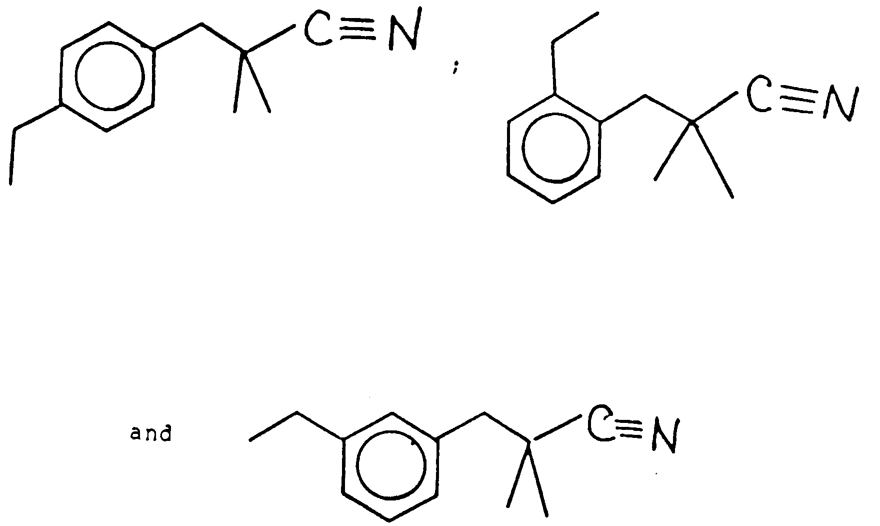
prepared according to Example II, bulked fractions 3-8, supra, the bleached laundry has a faint characteristic disagreeable "hypochlorite" aroma.

### EXAMPLE XXXIV

One gram of DOWFAX® 2A1 and one gram of DOWFAX® 3B2 is admixed with eight drops of a 50:50 mixture of one of the diisoamylene epoxide compositions of Example II of application for United States Letters Patent 4,335,009 issued June 15, 1982 and the mixture of compounds having the structures: 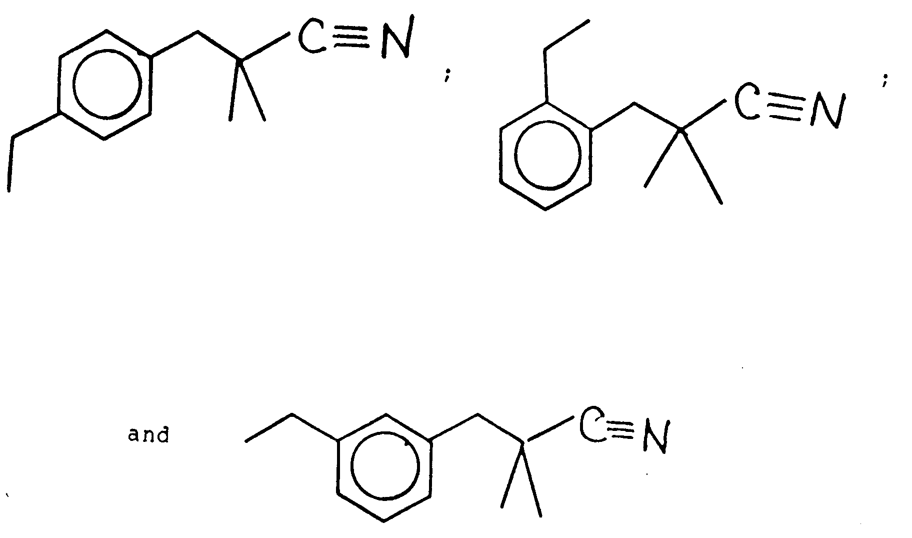
prepared according to Example II, bulked fractions 3-8. This premix is then added, with stirring to 200 grams of a mixture containing 4.5% aqueous sodium hypochlorite. Sufficient 4 M aqueous LiOH is added to bring the pH of the solution to 13.4. The mixture is then heated to 48.89°C (120°F) and maintained at that temperature for a period of one week. The resulting solution remains clear in a single phase. When used as a laundry bleach, the resulting bleached laundry on dry-out in an atmosphere of 50% relative humidity but does have a strong fresh ozoney, green and sweet vanilla aroma; whereas without the use of the perfume composition which is a mixture of diisoamylene epoxide and the mixture of compounds having the structures: 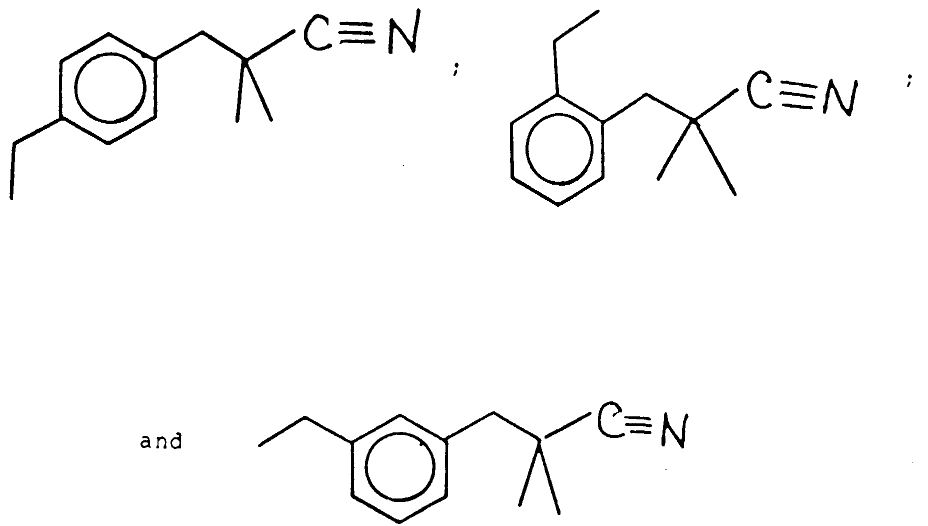
prepared according to Example II, bulked fractions 3-8, the bleached laundry has a faint characteristic disagreeable "hypochlorite" aroma.

### EXAMPLE XXXV

1.5 Grams of DOWFAX® 2A1 is admixed with eight drops of the mixture of compounds having the structures: 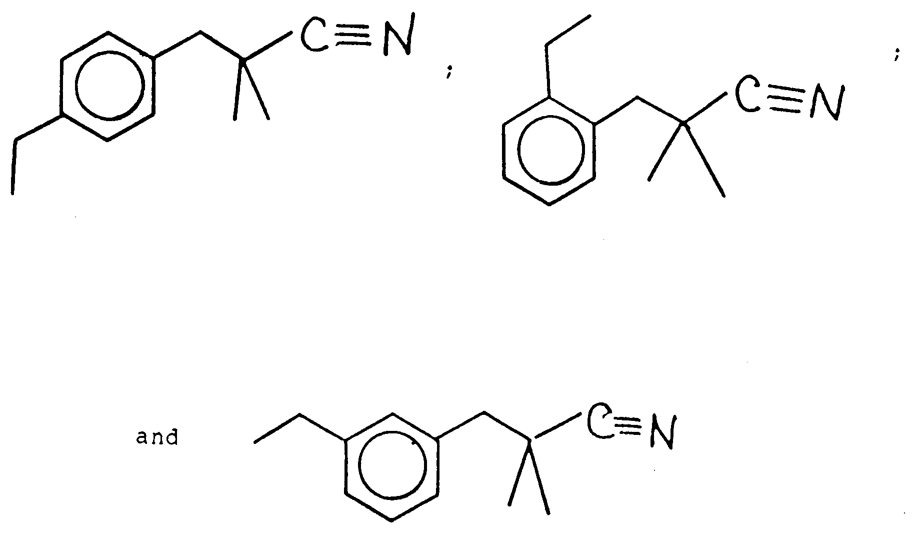
prepared according to Example II, bulked fractions 3-8, supra. This premix is then added with stirring to 200 grams of a mixture containing 4.5% aqueous sodiumn hypochlorite and 4.5% aqueous lithium hypochlorite. Sufficient 2 M aqueous NaOH is added to bring the pH of the solution to 13.4. The mixture is then heated to 43.33°C (110°F) and maintained at that temperature with stirring for a period of two weeks. The resulting solution remains clear as a single phase when used as a laundry bleach. The resulting bleached laundry on dry-out in an atmosphere of 50% relative humidity does have a strong fresh ozoney, green and sweet vanilla aroma, whereas without the use of the the mixture of compounds having the structures: 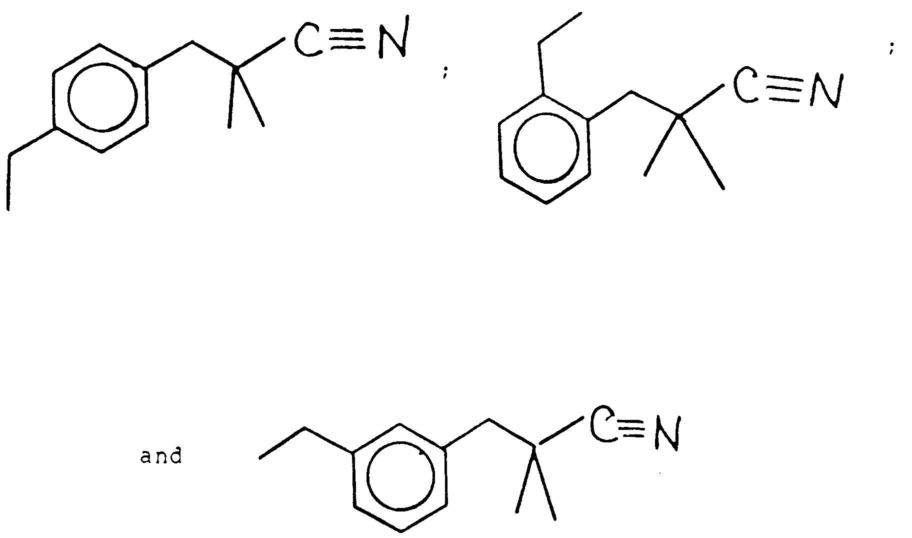
prepared according to Example II, bulked fractions 3-8, the bleached laundry has a faint characteristic disagreeable "hypochlorite" aroma.

### EXAMPLE XXXVI

Four drops of a 50:50 mixture of one of the diisoamylene epoxide mixtures produced according to Example II of application for United States Letters Patent 4,335,009 issued June 15, 1982 and the mixture of compounds having the structures: 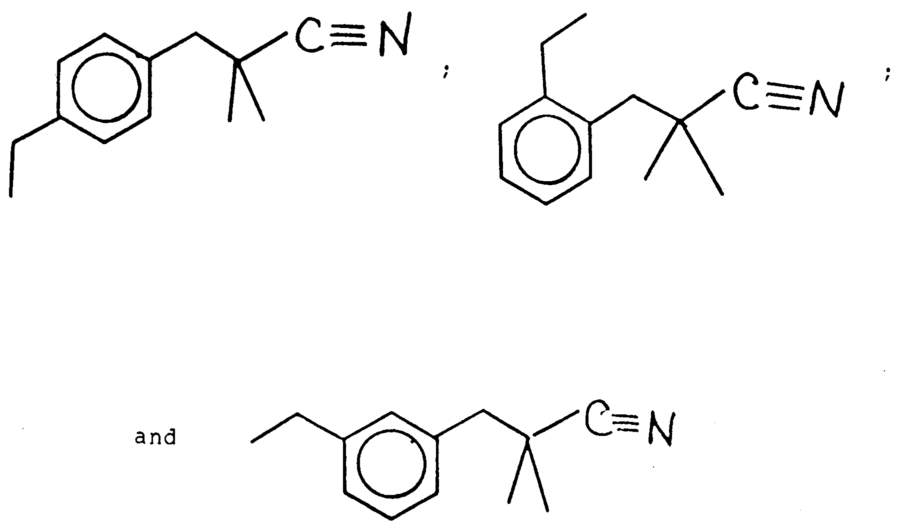
prepared according to Example II, bulked fractions 3-8, supra, is added to 1.0 grams of DOWFAX® 3B2 and 0.25 grams of AROMOX® NCMD-W to produce a clear premix. The clear premix is added to 200 grams of CLOROX® resulting in a clear stable single phase solution. Sufficient 1 M aqueous NaOH is added to bring the pH of the mixture up to 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields sustantially no characteristic "hypochlorite" odor, but does have a strong fresh ozoney, green and sweet vanilla aroma. Furthermore, no such characteristic "hypochlorite" aroma is retained on the hands of the individual handling such laundry in both the wet and the dry states.

### EXAMPLE XXXVII

### PREPARTION OF CANTHOXAL OXIME

### Reaction:

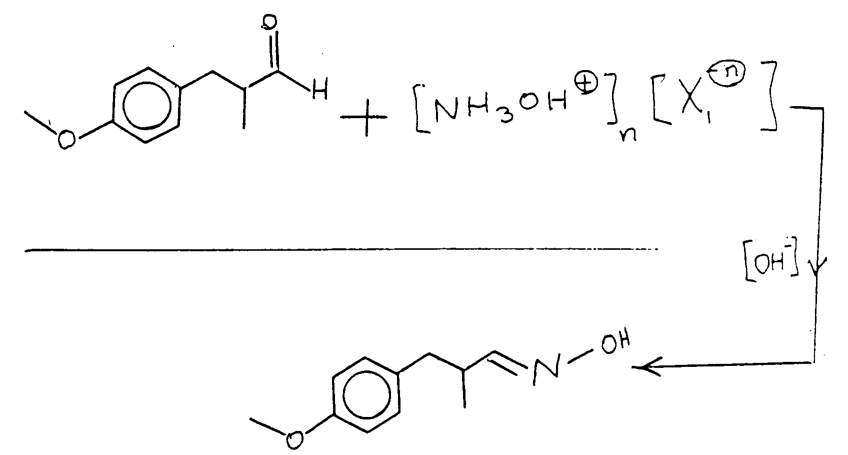

(wherein X is SO₄= and wherein n is 2).

Into a 5 liter reaction vessel equipped with stirrer, thermometer, heating mantle and addition funnel is placed 1500 ml water and 707 grams (4.3 moles) of hydroxylamine sulfate.

The hydroxylamine sulfate-water mixture is stirred until homogeneous while maintaining the temperature at 15°C. Over a period of 5 minutes while stirring the reaction mass and maintaining same at a temperature of 15°C, 1068 grams (6 moles) of the aldehyde having the structure: 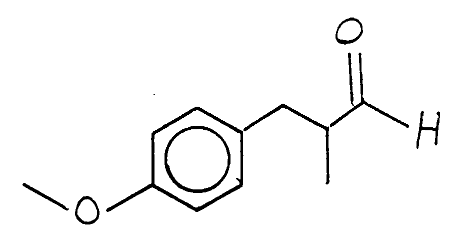
is added to the reaction mass.

791 Grams (9.89 moles) of sodium hydroxide in a 50% aqueous solution is then added to the reaction mass over a period of 1.5 hours while maintaining the reaction temperature in the range of 50-57°C.

The reaction mass is then aged at 55°C for a period of two hours.

The reaction mass is then allowed to cool to 40°C and the organic phase (product) precipitates as a solid. The solid is filtered and rinsed with three 1000 ml portions of water. The white filter cake is dissolved in methyl alcohol and refrigerated overnight. Crystals of the compound having the structure: 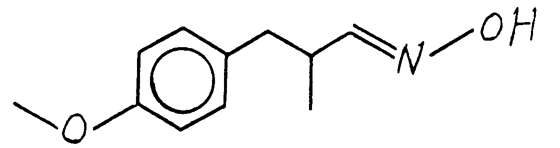
(650 grams) are then filtered from the product. The mother liquor is concentrated thereby causing additional compound having the structure: 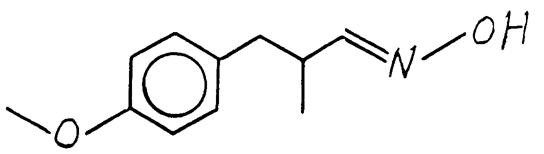
to precipitate the recrystallization of the second crop of crystals gives rise to 325 grams. The overall yield is 84%.

The resulting product has an intense and long-lasting anisic aroma with anisic topnotes.

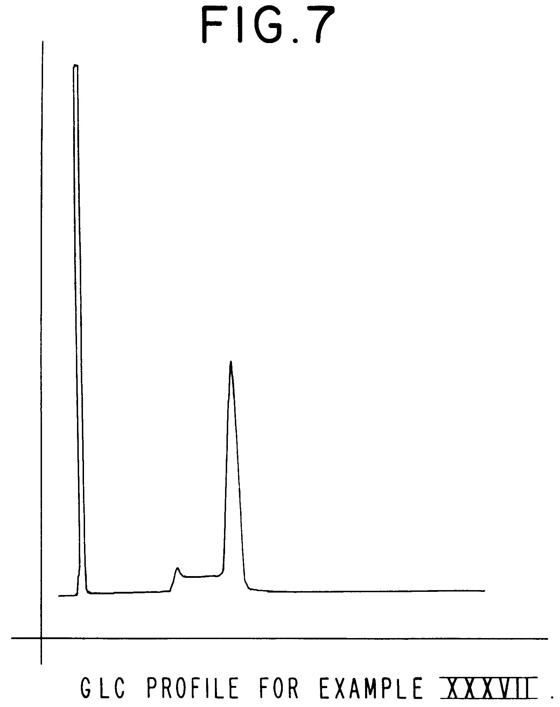

Figure 7 is the GLC profile for the reaction product prior to filtration.

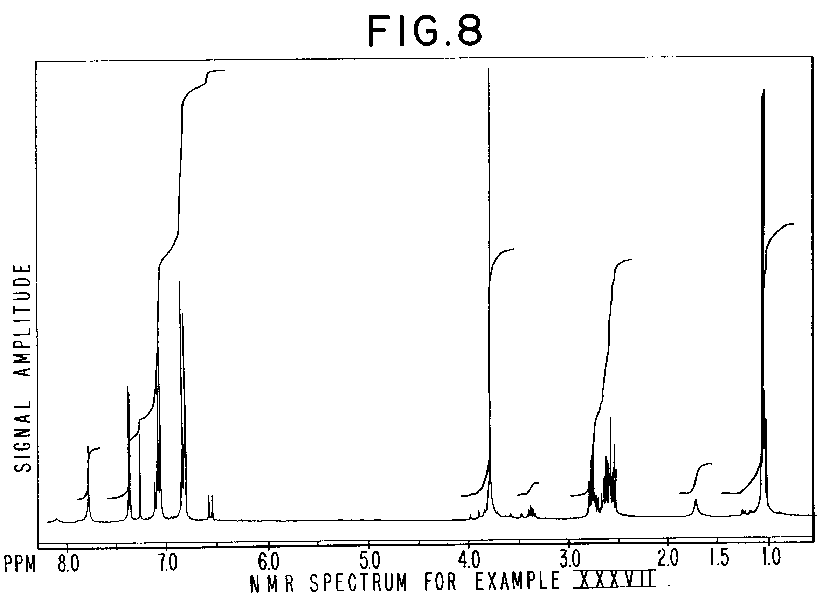

Figure 8 is the NMR spectrum for the compound having the structure:

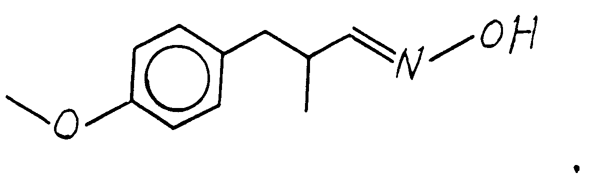

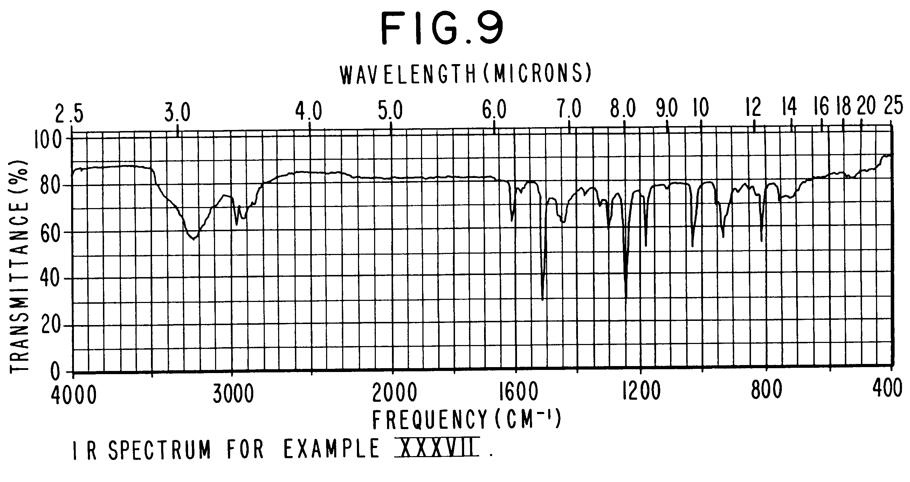

Figure 9 is the infrared spectrum for the compound having the structure:

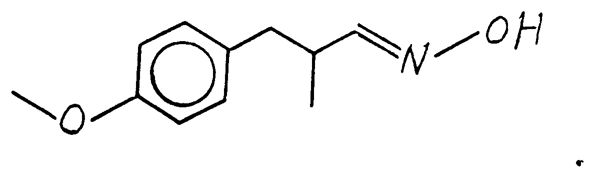

### EXAMPLE XXXVIII

### PREPARATION OF CANTHOXAL NITRILE (Not an embodiment of the invention)

### Reaction:

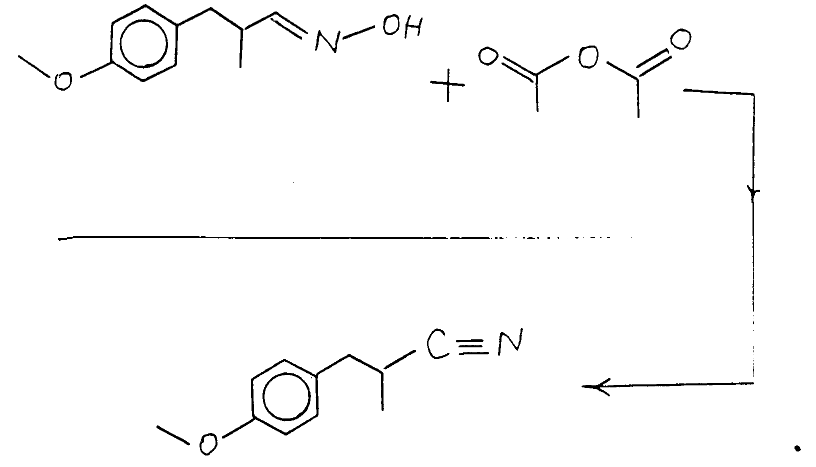

Into a 5 liter reaction flask equipped with thermometer, stirrer, reflux condenser and heating mantle are placed 917 grams of acetic anhydride (9 moles) and 8.1 grams of copper sulfate. The resulting mixture is heated to 100°C with stirring.

Over a 1.5 hour period while maintaining the reaction temperature at 98-100°C, 975 grams of the compound having the structure: 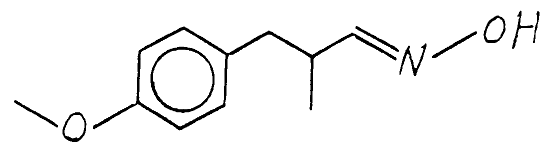
is added to the reaction mass in 10 gram portions.

The reaction mass is then aged with stirring for a period of 0.5 hours. The reaction mass is then quenched by means of the addition of 1 liter of water while maintaining the reaction mass temperature at 100°C. The reaction mass is then let stand overnight at room temperature. The reaction mass now exists in two phases; an aqueous phase and an organic phase. The organic phase is washed with 1000 ml water followed by two 400 ml portions of sodium bicarbonate solution (saturated) followed by two 500 ml portions of saturated sodium chloride solution.

The resulting product is filtered through anhydrous magnesium sulfate and fractionally distilled to yield the following fractions:

| Fraction No. | Vapor Temp. (°C) | Liquid Temp. (°C) | Vacuum mm/Hg. Pa Pressure |
|---|---|---|---|
| 1 | 85/126 | 131/139 | (1.87/2.43) 249.27/323.92 |
| 2 | 128 | 135 | (2.55) 339.92 |
| 3 | 127 | 135 | (2.35) 313.26 |
| 4 | 125 | 136 | (1.88) 250.60 |
| 5 | 125 | 138 | (1.88) 250.60 |
| 6 | 125 | 139 | (1.88) 250.60 |
| 7 | 127 | 138 | (1.92) 255.94 |
| 8 | 128 | 138 | (1.99) 265.27 |
| 9 | 130 | 138 | (2.14) 285.26 |
| 10 | 130 | 140 | (2.12) 282.60 |
| 11 | 130 | 140 | (2.12) 282.60 |
| 12 | 130 | 141 | (2.12) 282.60 |
| 13 | 130 | 141 | (2.12) 282.60 |
| 14 | 130 | 144 | (2.09) 278.60 |
| 15 | 130 | 144 | (2.10) 279.93 |
| 16 | 130 | 144 | (2.10) 279.93 |
| 17 | 132 | 145 | (2.20) 293.26 |
| 18 | 132 | 145 | (2.23) 297.26 |
| 19 | 134 | 159 | (2.39) 318.59 |
| 20 | 132 | 200 | (2.42). 322.59 |

Distillation fractions 10-18 are bulked. Bulked distillation fractions 10-18 have a sweet, anisic, animalic aroma with sweet and anisic topnotes.

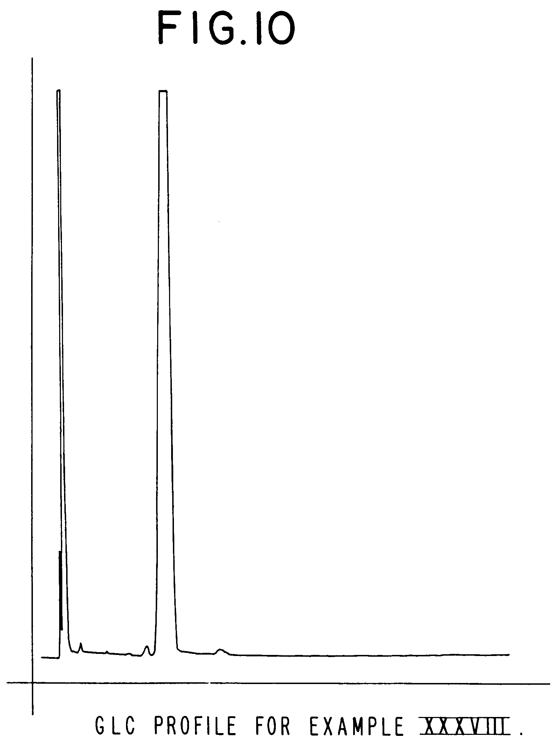

Figure 10 is the GLC profile for the reaction product prior to distillation (Conditions: SE-30 column programmed at 220°C isothermal).

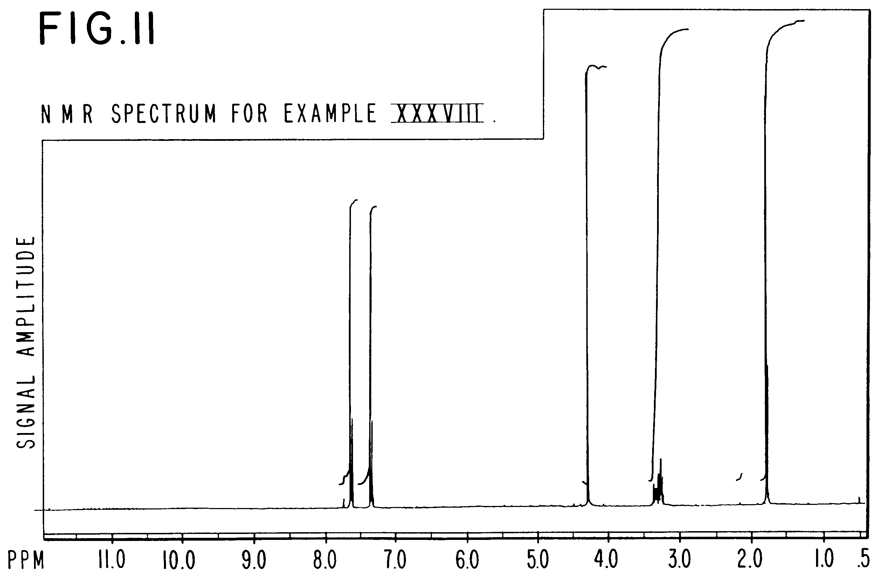

Figure 11 is the NMR spectrum for the compound having the structure:

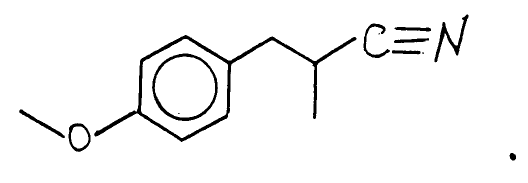

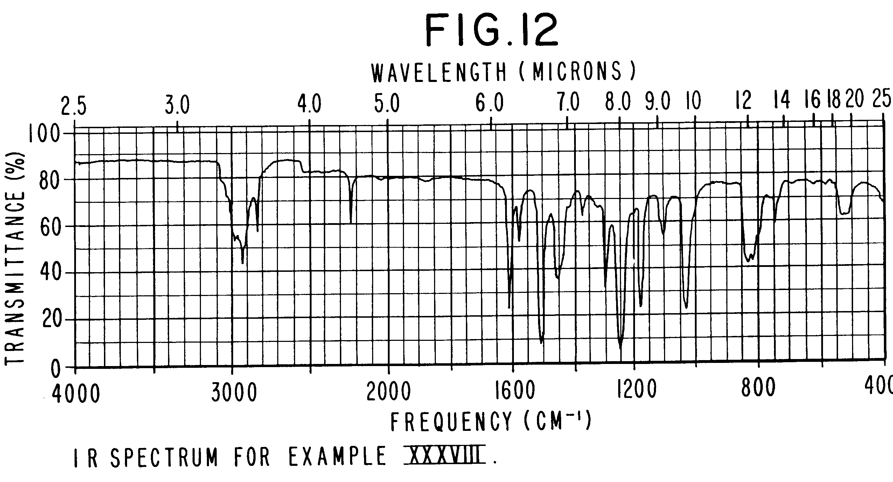

Figure 12 is the infra-red spectrum for the compound having the structure:

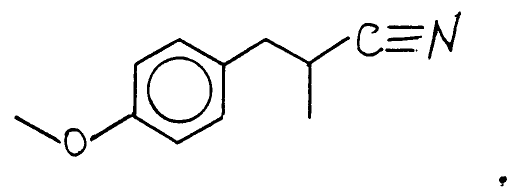

### EXAMPLE XXXIX

The following Acacia soap perfume formulations are prepared:

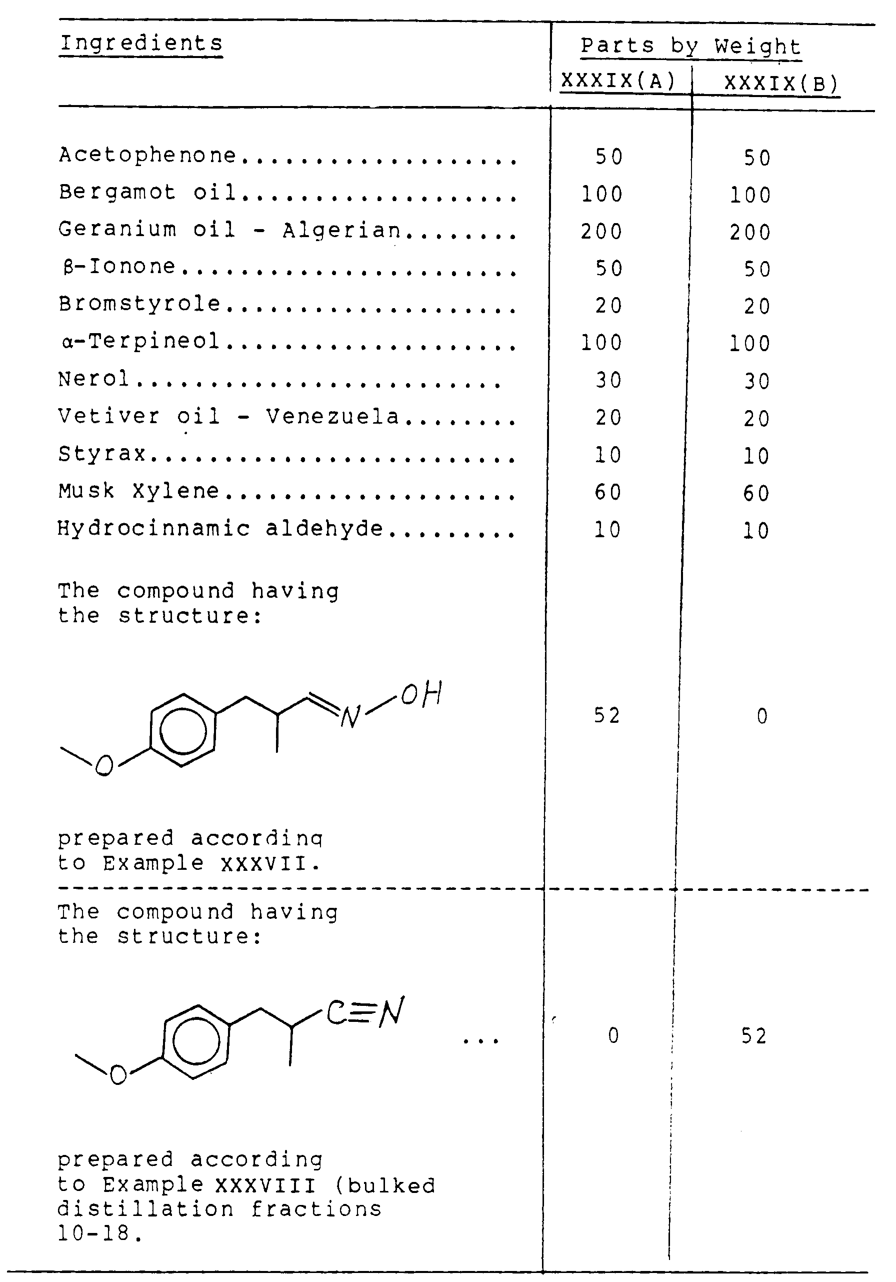

The resulting Acacia soap perfume of Example XXXIX(A) has an acacia, cassie and new mown hay aroma with anisic undertones and anisic topnotes, said anisic undertones and anisic topnotes being present as a result of the use of the compound having the structure: 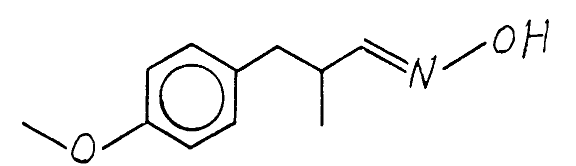
The resulting perfume formulation of Example XXXIX(B) has an acacia, cassie and new mown hay aroma with sweet, anisic topnotes and sweet, anisic and animalic undertones. The sweet, anisic topnotes and the sweet, anisic and animalic undertones are present in the formulation as a result of the addition of the compound having the structure:

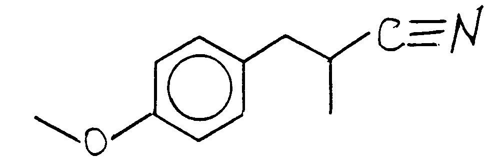

### EXAMPLE XL

### PREPARATION OF COSMETIC POWDER COMPOSITIONS

Cosmetic powder compositions are prepared by mixing in a ball mill 100 grams of talcum powder with 0.25 grams of each of the substances set forth in Table II below. Each of the cosmetic powder compositions has an excellent aroma as described in Table IV below:

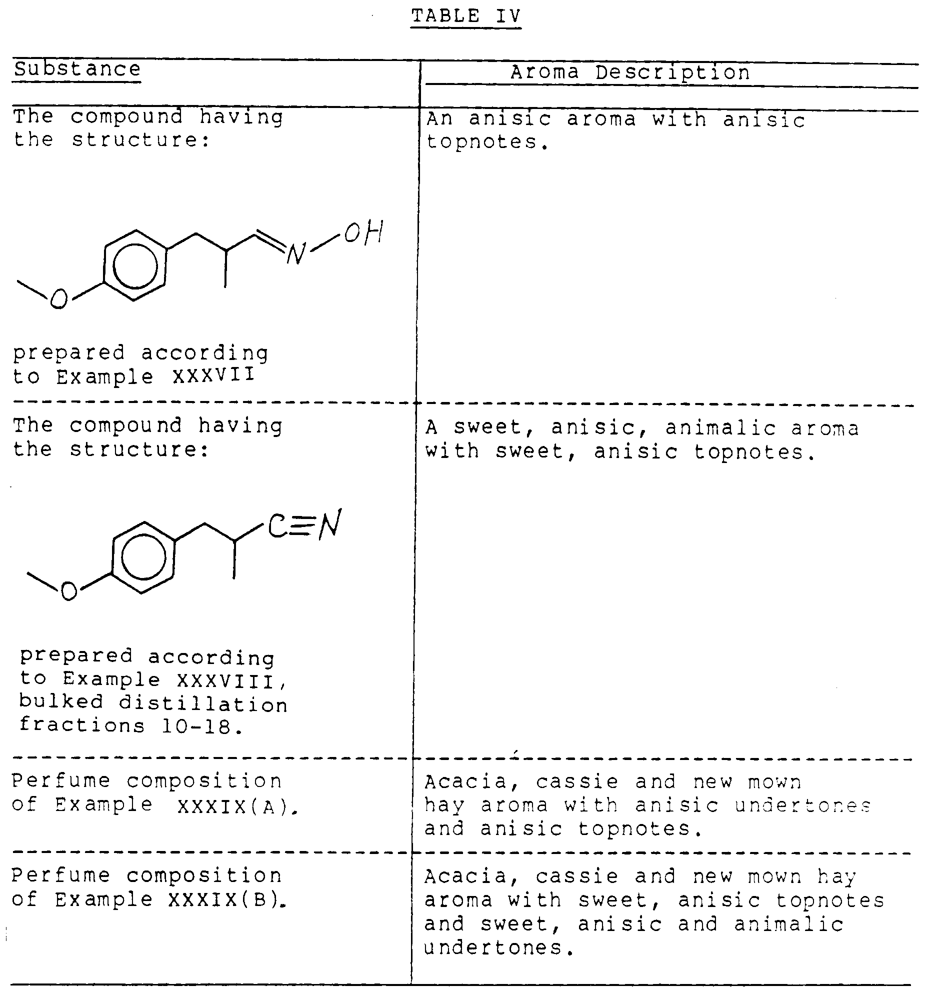

### EXAMPLE XLI

### PERFUMED LIQUID DETERGENTS

Concentrated liquid detergents (Lysine salt of n-dodecylbenzene sulfonic acid as more specifically described in U.S. Patent No. 3,948,818 issued on April 6, 1976) with aroma nuances as set forth in Table IV of Example XL are prepared containing 0.10%, 0.15%, 0.20%, 0.25%, 0.30% and 0.35% of the substance set forth in Table IV of Example XL. They are prepared by adding and homogeneously mixing the appropriate quantity of substance set forth in Table IV of Example XL in the liquid detergent. The detergents all possess excellent aromas as set forth in Table IV of Example XL, the intensity increasing with greater concentrations of substance as set forth in Table IV of Example XL.

### EXAMPLE XLII

### PREPARATION OF COLOGNES AND HANDKERCHIEF PERFUMES

Compositions as set forth in Table IV of Example XL are incorporated into colognes at concentrations of 2.0%, 2.5%, 3.0%, 3.5%, 4.0%, 4.5% and 5.0% in 80%, 85%, 90% and 95% aqueous food grade ethanol solutions; and into handkerchief perfumes at concentrations of 15%, 20%, 25% and 30% (in 80%, 85%, 90% and 95% aqueous food grade ethanol solutions). Distinctive and definitive fragrances as set forth in Table IV of Example XL are imparted to the colognes and to the handkerchief perfumes at all levels indicated.

### EXAMPLE XLIII

### PREPARATION OF SOAP COMPOSITIONS

One hundred grams of soap chips [per sample](IVORY®, produced by the Procter & Gamble Company of Cincinnati, Ohio), are each mixed with one gram samples of substances as set forth in Table IV of Example XL until homogeneous compositions are obtained. In each of the cases, the homogeneous compositions are heated under 8 atmospheres pressure at 180°C for a period of three hours and the resulting liquids are placed into soap molds. The resulting soap cakes, on cooling, manifest aromas as set forth in Table IV of Example XL.

### EXAMPLE XLIV

### PREPARATION OF SOLID DETERGENT COMPOSITIONS

Detergents are prepared using the following ingredients according to Example I of Canadian Patent No. 1,007,948:

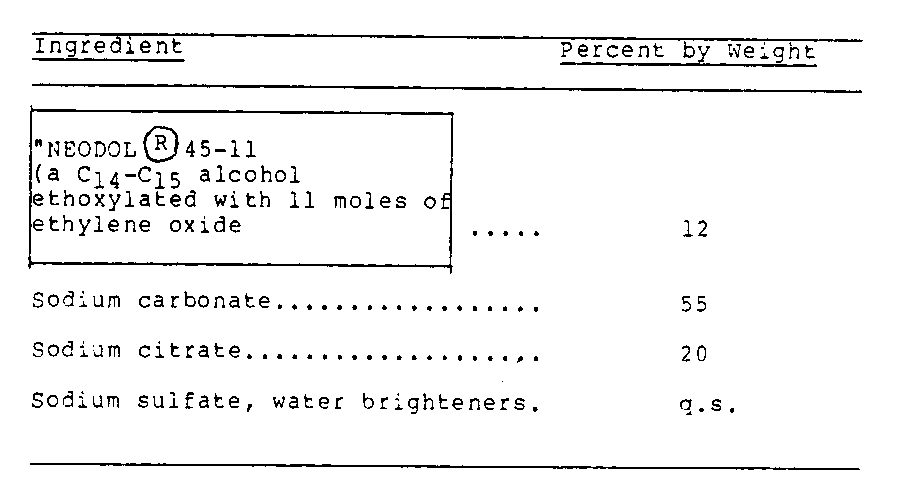

This detergent is a phosphate-free detergent. Samples of 100 grams each of this detergent are admixed with 0.10, 0.15, 0.20 and 0.25 grams of each of the substances as set forth in Table IV of Example XL. Each of the detergent samples has an excellent aroma as indicated in Table IV of Example XL.

### EXAMPLE XLV

Utilizing the procedure of Example I at column 15 of U.S. Patent No. 3,632,396, non-woven cloth substrates useful as drier-added fabric softening articles of manufacture are prepared wherein the substrate, the substrate coating and the outer coating and the perfuming material are as follows:
1. A water "dissolvable" paper ("Dissolvo Paper");
2. Adogen 448 (m.p. about 60°C (140°F)) as the substrate coating; and
3. An outer coating having the following formulation (m.p. about 65.56°C (150°F)):
   - 57% -: C₂₀₋₂₂ _{HAPS}
   - 22% -: isopropyl alcohol
   - 20% -: antistatic agent
   - 1% -: of one of the substances as set forth in Table IV of Example XL.

Fabric softening compositions prepared according to Example I at column 15 of U.S. Patent No. 3,632,396 having aroma characteristics as set forth in Table IV of Example XL, consist of a substrate coating having a weight of about 3 grams per 645.16 cm² (100 square inches) of substrate; a first coating on the substrate coating consisting of about 1.85 grams per 645.16 cm² (100 square inches) of substrate; and an outer coating on the first coating consisting of about 1.4 grams per 645.16 cm² (100 square inches) of substrate. One of the substances of Table IV of Example XL is admixed in each case with the outer coating mixture, thereby providing a total aromatized outer coating weight ratio to substrate of about 0.5:1 by weight of the substrate. The aroma characteristics are imparted in a pleasant manner to the head space in a dryer on operation thereof in each case using said dryer-added fabric softener non-woven fabrics and these aroma characteristics are described in Table IV of Example XL.

### EXAMPLE XLVI

### CONDITIONING SHAMPOOS

The following hair spray formulation is prepared by first dissolving PVP/VA E-735 copolymer manufactured by the GAF Corporation of 140 West 51st Street, New York, New York, in 91.62 grams of 95% food grade ethanol, 8.0 grams of the polymer is dissolved in the alcohol. The following ingredients are added to the PVP/VA alcoholic solution:

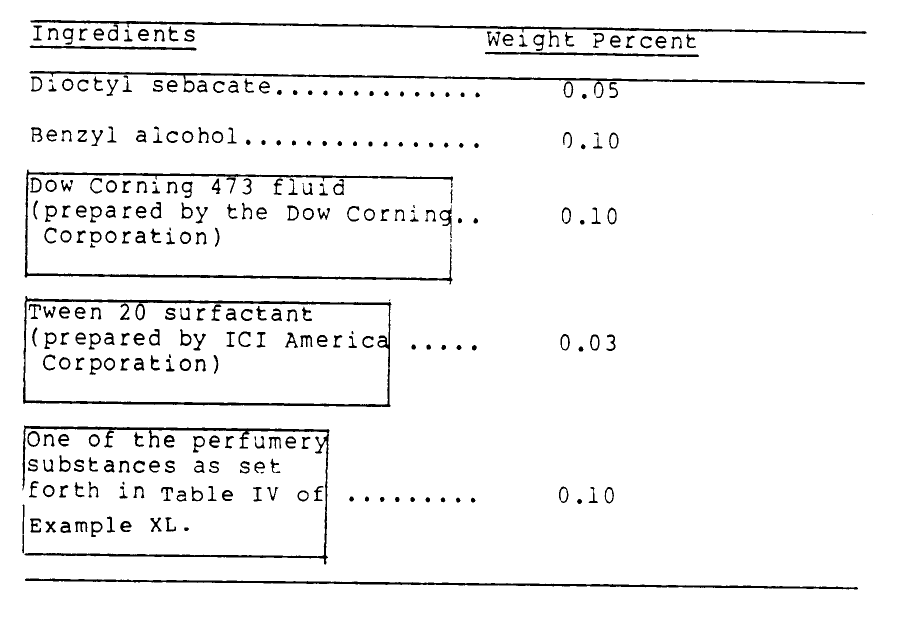

The perfuming substances as set forth in Table IV of Example XL add aroma characteristics as set forth in Table IV of Example XL which are rather intense and aesthetically pleasing to the users of the soft-feel, good-hold pump hair sprays.

### EXAMPLE XLVII

### CONDITIONING SHAMPOOS

Monamid CMA (prepared by the Mona Industries Company)(3.0 weight percent) is melted with 2.0 weight percent coconut fatty acid (prepared by Procter & Gamble Company of Cincinnati, Ohio); 1.0 weight percent ethylene glycol distearate (prepared by the Armak Corporation) and triethanolamine (a product of Union Carbide Corporation)(1.4 weight percent). The resulting melt is admixed with Stepanol WAT produced by the Stepan Chemical Company (35.0 weight percent). The resulting mixture is heated to 60°C and mixed until a clear solution is obtained (at 60°C).

GAFQUAT® 755N polymer (manufactured by GAF Corporation of 140 West 51st Street, New York, New York)(5.0 weight percent) is admixed with 0.1 weight percent sodium sulfite and 1.4 weight percent polyethylene glycol 6000 distearate produced by Armak Corporation.

The resulting material is then mixed and cooled to 45°C and 0.3 weight percent of perfuming substance as set forth in Table IV of Example XL is added to the mixture. The resulting mixture is cooled to 40°C and blending is carried out for an additional one hour in each case. At the end of this blending period, the resulting material has a pleasant fragrance as indicated in Table IV of Example XL.

### EXAMPLE XLVIII

Four drops of each of the substances set forth in Table IV of Example XL, supra, is added separately to two grams of AROMOX® DMC-W to produce a clear premix. The clear premix is added to 200 grams of CLOROX® with stirring resulting in a clear stable, single phase solution. Sufficient 1 M aqueous NaOH is added to bring the pH of the mixture up to 12.8. The solution remains substantially stable at 48.89°C (120°F) for a period of seven days. When the 5% aqueous sodium hypochlorite solution is used as a laundry bleach, the resulting laundry, on dry-out in an atmosphere of 65% relative humidity yields substantially no characteristic "hypochlorite" odor, but does have a faint pleasant aroma as set forth in Table IV of Example XL. Furthermore, no such characteristic "hypochlorite" aroma is retained on the hands of the individual handling such laundry in both the wet and the dry states.

### EXAMPLE XLVIX

AROMOX® DMMC-W in various quantities is mixed with 0.1 grams of one of the substances set forth in Table IV of Example XL, supra. The resulting premixes are then added to 200 grams of an aqueous 5% sodium hypochlorite solution. Sufficient 12.5 M aqueous NaOH is added to bring the pH of the mixture up to 13. The following results are obtained:

| Percentage AROMOX® DMMC- W | Clarity of hypochlorite solution after addition of premix |
|---|---|
| 0.23% | Clear after three days |
| 0.15% | Clear after three days |
| 0.08% | Initially slightly turbid; two phases exist after three days. |

When the 5% aqueous sodium hypochlorite solution is used as a laundry bleach, the resulting laundry on dry-out, in an atmosphere of 65% relative humidity, yields substantially no characteristic "hypochlorite" odor, but does have a faint, pleasant aroma as set forth in Table IV of Example XL. Furthermore, no such characteristic "hypochlorite" aroma is retained on the hands of the individual handling such laundry in both the wet and dry states.

### EXAMPLE L

Two grams of AROMOX® DMMC-W is admixed with eight drops of one of the substances set forth in Table IV of Example XL, supra. The premix is then added with stirring to 200 grams of a 7% aqueous solution of lithium hypochlorite. Sufficient 3 M aqueous LiOH is added to bring the pH of the solution to 13.4. The mixture is then heated to 48.89°C (120°F) and maintained at that temperature with stirring for a period of one week. The resulting solution remains clear in a single phase. When used as a laundry bleach, the resulting bleached laundry, on dry-out in an atmosphere of 50% relative humidity retains a "clean" warm aroma as set forth in Table IV of Example XL, supra; whereas without the use of the substance set forth in Table IV of Example XL, supra, the bleached laundry has a faint characteristic disagreeable "hypochlorite" aroma.

### EXAMPLE LI

Two grams of AROMOX® DMMC-W is admixed with eight drops of one of the substances of Table IV of Example XL, supra. This premix is then added, with stirring to 200 grams of a mixture containing 4.5% aqueous sodium hypochorite. Sufficient 4 M aqueous LiOH is added to bring the pH of the solution to 13.4. The mixture is then heated to 48.89°C (120°F) and maintained at that temperature for a period of one week. The resulting solution remains clear in a single phase. When used as a laundry bleach, the resulting bleached laundry on dry-out in an atmosphere of 50% relative humidity retains a "clean fresh" warm aroma as set forth in Table IV of Example XL, supra; whereas without the use of the substance set forth in Table IV of Example XL, supra, the bleached laundry has a faint characteristic disagreeable "hypochlorite" aroma.

### EXAMPLE LII

Two grams of AROMOX® DMMC-W is admixed with eight drops of one of the substances as set forth in Table IV of Example XL, supra. This premix is then added with stirring to 200 grams of a mixture containing 4.5% aqueous sodium hypochlorite and 4.5% aqueous lithium hypochlorite. Sufficient 2 M aqueous NaOH is added to bring the pH of the solution to 13.4. The mixture is then heated to 43.33°C (110°F) and maintained at that temperature with stirring for a period of 2 weeks. The resulting solution remains clear as a single phase when used as a laundry bleach. The resulting laundry bleach, on dry-out in an atmosphere of 50% relative humidity, retains an aroma as set forth in Table IV of Example XL, supra, whereas without the use of the substance set forth in Table IV of Example XL, supra, the bleached laundry has a faint characteristic disagreeable "hypochlorite" aroma.

### EXAMPLE LIII

Four drops of one of the substances set forth in Table IV of Example XL, supra, is added to 1.5 grams of AROMOX® to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 48.89°C (120°F) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant warm, long-lasting aroma as set forth in Table IV of Example XL, supra. Furthermore, no such characteristic "hypochlorite" aroma is retained on the hands of the individual handling such laundry in both the wet and dry states.

### EXAMPLE LIV

Four drops of one of the substances set forth in Table IV of Example XL, supra, is added to 1 gram n-undecyl dimethyl amine oxide to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 48.89°C (120°F) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant warm aroma as set forth in Table IV of Example XL, supra. Furthermore, no such characteristic "hypochlorite" aroma is retained on the hands of the individual handling such laundry in both the wet and the dry states.

### EXAMPLE LV

Four drops of one of the substances as set forth in Table IV of Example XL, supra are added to 1 gram of n-dododecyl dimethyl amine oxide to produce a clear premix. The clear premix is added to 200 grams of CLOROX® with stirring resulting in a clear, stable single phase solution. Sufficient 1 M aqueous NaOH is added to bring the pH of the mixture up to 12.8. The solution remains substantially stable at 48.89°C (120°F) for a period of 7 days. When the 5% aqueous sodium hypochlorite solution is used as a laundry bleach, the resulting laundry on dry-out in an atmosphere of 65% relative humidity yields substantially no characteristic "hypochlorite" aroma, but does have a warm, pleasant, long-lasting aroma as set forth in Table IV of Example XL, supra. Furthermore, no such characteristic "hypochlorite" aroma is retained on the hands of the individual handling such laundry in both the wet and dry states.

### EXAMPLE LVI

One gram of n-tridecyl dimethyl amine oxide is admixed with eight drops of one of the substances as set forth in Table IV of Example XL, supra. This premix is then added with stirring to 200 grams of a 7% aqueous solution of lithium hypochlorite. Sufficient 3 M aqueous LiOH is added to bring the pH of the solution to 13.4. The mixture is then heated to 48.89°C (120°F) and maintained at that temperature with stirring for a period of one week. The resulting solution remains clear in a single phase. When used as a laundry bleach, the resulting bleached laundry on dry-out in an atmosphere of 50% relative humidity retains a warm, fresh aroma described in Table IV of Example XL, supra, whereas without the use of one of the substances of Table IV of Example XL, supra, the bleached laundry has a faint characteristic disagreeable "hypochlorite" aroma.

### EXAMPLE LVII

Four drops of the compound having the structure: 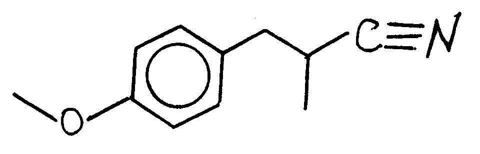
prepared according to Example II (bulked fractions 10-18) is added to 2 grams of AROMOX® DMC-W to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a sweet, anisic and animalic aroma. Furthermore, no such characteristic "hypochlorite" aroma is retained on the hands of the individual handling such laundry in both the wet and the dry states.

### EXAMPLE LVIII

AROMOX® DMMC-W in various quantities is mixed with 0.1 grams of the compound having the structure: 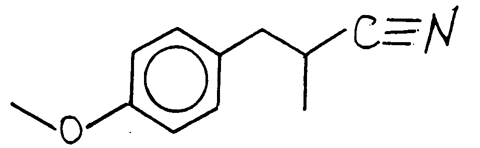
prepared according to Example XXXVIII (bulked fractions 10-18). The resulting premix is then added to 200 grams of an aqueous 5% sodium hypochlorite solution. Sufficient 12.5 M aqueous NaOH is added to bring the pH of the mixture up to 13. The following results are obtained:

| Percentage AROMOX® DMMC-W | Clarity of Hypochlorite Solution After Addition of Premix |
|---|---|
| 0.23% | Clear after three days. |
| 0.15% | Clear after three days. |
| 0.08% | Initially slightly turbid; two phases exist after three days. |

When the 5% aqueous sodium hypochlorite solutions are used as laundry bleaches, the resulting laundry batches on dry-out in an atmosphere of 65% relative humidity yields substantially no characteristic "hypochlorite" odor, but does have a sweet, anisic and animalic aroma. Furthermore, no such characteristic "hypochlorite" aroma is retained on the hands of the individual handling such laundry batches in both the wet and the dry states.

### EXAMPLE LVIX

Two grams of AROMOX® DMMC-W are admixed with eight drops of the compound having the structure: 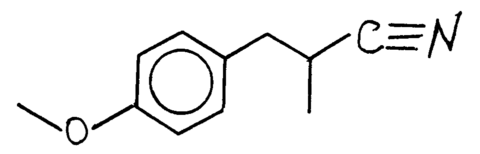
prepared according to Example XXXVIII (bulked fractions 10-18). The premix is then added with stirring to 200 grams of a 7% aqueous, solution of lithium hypochlorite. Sufficient 3 M aqueous LiOH is added to bring the pH of the solution to 13.4. The mixtures are then heated to 48.89°C (120°F) and maintained at that temperature with stirring for a period of one week. The resulting solution remains clear in a single phase. When used as laundry bleaches, the resulting bleached laundry batches on dry-out in an atmosphere of 50% relative humidity retain a sweet, anisic and animalic aroma profile whereas without the use of the the compound having the structure: 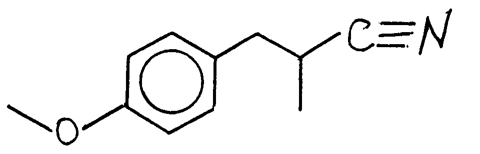
prepared according to Example XXXVIII (bulked fractions 10-18), the bleached laundry batches have a faint characteristic disagreeable "hypochlorite" aroma.

### EXAMPLE LX

Two grams of AROMOX® DMMC-W are admixed with eight drops of the compound having the structure: 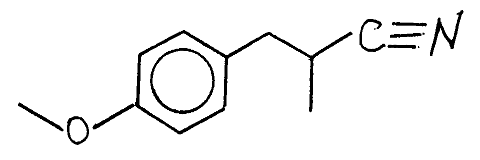
prepared according to Example XXXVIII (bulked fractions 10-18). The premix is then added with stirring to 200 grams of a mixture premix is then added with stirring to 200 grams of a mixture containing 4.5% aqueous sodium hypochlorite and 4.5% aqueous lithium hypochlorite. Sufficient 4 M aqueous LiOH is added to bring the pH of the solution to 13.4. The mixture is then heated to 48.89°C (120°F) and maintained at that temperature for a period of one week, The resulting solution remains clear in a single phase. When used as a laundry bleach, the resulting bleached laundry batches on dry-out in an atmosphere of 50% relative humidity retain a sweet, anisic and animalic aroma profile whereas without the use of the compound having the structure: 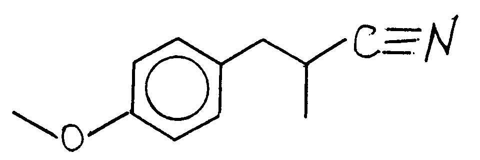
prepared according to Example XXXVIII (bulked fractions 10-18), the bleaded laundry batches have faint characteristic disagreeable "hypochlorite" aroma.

### EXAMPLE LXI

Two grams of AROMOX® DMMC-W are admixed with eight drops of either (a) the compound having the structure: 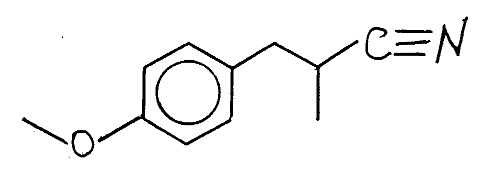
prepared according to Example XXXVIII (bulked fractions 10-18); or (b) a 50-50 of the compound having the structure: 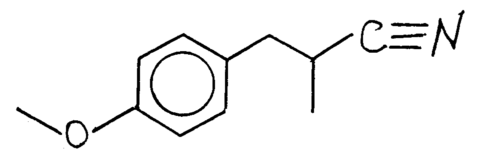
prepared according to Example XXXVIII, bulked fractions 10-18 and diisoamylene epoxide produced according to Example II of application for United States Letters Patent 4,335,009 issued June 15, 1982 the disclosure of which is incorporated herein by reference. These premixes are then added with stirring to 200 grams of a mixture containing 4% aqueous sodium hypochlorite and 4% aqueous lithium hypochlorite. Sufficient 2 M aqueous NaOH is added to bring the pH of the solutions to 13.4. The mixtures are then heated to 43.33°C (110°F) and maintained at that temperature with stirring for a period of two weeks. The resulting solutions remain clear as a single phase when used as laundry bleaches. The resulting bleached laundry batches on dry-out in an atmosphere of 50% relative humidity have a sweet, anisic and animalic aroma (when using the compound having the structure: 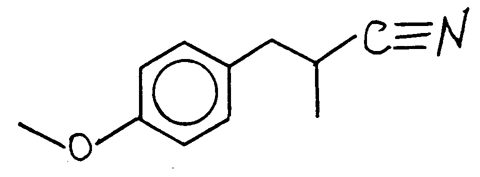
prepared according to Example XXXVIII, bulked fractions 10-18 have a sweet, anisic and animalic aroma when using the mixture of the diisoamylene epoxide and the compound having the structure: 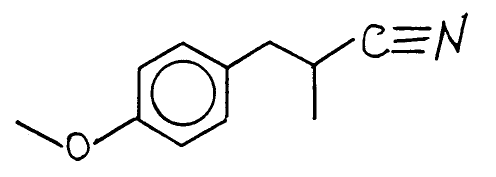
prepared according to Example XXXVIII, bulked fractions 10-18; whereas without the use of the compound having the structure: 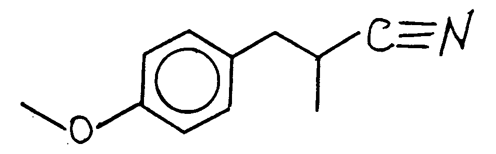
prepared according to Example XXXVIII, bulked fractions 10-18 containing compositions of matter the bleached laundry batches have faint characteristic disagreeable "hypochlorite" aromas.

### EXAMPLE LXII

Four drops of the compound having the structure: 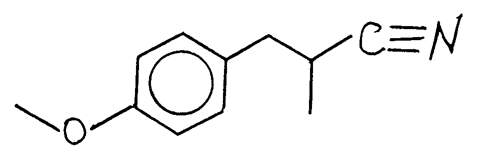
prepared according to Example XXXVIII, bulked fractions 10-18 are added to 1.5 grams of AROMOX® NCMD-W to produce a clear premix. The clear premix is added to 200 grams of CLOROX® with stirring resulting in a clear stable single phase solution. Sufficient 1M aqueous NaOH is added to bring the pH of the mixture up to 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a sweet, anisic and animalic aroma. Furthermore, no such characteristic "hypochlorite" aroma is retained on the hands of the individual handling such laundry in both the wet and the dry states.

### EXAMPLE LXIII

Four drops of the compound having the structure: 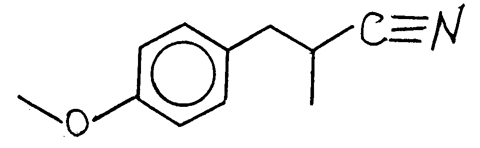
prepared according to Example XXXVIII, bulked fractions 10-18 is added to 1 gram of n-undecyl dimethyl amine oxide to produce a clear premix. The clear premix is added to 200 grams of CLOROX® with stirring resulting in a clear stable single phase solution. Sufficient 1 M aqueous NaOH is added to bring the pH of the mixture to 12.8. The solution remains sustantially stable at 48.89°C (120°F) for a period of seven days. When the 5% aqueous sodium hypochlorite solution is used as a laundry bleach the resulting laundry on dry-out in an atmosphere of 65% relative humidity yields substantially no characteristic "hypochlorite" odor but does have a sweet, anisic and animalic aroma. Furthermore, no such characteristic "hypochlorite" aroma is retained on the hands of the individual handling such laundry in both the wet and the dry states.

### EXAMPLE LXIV

One drop of n-tridecyl dimethyl amine oxide is admixed with eight drops of a 50:50 mixture of the diisoamylene epoxide prepared according to Example I(B) of application for United States Letters Patent 4,335,009 issued June 15, 1982 and the compound having the structure: 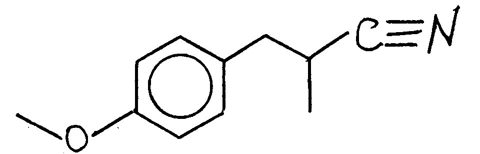
prepared according to Example XXXVIII, bulked fractions 10-18, supra. This premix is then added with stirring to 200 grams of a 7% aqueous solution of lithium hypochlorite. Sufficient 3 M aqueous LiOH is added to bring the pH of the solution to 13.4. The mixture is then heated to 48.89°C (120°F) and maintained at that temperature with stirring for a period of one week. The resulting solution remains clear in a single phase. When used as a laundry bleach, the resulting bleached laundry on dry-out in an atmosphere of 50% relative humidity does have a sweet, anisic and animalic aroma; whereas without the use of the mixture of diisoamylene epoxide and the compound having the structure: 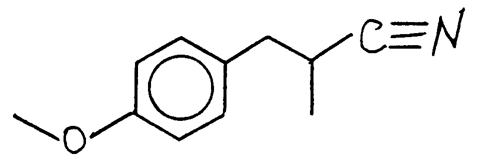
prepared according to Example XXXVIII, bulked fractions 10-18, the bleached laundry has a faint characteristic disagreeable "hypochlorite" aroma.

### EXAMPLE LXV

AROMOX® DMMC-W in various quantities is mixed with 0.1 gram of a 25:75 weight:weight mixture of diisoamylene epoxide:the compound having the structure: 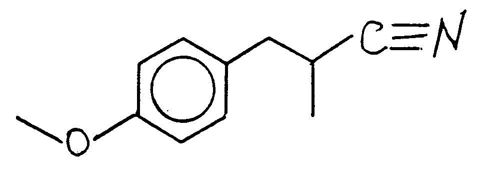
prepared according to Example XXXVIII, bulked fractions 10-18. The resulting premixes are then added to 200 grams of an aqueous 5% sodium hypochlorite solution. Sufficient 12.5 M aqueous NaOH is added to bring the pH of the mixture up to 13. The following results are obtained:

| Percentage AROMOX® DMMC-W | Clarity of Hypochlorite Solution After Addition of Premix |
|---|---|
| 0.23% | Clear after three days. |
| 0.15% | Clear after three days. |
| 0.08% | Initially slightly turbid; two phases exist after three days. |

When used as laundry bleaches, the resulting bleached laundries on dry-out in an atmosphere of 50% relative humidity in each of the three cases above but does have a sweet, anisic and animalic aroma" whereas without the use of the composition of matter set forth above containing diisoamylene epoxide and the compound having the structure: 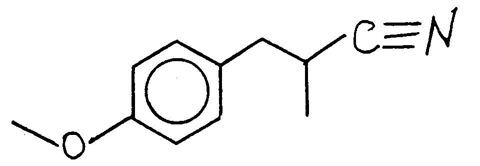
prepared according to Example XXXVIII, bulked fractions 10-18, the bleached laundry has the same characteristic disagreeable "hypochlorite" aroma.

### EXAMPLE LXVI

DOWFAX® 2A1 (see Note 1, infra) in various quantities, as set forth below, is mixed with 0.1 grams of a 50:50 mixture of (a) one of the diisoamylene epoxide compositions prepared according to Example II of application for United States Letters Patent 4,335,009 issued June 15, 1982 and (b) the compound having the structure: 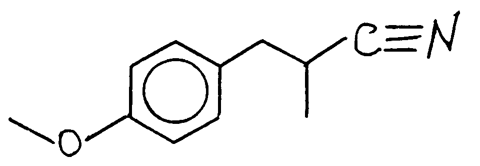
prepared according to Example XXXVIII, bulked fractions 10-18. The resulting premixes are then added to 200 grams of an aqueous 7% sodium hypochlorite solution. Sufficient 12.5 M aqueous sodium hydroxide is added to bring the pH of the mixture up to 13.5. The following results are obtained:

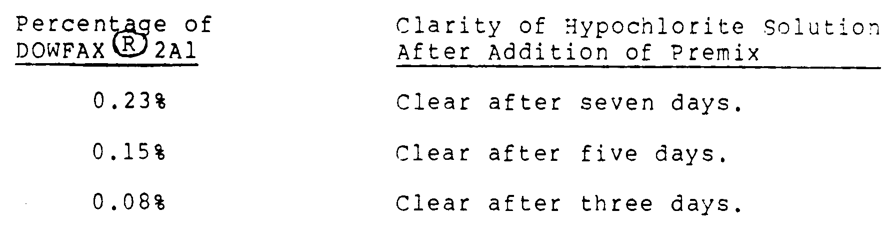

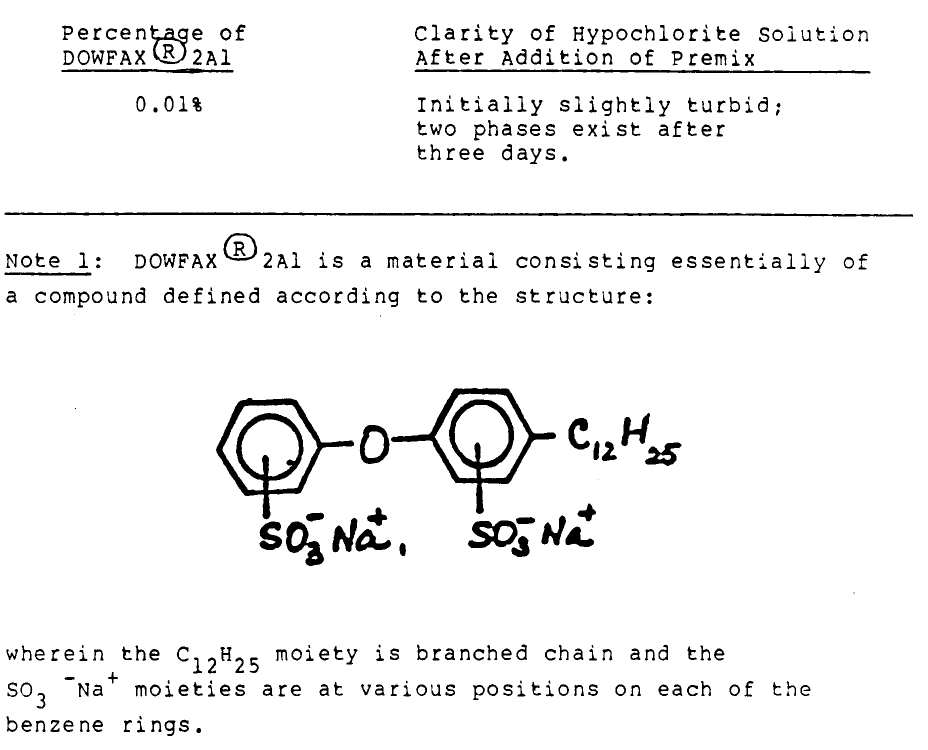

### EXAMPLE LXVII

DOWFAX® 3B2 (see Note 2, infra) in various quantities as set forth below, is mixed with 0.1 gram of the compound having the structure: 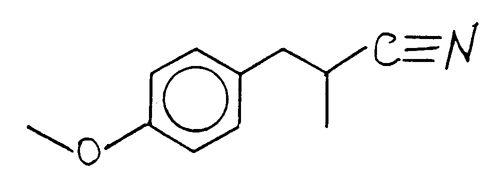
Note 2: DOWFAX® 3B2 is a compound essentially defined according to the structure: 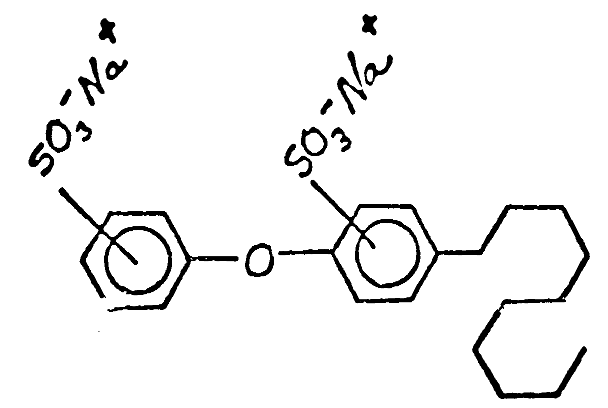
wherein the SO₃ ⁻Na⁺ moieties are at various positions on the phenyl moieties. DOWFAX® 3B2 is a registered trademark of the Dow Chemical Company of Midland, Michigan.

In the following examples, AROMOX® DMC-W and AROMOX® DMMC-W are 30% aqueous solutions of dimethyl cocoamine oxide; and AROMOX® NCMDW is a 40% aqueous solution of N-cocomorphloline oxide produced by Armac Division of Akzo of Chicago, Illinois prepared according to Example I(B). The resulting premixes are then added to 200 grams of an aqueous 7% sodium hypochlorite solution. Sufficient 12.5 M aqueous NaOH is added to bring the pH of the mixture up to 13.5. The following results are obtained:

| Percentage of DOWFAX® 3B2 | Clarity of Hypochlorite Solution After Addition of Premix |
|---|---|
| 0.23% | Clear after seven days. |
| 0.15% | Clear after five days. |
| 0.08% | Clear after three days. |
| 0.01% | Clear after three days. Initially slightly turbid; two phases exist after three days. |

When the 7% aqueous sodium hypochlorite solutions are used as laundry bleaches, the resulting laundry batches on dry-out in an atmosphere of 65% relative humidity yield substantially no characteristic "hypochlorite" odors but but does have a sweet, anisic and animalic aroma. Furthermore, no such characteristic "hypochlorite" aromas are retained on the hands of the individuals handling such laundry batches in both the wet and the dry states.

### EXAMPLE LXVIII

Four drops of a 25:75 weight/weight mixture of diisoamylene epoxide prepared according to Example II of application for United States Letters Patent 4,335,009 issued June 15, 1982 and the compound having the structure: 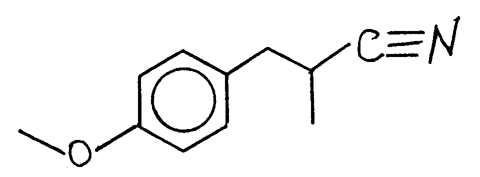
prepared according to Example XXXVIII, bulked fractions 10-18, supra, is added to 2 grams of DOWFAX® 3B2 and 0.5 grams of AROMOX® DMC-W to produce a clear premix. The clear premix is added to 200 grams of CLOROX® with stirring resulting in a clear stable single phase solution. Sufficient 1 M aqueous NaOH is added to bring the pH of the mixture of 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a sweet, anisic and animalic aroma. Furthermore, no such characteristic "hypochlorite" aroma is retained on the hands of the individual handling such laundry in both the wet and the dry states.

### EXAMPLE LXIX

One gram of DOWFAX® 3B2; one gram of DOWFAX® 2A1 and 0.25 grams of AROMOX® DMMC-W is admixed with eight drops of the compound having the structure: 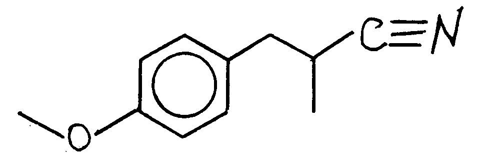
prepared according to Example XXXVIII, bulked fractions 10-18. This premix is then added, with stirring to 200 grams of a mixture containing 4.5% aqueous sodium hypochlorite and 4.5% aqueous lithium hypochlorite. Sufficient 4 M aqueous LiOH is added to bring the pH of the solution to 13.4. The mixture is then heated to 48.89°C (120°F) and maintained at that temperature for a period of one week. The resulting solution remains clear in a single phase. When used as a laundry bleach, the resulting bleached laundry on dry-out in an atmosphere of 50% relative humidity but does have a sweet, anisic and animalic aroma; whereas without the use of the compound having the structure: 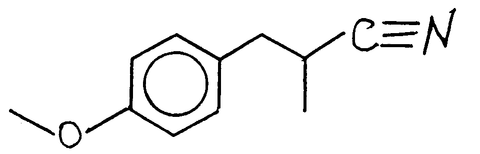
prepared according to Example XXXVIII, bulked fractions 10-18, supra, the bleached laundry has a faint characteristic disagreeable "hypochlorite" aroma.

### EXAMPLE LXX

One gram of DOWFAX® 2A1 and one gram of DOWFAX® 3B2 is admixed with eight drops of a 50:50 mixture of one of the diisoamylene epoxide compositions of Example II of application for United States Letters Patent 4,335,009 issued June 15, 1982 and the compound having the structure: 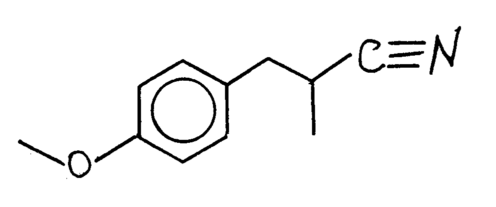
prepared according to Example XXXVIII, bulked fractions 10-18. This premix is then added, with stirring to 200 grams of a mixture containing 4.5% aqueous sodium hypochlorite. Sufficient 4 M aqueous LiOH is added to bring the pH of the solution to 13.4. The mixture is then heated to 48.89°C (120°F) and maintained at that temperature for a period of one week. The resulting solution remains clear in a single phase. When used as a laundry bleach, the resulting bleached laundry on dry-out in an atmosphere of 50% relative humidity but does have a sweet, anisic and animalic aroma; whereas without the use of the perfume composition which is a mixture of diisoamylene epoxide and the compound having the structure: 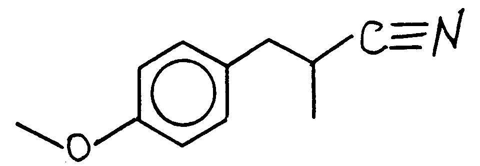
prepared according to Example XXXVIII, bulked fractions 10-18, the bleached laundry has a faint characteristic disagreeable "hypochlorite" aroma.

### EXAMPLE LXXI

1.5 Grams of DOWFAX® 2A1 is admixed with eight drops of the compound having the structure: 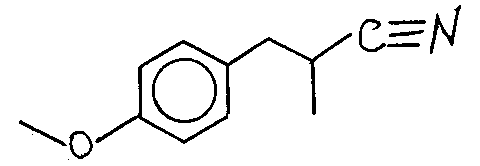
prepared according to Example XXXVIII, bulked fractions 10-18, supra. This premix is then added with stirring to 200 grams of a mixture containing 4.5% aqueous sodiumn hypochlorite and 4.5% aqueous lithium hypochlorite. Sufficient 2 M aqueous NaOH is added to bring the pH of the solution to 13.4. The mixture is then heated to 43.33°C (110°F) and maintained at that temperature with stirring for a period of two weeks. The resulting solution remains clear as a single phase when used as a laundry bleach. The resulting bleached laundry on dry-out in an atmosphere of 50% relative humidity does have a sweet, anisic and animalic aroma, whereas without the use of the the compound having the structure: 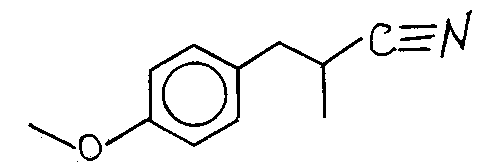
prepared according to Example XXXVIII, bulked fractions 10-18, the bleached laundry has a faint characteristic disagreeable "hypochlorite" aroma.

### EXAMPLE LXXII

Four drops of a 50:50 mixture of one of the diisoamylene epoxide mixtures produced according to Example II of application for United States Letters Patent 4,335,009 issued June 15, 1982 and the compound having the structure: 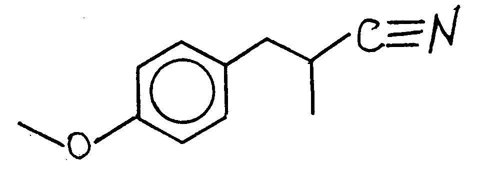
prepared according to Example XXXVIII, bulked fractions 10-18, supra, is added to 1.0 grams of DOWFAX® 3B2 and 0.25 grams of AROMOX® NCMD-W to produce a clear premix. The clear premix is added to 200 grams of CLOROX® resulting in a clear stable single phase solution. Sufficient 1 M aqueous NaOH is added to bring the pH of the mixture up to 12.8. The solution remains substantially stable at 48.89°C (120°F) for a period of seven days. When the 5% aqueous sodium hypochlorite solution is used as a laundry bleach, the resulting laundry on dry-out in an atmosphere of 65% relative humidity yields sustantially no characteristic "hypochlorite" odor, but does have a sweet, anisic and animalic aroma. Furthermore, no such characteristic "hypochlorite" aroma is retained on the hands of the individual handling such laundry in both the wet and the dry states.

### DETAILED DESCRIPTION OF THE DRAWINGS

Figure 1 is the GLC profile for the reaction product of Example I containing the compounds having the structures: 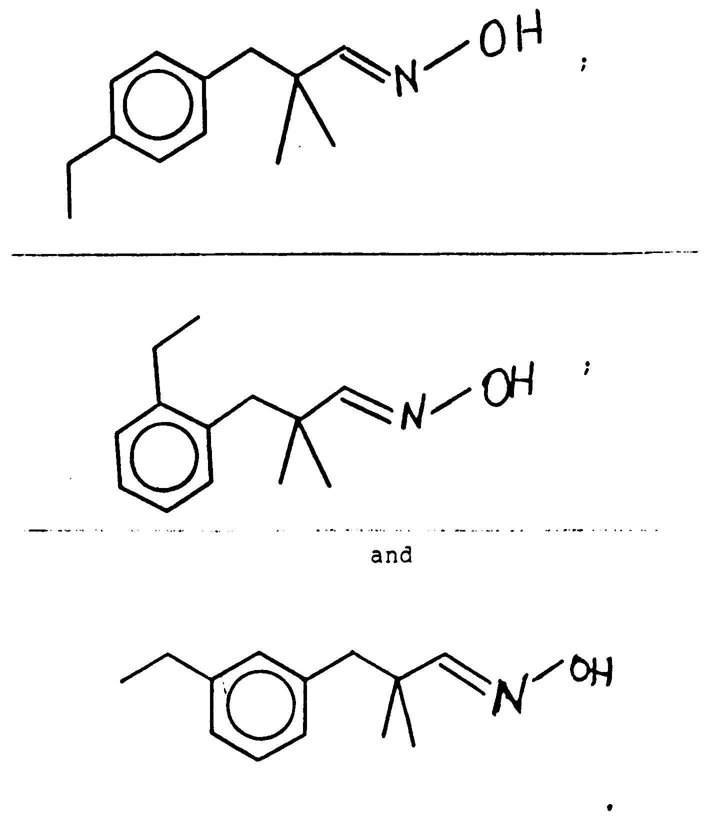
The peak indicated by reference number 10 is the peak for the mixture of compounds having the structures:

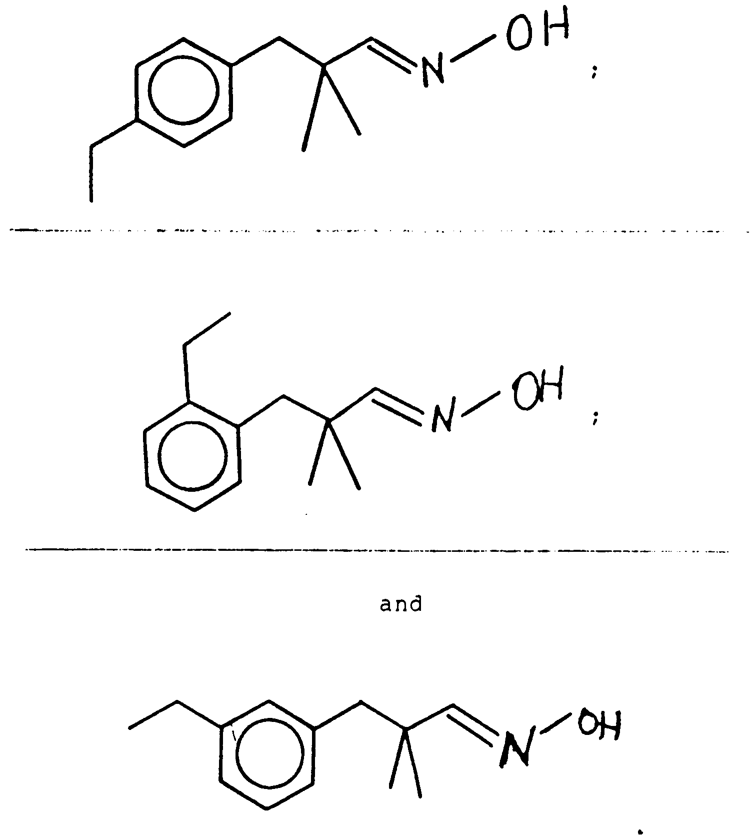

The peak indicated by reference numeral 12 is the peak for the compounds having the structures:

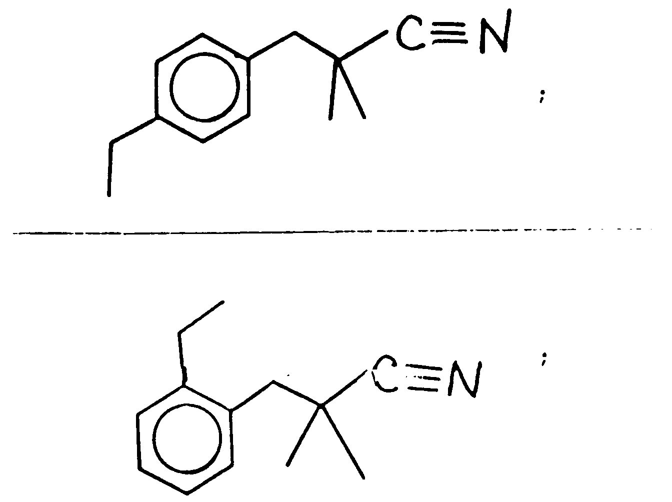

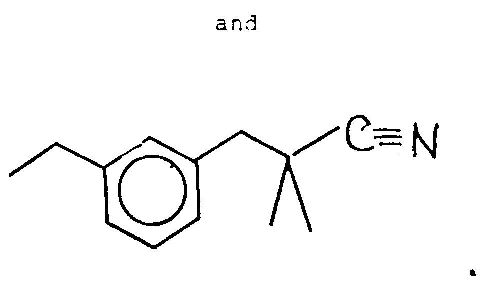

The peak indicated by reference numeral 14 is the peak for the mixture of compounds having the structures: 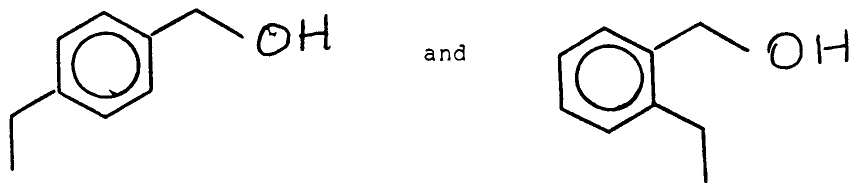
(Conditions: SF-30 column programmed at 220°C isothermal).

Figure 4 is the GLC profile for the reaction product of Example II containing the compounds having the structures:

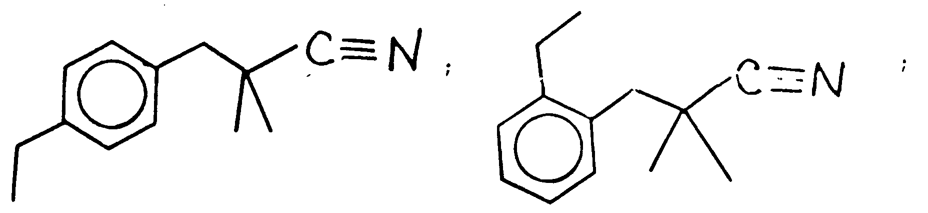

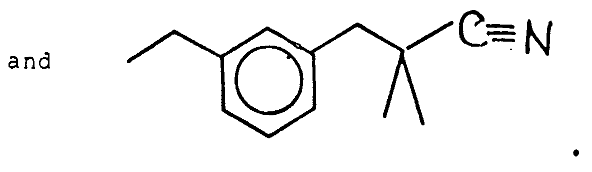

The peak indicated by reference numeral 41 is the peak for the reaction solvent, and reagent, acetic anhydride.

The peak indicated by reference numeral 42 is the peak for the reaction products having the structures: 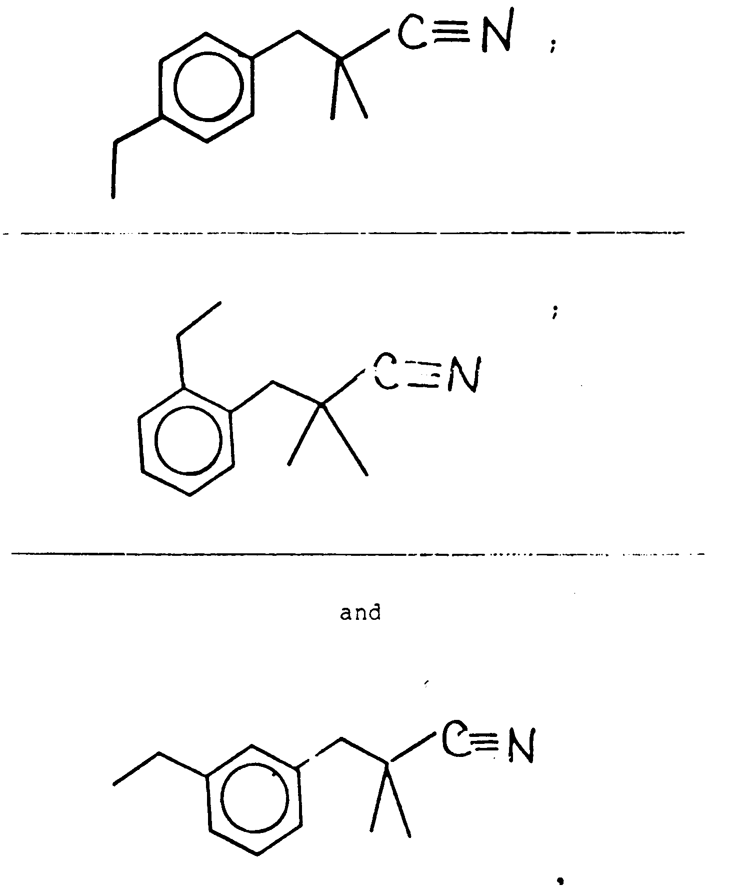
The peak indicated by reference numeral 44 is the peak for the reaction precursors having the structures: 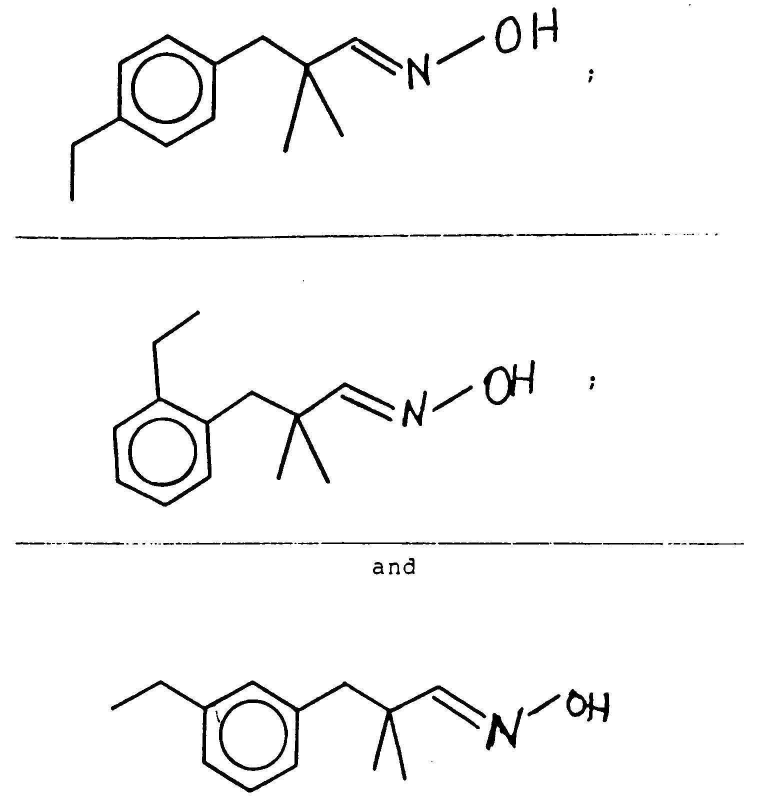
(Conditions: SE-30 column programmed at 220°C isothermal).

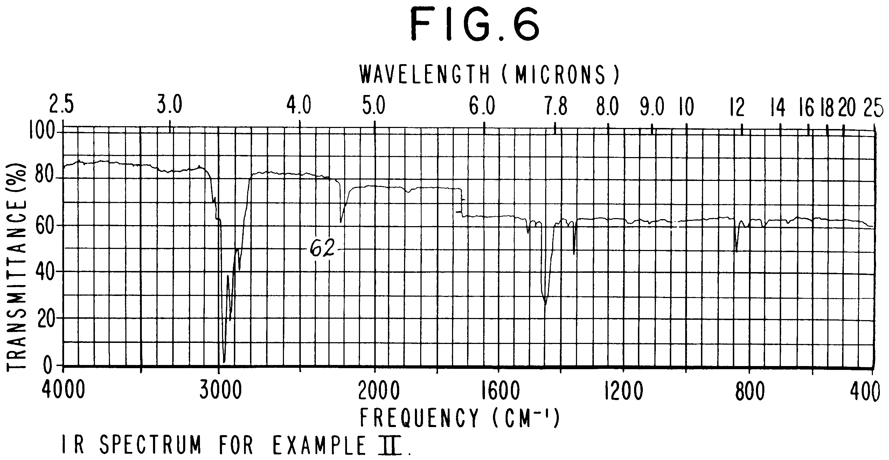

Figure 6 is the infra-red spectrum for the mixture of compounds having the structures: 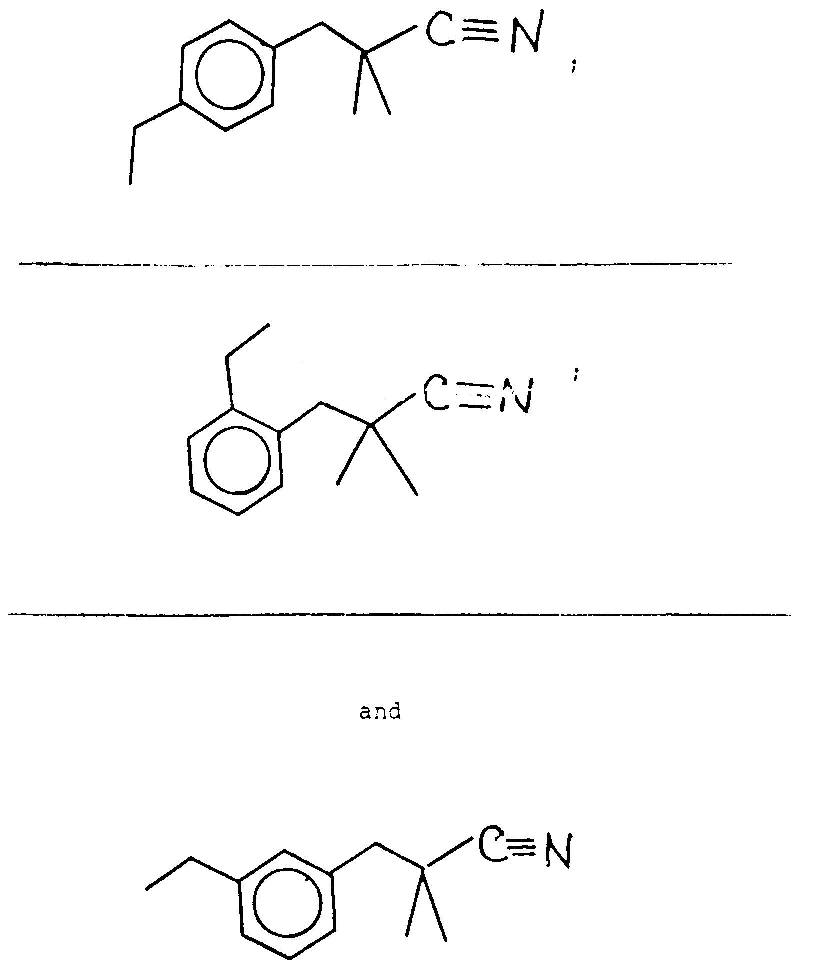
prepared according to Example II. The peak indicated by reference numeral 62 is the peak that signifies nitriles or the "cyanide" moiety having the structure:

⁅C≡N].

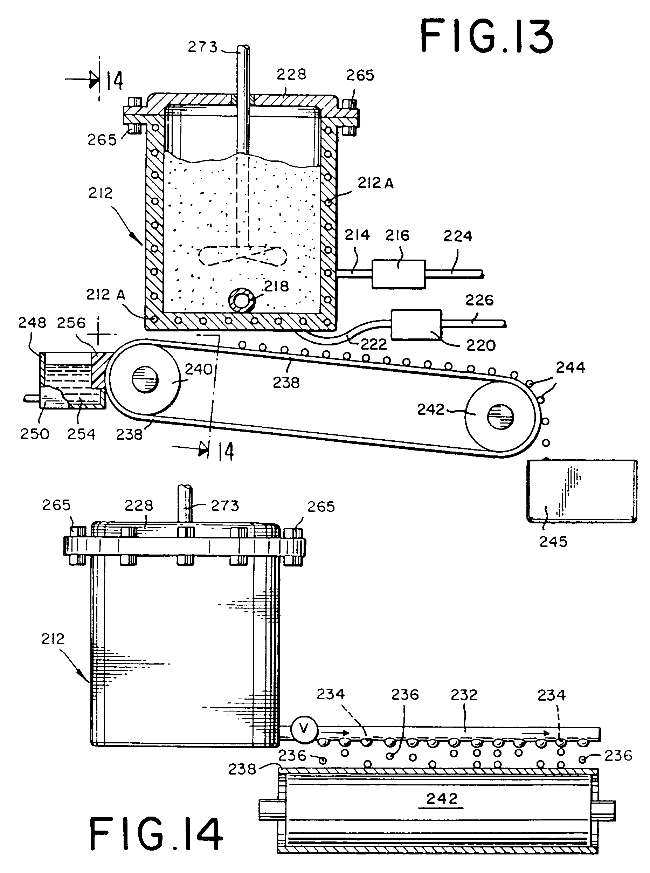

Referring to Figures 13 and 14, there is provided a process for forming scented polymer elements (wherein the polymer may be a thermoplastic polymer such as low density polyethylene or polypropylene or copolymers of ethylene and vinyl acetate or mixtures of polymers and copolymers such as copolymers of ethylene and vinyl acetate and polyethylene) such as pellets useful in the formation of plastic particles useful in fabricating certain articles which may be perfumed (and, further, which may be exposed to chlorine bleaches). This process comprises heating the polymer or mixture of polymers, e.g., 250°C in the case of low density polyethylene. The lower most portion of the container is maintained at at slightly lower temperature and the material in the container is taken off at such location for delivery through the conduit. Thus, referring to Figures 13 and 14 in particular, the apparatus used in producing such elements comprises a device for forming the polymer containing the perfume, e.g., polyethylene or polyethylene-polyvinyl acetate or mixtures of same or polypropylene, which comprises a vat or container 212 into which the polymer taken alone or in admixture with other copolymers and the perfuming substance which is at least one of the substituted propanes of our invention or mixtures of substituted propanes of our invention and other compatible perfumes is placed. The container is closed by means of an airtight lid 228 and clamped to the container by bolts 265. A stirrer 273 traverses the lid or cover 228 in an airtight manner and is rotatable in a suitable manner. A surrounding cylinder 212A having heating coils which are supplied with electric current through cable 214 from a rheostat or control 216 is operated to maintain the temperature inside the container 212 such that the polymer in the container will be maintained in the molten or liquid state. It has been found advantageous to employ polymers at such a temperature that the viscosity will be in the range of 90-100 sayboldt seconds. The heater 218 is operated to maintain the upper portion of the container 212 within a temperature range of, for example, 220-270°C in the case of low density polyethylene. The bottom portion of the container 212 is heated by means of heating coils 212A regulated through the control 220 connected thereto through a connecting wire 222 to maintain the lower portion of the container 212 within a temperature range of 220-270°C.

Thus, the polymer or mixture of polymers added to the container 212 is heated from 10-12 hours, whereafter the perfume composition or perfume material which contains one or more of the substituted propanes of our invention is quickly added to the melt. Generally, about 10-45 percent by weight of the resulting mixture of the perfumery substance is added to the polymer.

After the perfume material is added to the container 212, the mixture is stirred for a few minutes, for example, 5-15 minutes and maintained within the temperature ranges indicated previously by the heating coil 212A. The controls 216 and 220 are connected through cables 224 and 226 to a suitable supply of electric current for supplying the power for heating purposes.

Thereafter, the valve "V" is opened permitting the mass to flow outwardly through conduit 232 having a multiplicity of orifices 234 adjacent to the lower side thereof. The outer end of the conduit 232 is closed so that the liquid polymer in intimate admixture with one or more of the substituted propanes of our invention and one or more other substances, will continuously drop through the orifices 234 downwardly from the conduit 232. During this time, the temperature of the polymer intimately admixed with the perfumery substance in the container 212 is accurately controlled so that a temperature in the range of from about 240-250°C, for example, in the case of low density polyethylene) will exist in the conduit 232. The regulation of the temperature through the controls 216 and 220 is essential in order to insure the temperature balance to provide for the continuous dripping or dropping of molten polymer intimately admixed with the perfume substance which is all of or which contains one or more of the substituted propanes of our invention, through the orifices 234 at a rate which will insure the formation of droplets 236 which will fall downwardly onto a moving conveyor belt 238 caused to run between conveyor wheels 240 and 242 beneath the conduit 232.

When the droplets 236 fall onto the conveyor 238, they form pellets 244 which harden almost instantaneously and fall off the end of the conveyor 238 into a container 250 which is advantageously filled with water or some other suitable cooling liquid to insure the rapid cooling of each of the pellets 244. The pellets 244 are then collected from the container 250 and utilized for the formation of functional products, e.g., garbage bags and the like.

The features disclosed in the foregoing description, in the following claims and/or in the accompanying drawings may, both separately and in any combination thereof, be material for realising the invention in diverse forms thereof.

## Claims

1. A substituted propane defined according to the structure: 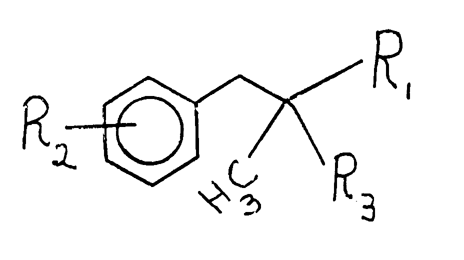 wherein R₁ is cyanide having the structure:
⁅C≡N]
or hydroxyimino methyl having the structure: 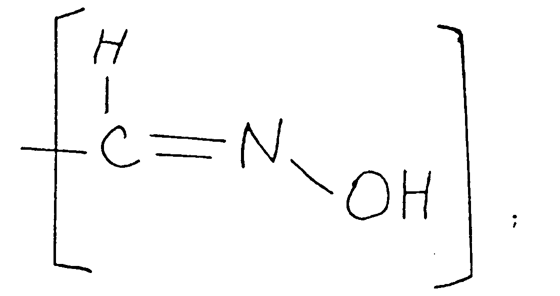 R₂ is ethyl or methoxy and R₃ is hydrogen or methyl, but wherein R₃ is not hydrogen when R₂ is a p-methoxy.

2. A process for augmenting or enhancing the aroma of a consumable material which is in the alternative a perfume composition, a cologne, a perfumed article or a bleach composition comprising the step of adding to said consumable material and aroma augmenting or enhancing quantity of at least one compound defined according to Claim 1.

3. At least one 2,2-dimethyl-1-nitrilo or hydroxyimino-3-(alkyl phenyl)-substituted propane defined according to the structure: 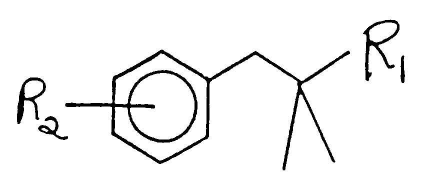 wherein R₁ is a nitrogen-containing moiety selected from the group consisting of:
(i) cyanide having the structure:
⁅C≡N]
and
(ii) hydroxyimino methyl having the structure: 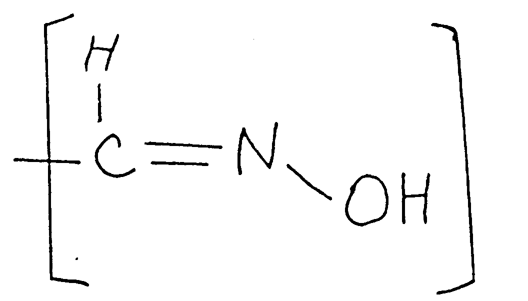
and wherein R₂ is C₁-C₅ lower alkyl

4. The 2,2-dimethyl-1-nitrilo or hydroxyimino-3-(alkyl phenyl)-substituted propane of Claim 3 defined according to the structure:

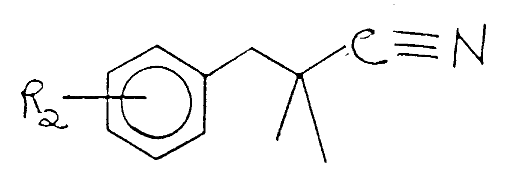

5. The 2,2-dimethyl-1-nitrilo or hydroxyimino-3-(alkyl phenyl)-substituted propane of Claim 3 defined according to the structure:

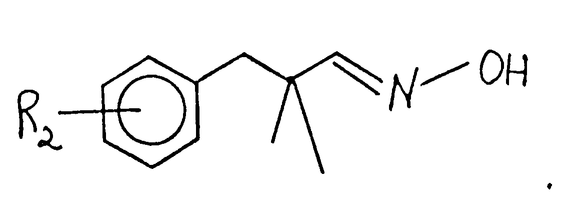

6. The 2,2-dimethyl-1-nitrilo or hydroxyimino-3-(alkyl phenyl)-substituted propane of Claim 3 having the structure:

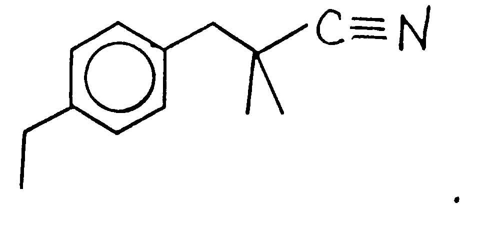

7. The 2,2-dimethyl-1-nitrilo or hydroxyimino-3-(alkyl phenyl)-substituted propane of Claim 3 having the structure:

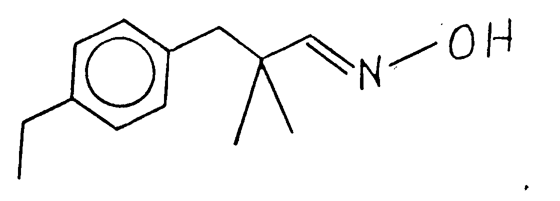

8. A mixture of 2,2-dimethyl-1-nitrilo or hydroxyimino-3-(alkyl phenyl)-substituted propanes defined according to Claim 3 having the structure:

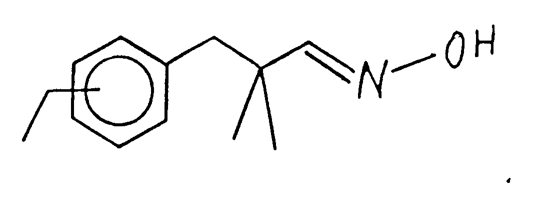

9. A mixture of 2,2-dimethyl-1-nitrilo or hydroxyimino-3-(alkyl phenyl)-substituted propanes defined according to Claim 3 having the structure:

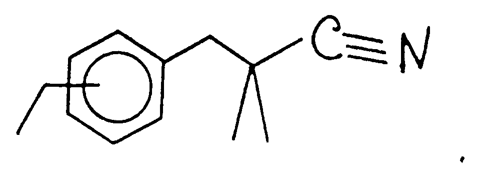

10. A mixture including a mixture of 2,2-dimethyl-1-nitrilo or hydroxyimino-3-(alkyl phenyl)-substituted propanes having the structure: 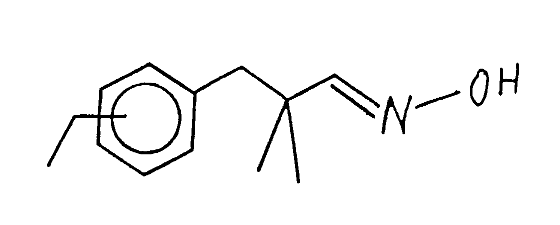 prepared according to the process comprising the steps of reacting a mixture of compounds having the structure: 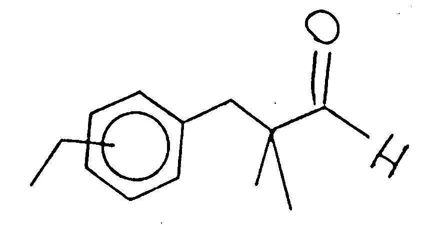 with a hydroxylamine salt having the structure: 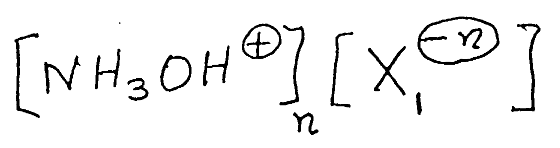 in the presence of base wherein n is an integer of from 1 up to 3 and x₁⁻ⁿ is an anion having a valence of -n.

11. A mixture of compounds including a mixture of 2,2-dimethyl-1-nitrilo or hydroxyimino-3-(alkyl phenyl)-substituted propanes having the structure: 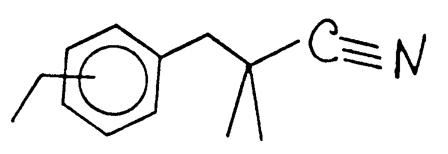 prepared according to the process of:
(i) first reacting a mixture of compounds having the structure: 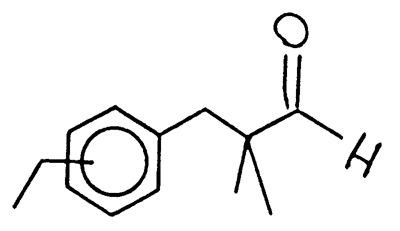 with a hydroxylamine salt having the structure: 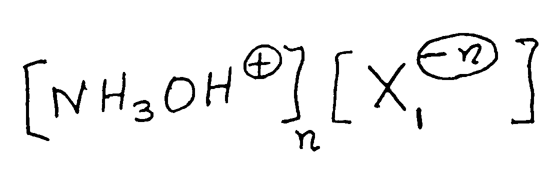 in the presence of base whereby a mixture of compounds containing the mixture defined according to the structure: 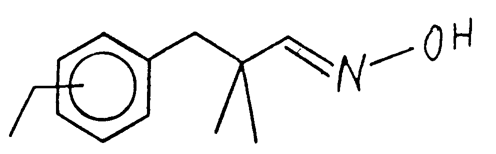 is obtained;
and then
(ii) reacting the mixture containing the mixture of compounds having the structure: 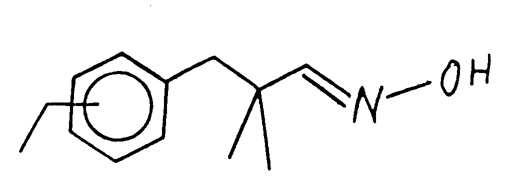 with a dehydrating agent to produce a mixture of compounds containing the mixture defined according to the structure:

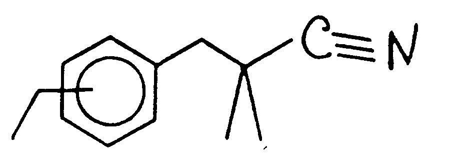

12. A chlorine-containing bleach composition comprising:
(a) a chlorine bleach base; and
(b) intimately admixed therewith at least one compound defined according to Claim 3.

13. A 2-methyl-1-hydroxyimino-3-(methoxyphenyl)-substituted propane having the structure:

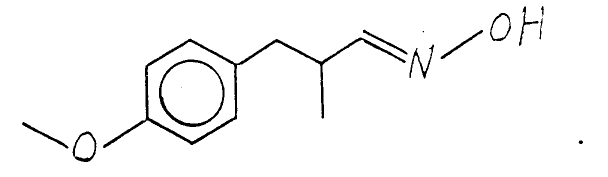

14. A process for augmenting or enhancing the aroma of a consumable material selected from the group consisting of perfume compositions, perfumed articles and perfumed polymers comprising the step of intimately admixing with said consumable material an aroma augmenting or enhancing quantity of the compound defined according to Claim 13.

15. A chlorine-containing bleach composition comprising:
(a) a chlorine bleach base; and
(b) intimately admixed therewith the compound defined according to Claim 13

16. A mixture of nitriles having the structures :

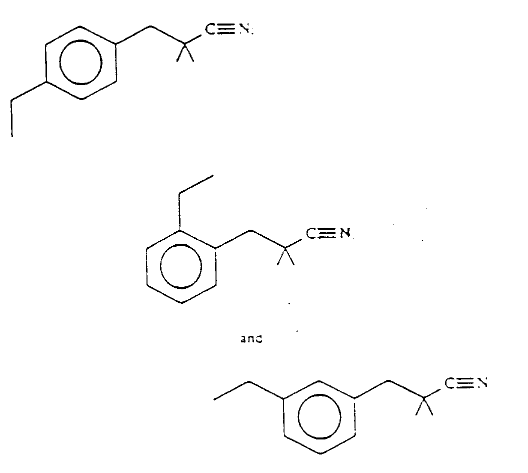

17. A mixture of oximes having the structures :

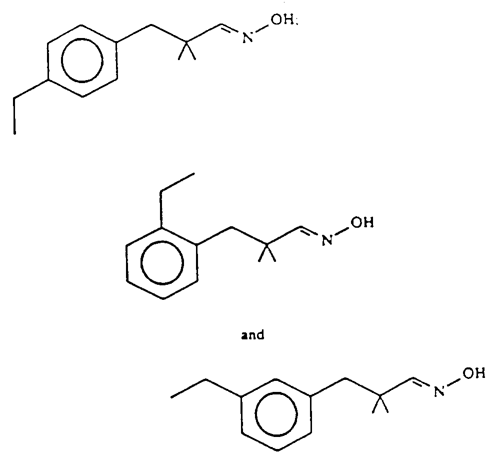

18. A process for augmenting or enhancing the aroma of a consumable material selected from the group consisting of perfume compositions, perfumed articles and perfumed polymers comprising the step of intimately admixing with said consumable material an aroma augmenting or enhancing quantity of at least one compound defined according to Claim 16 or 17.

19. A chlorine-containing bleach composition comprising:
(a) a chlorine bleach base; and
(b) intimately admixed therewith at least one compound defined according to Claim 16 or 17.

## Patentansprüche

1. Substituiertes Propan der folgenden Formel: 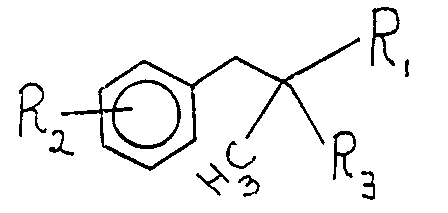 worin R₁ für einen Cyanidrest der folgenden Formel:
⁅C≡N]
oder einen Hydroxyiminomethylrest folgender Formel: 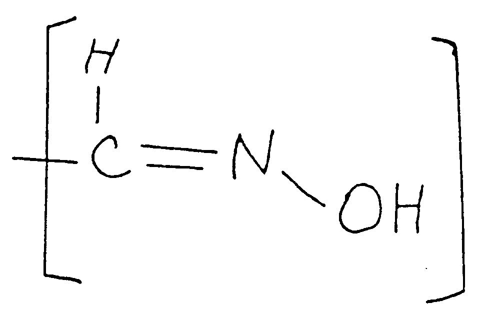 steht,
R₂ für einen Ethyl- oder Methoxyrest steht und R₃ für einen Wasserstoff- oder Methylrest steht, wobei jedoch R₃ nicht für Wasserstoff steht, wenn R₂ für p-Methoxy steht.

2. Verfahren zur Steigerung oder Verstärkung des Aromas eines verbrauchbaren Materials, bei dem es sich entweder um eine Parfumzusammensetzung, ein Eau de Cologne, einen parfümierten Artikel oder eine Bleichzusammensetzung handelt, worin eine das Aroma erhöhende oder verstärkende Menge mindestens einer im Anspruch 1 definierten Verbindung zu diesem verbrauchbaren Material hinzugegeben wird.

3. Mindestens ein 2,2-Dimethyl-1-nitrilo- oder Hydroxyimino-3-(alkylphenyl)-substituiertes Propan mit folgender Formel: 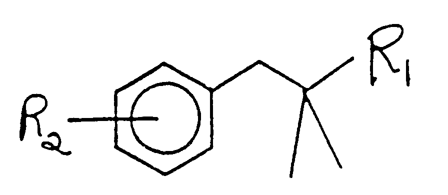 worin R₁ eine stickstoffhaltige Einheit bedeutet, die ausgewählt ist aus der Gruppe bestehend aus:
(i) einem Cyanidrest mit folgender Formel:
⁅C≡N]
und
(ii) einem Hydroxyiminomethylrest mit folgender Formel: 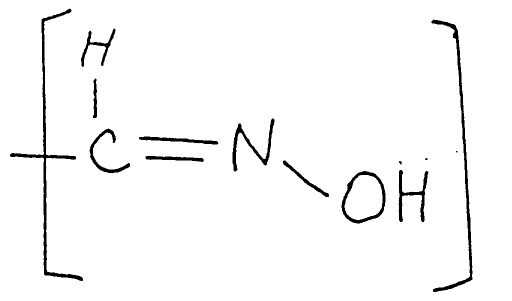 und worin R₂ einen C₁-C₅ Niedrigalkylrest bedeutet.

4. 2,2-Dimethyl-1-nitrilo- oder Hydroxyimino-3-(alkylphenyl)-substituiertes Propan nach Anspruch 3 mit folgender Formel:

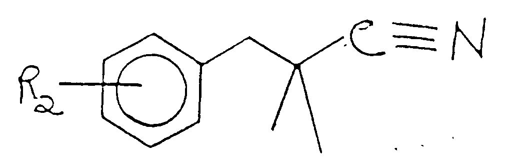

5. 2,2-Dimethyl-1-nitrilo- oder Hydroxyimino-3-(alkylphenyl)-substituiertes Propan nach Anspruch 3 mit folgender Formel:

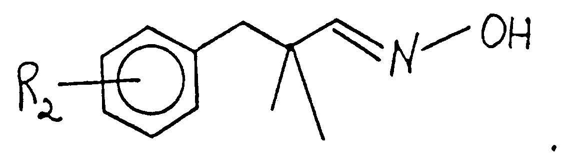

6. 2,2-Dimethyl-1-nitrilo- oder Hydroxyimino-3-(alkylphenyl)-substituiertes Propan nach Anspruch 1 mit folgender Formel:

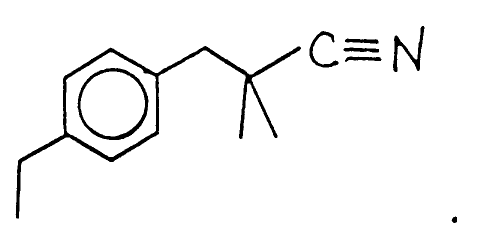

7. 2,2-Dimethyl-1-nitrilo- oder Hydroxyimino-3-(alkylphenyl)-substituiertes Propan nach Anspruch 3 mit folgender Formel:

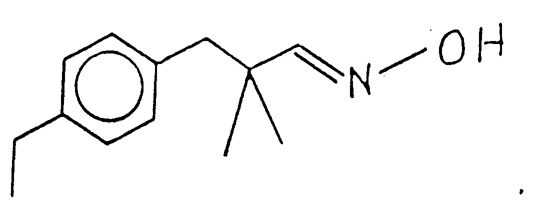

8. Gemisch aus 2,2-Dimethyl-1-nitrilo- oder Hydroxyimino-3-(alkylphenyl)-substituierten Propanen nach Anspruch 3 mit folgender Formel:

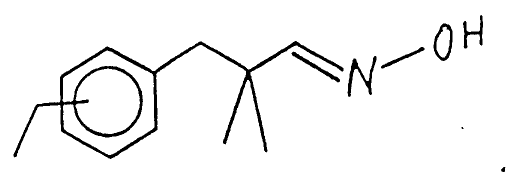

9. Gemisch aus 2,2-Dimethyl-1-nitrilo- oder Hydroxyimino-3-(alkylphenyl)-substituierten Propanen nach Anspruch 3 mit folgender Formel:

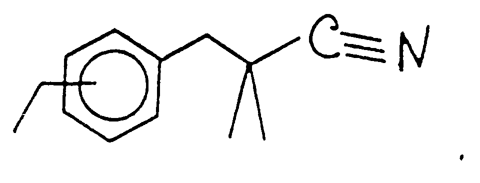

10. Gemisch einschließend ein Gemisch aus 2,2-Dimethyl-1-nitrilo- oder Hydroxylamino-3-(alkylphenyl)-substituierten Propanen mit folgender Formel: 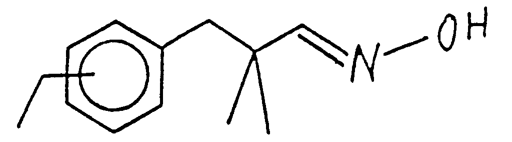 hergestellt gemäß dem Verfahren, worin ein Gemisch von Verbindungen mit folgender Formel: 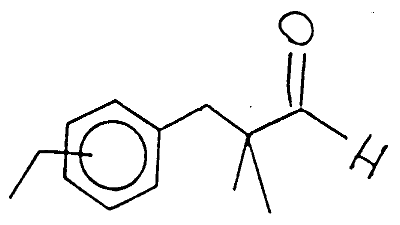 mit einem Hydroxylaminsalz folgender Formel: 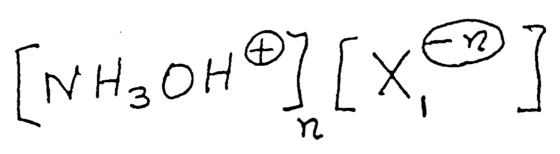 worin n für eine ganze Zahl von 1 bis 3 und x₁⁻ⁿ für ein Anion mit einer Valence von -n steht, in Anwesenheit einer Base umsetzt.

11. Gemisch aus Verbindungen einschließlich eines Gemisch aus 2,2-Dimethyl-1-nitrilo- oder Hydroxyimino-3-(alkylphenyl)-substituierten Propanen mit der folgenden Formel: 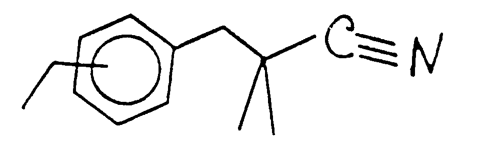 hergestellt gemäß dem Verfahren worin
(i) ein Gemisch aus Verbindungen mit der Formel: 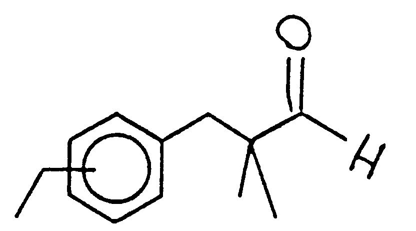 mit einem Hydroxylaminsalz folgender Formel: 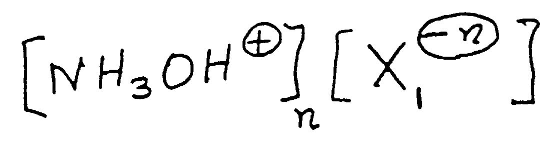 in Anwesenheit einer Base umgesetzt wird, wobei ein Gemisch von Verbindungen erhalten wird, die das Gemisch mit folgender Formel: 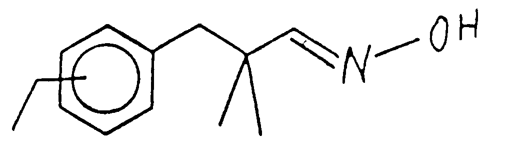 enthält,
und dann
(ii) das Gemisch, welches das Gemisch aus Verbindungen mit der folgenden Formel: 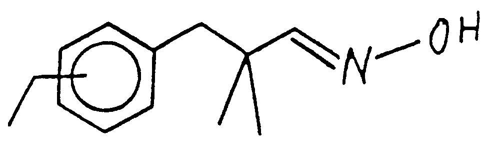 enthält, mit einem Dehydratisierungsmittel zu einem Gemisch aus Verbindungen umsetzt, das das Gemisch mit folgender Formel: 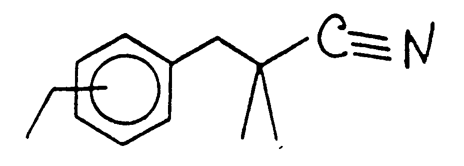 enthält.

12. Chlorhaltige Bleichzusammensetzung enthaltend:
(a) eine Chlorbleichbase und
(b) mindestens eine gründlich damit vermischte Verbindung nach Anspruch 3.

13. 2-Methyl-1-nitrilo- oder Hydroxyimino-3-(methoxyphenyl)-substituiertes Propan mit folgender Formel:

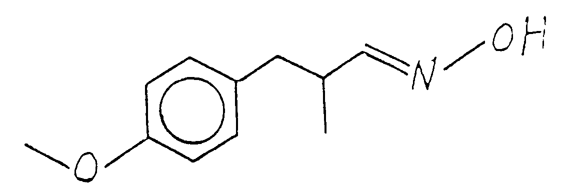

14. Verfahren zur Erhöhung oder Steigerung des Aromas eines verbrauchbaren Materials, das ausgewählt ist aus der Gruppe bestehend aus Parfumzusammensetzungen, parfümierten Artikeln und parfümierten Polymeren, worin das verbrauchbare Material mit einer das Aroma steigernden oder verstärkenden Menge der Verbindung nach Anspruch 13 gründlich vermischt wird.

15. Chlorhaltige Bleichzusammensetzung enthaltend:
(a) eine Chlorbleichbase und
(b) die gründlich damit vermischte Verbindung nach Anspruch 13.

16. Gemisch von Nitrilen mit den Formeln:

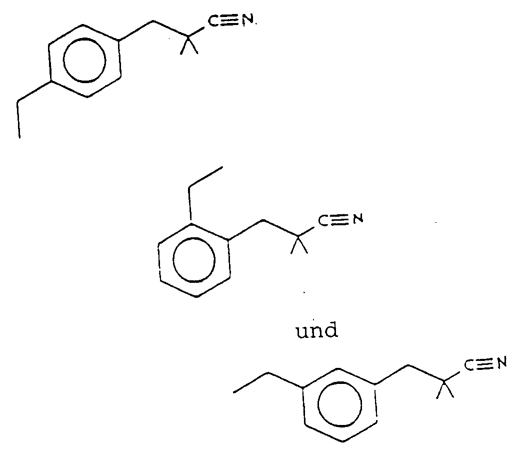

17. Gemisch von Oximen mit den Formeln:

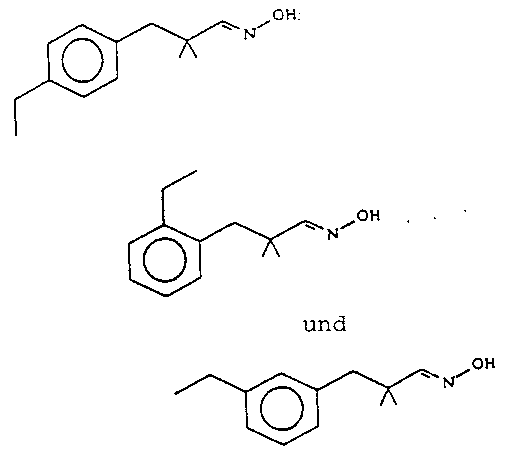

18. Verfahren zur Erhöhung oder Steigerung des Aromas eines verbrauchbaren Materials, das ausgewählt ist aus der Gruppe bestehend aus Parfumzusammensetzungen, parfümierten Artikeln und parfümierten Polymeren, worin das verbrauchbare Material mit einer das Aroma steigernden oder verstärkenden Menge mindestens einer Verbindung nach Anspruch 16 oder Anspruch 17 gründlich vermischt wird.

19. Chlorhaltige Bleichzusammensetzung enthaltend
(a) eine Chlorbleichbase und
(b) mindestens eine gründlich damit vermischte Verbindung nach Anspruch 16 oder 17.

## Revendications

1. Propane substitué défini par la structure suivante: 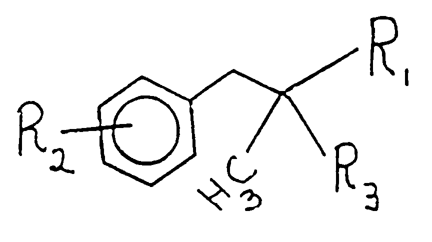 dans laquelle R₁ est un cyanure ayant la structure suivante :
⁅C≡N]
ou un radical hydroxyiminométhyle ayant la structure suivante : 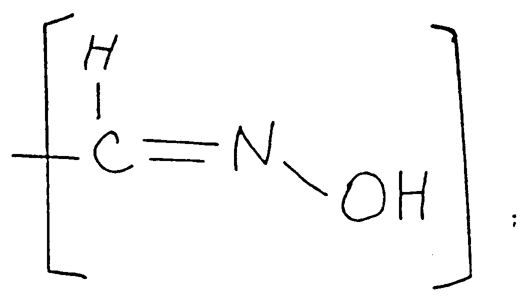 R₂ est le radical éthyle ou méthoxy, et R₃ est un hydrogène ou le radical méthyle, mais où R₃ n'est pas un hydrogène quand R₂ est le radical p-méthoxy.

2. Procédé pour renforcer ou augmenter l'arôme d'une matière consommable, qui peut être au choix une composition de parfum, une eau de Cologne, un article parfumé ou une composition de blanchiment, qui comprend l'étape consistant à ajouter à ladite matière consommable une quantité renforçant ou augmentant l'arôme d'au moins un composé selon la revendication 1.

3. Au moins un propane à substitution 2,2-diméthyl-1-nitrilo ou hydroxyimino-3-(alkylphényle) ayant la structure suivante : 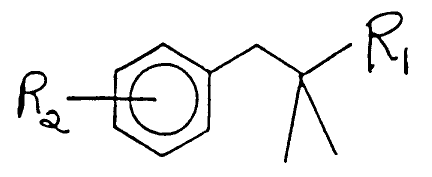 dans laquelle R₁ est un fragment azoté choisi parmi l'ensemble comprenant :
(i) le radical cyanure ayant la structure suivante :
⁅C≡N]
et
(ii) le radical hydroxyiminométhyle, ayant la structure suivante : 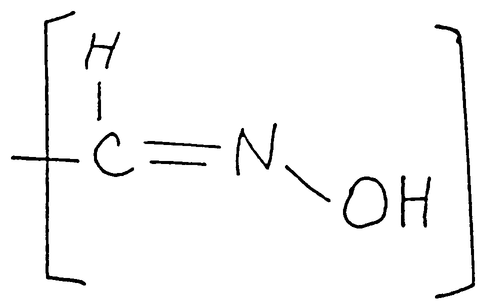
et dans laquelle R₂ est un radical alkyle inférieur en C₁-C₅.

4. Propane à substitution 2,2-diméthyl-1-nitrilo ou hydroxyimino-3-(alkylphényle) selon la revendication 3, ayant la structure suivante :

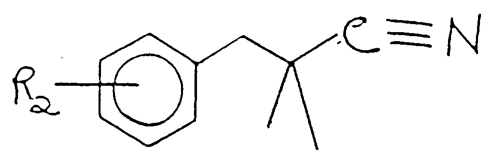

5. Propane à substitution 2,2-diméthyl-1-nitrilo ou hydroxyimino-3-(alkylphényle) selon la revendication 3, ayant la structure suivante :

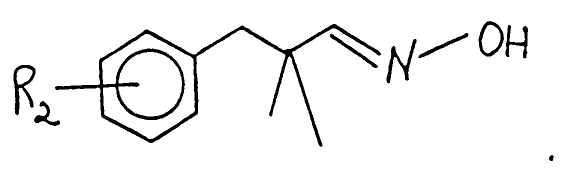

6. Propane à substitution 2,2-diméthyl-1-nitrilo ou hydroxyimino-3-(alkylphényle) selon la revendication 3, ayant la structure suivante :

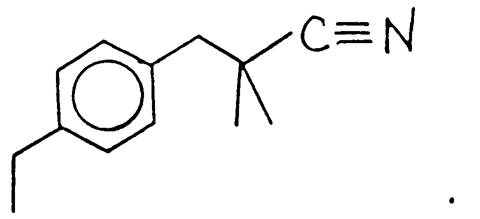

7. Propane à substitution 2,2-diméthyl-1-nitrilo ou hydroxyimino-3-(alkylphényle) selon la revendication 3, ayant la structure suivante :

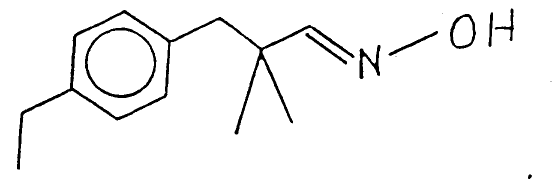

8. Mélange de propanes à substitution 2,2-diméthyl-1-nitrilo ou hydroxyimino-3-(alkylphényle) selon la revendication 3, ayant la structure suivante :

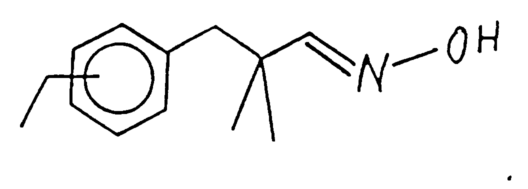

9. Mélange de propanes à substitution 2,2-diméthyl-1-nitrilo ou hydroxyimino-3-(alkylphényle) selon la revendication 3, ayant la structure suivante :

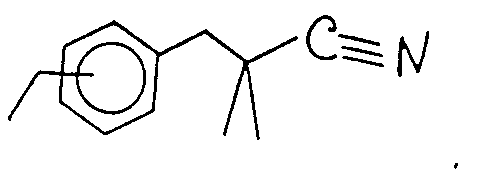

10. Mélange comprenant un mélange de propanes à substitution 2,2-diméthyl-1-nitrilo ou hydroxyimino-3-(alkylphényle) ayant la structure suivante : 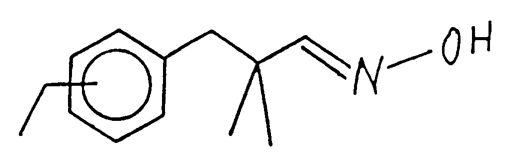 préparé par le procédé qui comprend les étapes consistant à faire réagir un mélange de composés ayant la structure suivante : 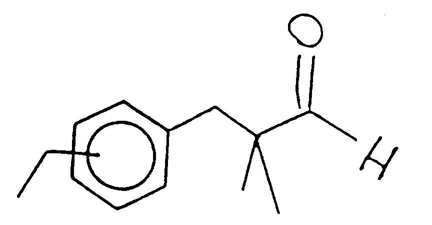 avec un sel d'hydroxylamine ayant la structure suivante : 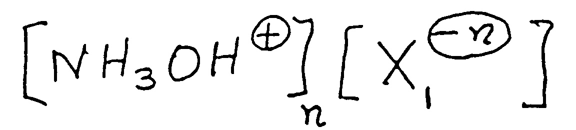 en présence d'une base, où n est un entier de 1 à 3, et X₁⁻ⁿ est un anion ayant la valence -n.

11. Mélange de composés comprenant un mélange de propanes à substitution 2,2-diméthyl-1-nitrilo ou hydroxyimino-3-(alkylphényle) ayant la structure suivante: 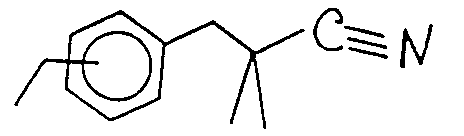 préparé par le procédé consistant :
(i) à faire d'abord réagir un mélange de composés ayant la structure suivante : 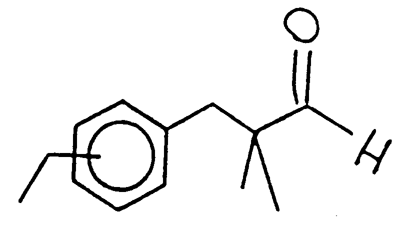 avec un sel d'hydroxylamine ayant la structure suivante : 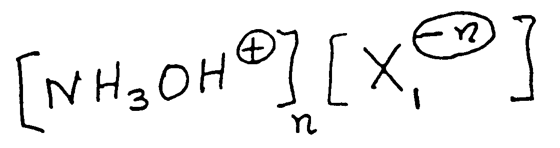 en présence d'une base, de façon à obtenir un mélange de composés contenant le mélange ayant la structure suivante: 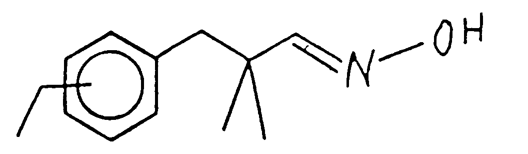 puis
(ii) à faire réagir le mélange contenant le mélange de composés ayant la structure suivante : 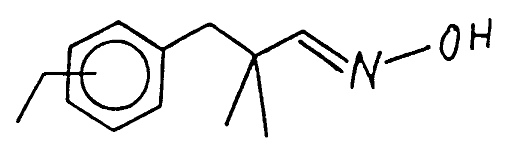 avec un agent de déshydratation, pour produire un mélange de composés contenant le mélange ayant la structure suivante :

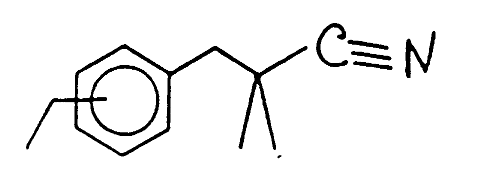

12. Composition de blanchiment chlorée comprenant :
(a) une base de blanchiment au chlore ; et
(b) en mélange intime avec cette dernière, au moins un composé selon la revendication 3.

13. Propane à substitution 2-méthyl-1-hydroxyimino-3-(alkylphényle) ayant la structure suivante :

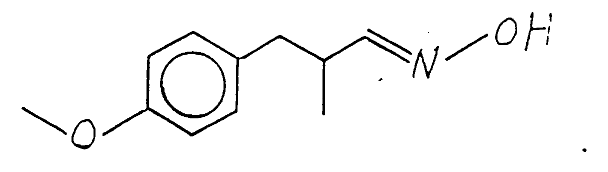

14. Procédé pour renforcer ou augmenter l'arôme d'une matière consommable choisie parmi l'ensemble comprenant les compositions de parfum, les articles parfumés et les polymères parfumés, qui comprend l'étape consistant à mélanger intimement à ladite matière consommable une quantité renforçant ou augmentant l'arôme du composé selon la revendication 13.

15. Composition de blanchiment chlorée comprenant :
(a) une base de blanchiment au chlore ; et
(b) en mélange intime avec cette dernière, au moins un composé selon la revendication 13.

16. Mélange de nitriles ayant les structures suivantes :

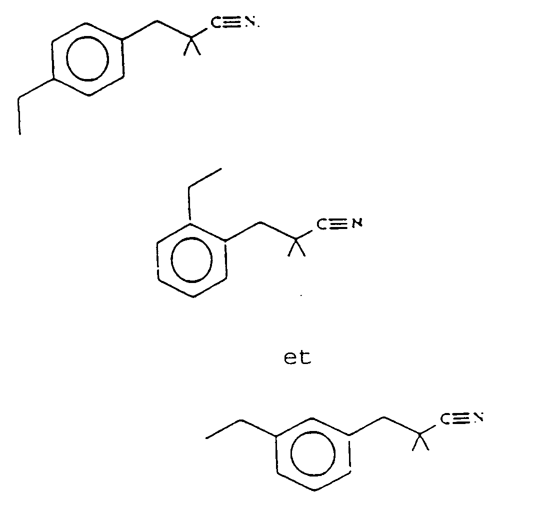

17. Mélange d'oximes ayant les structures suivantes:

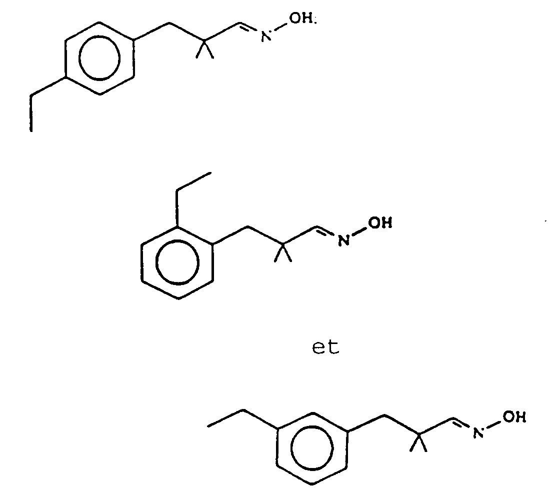

18. Procédé pour renforcer ou augmenter l'arôme d'une matière consommable, choisie parmi l'ensemble comprenant les compositions de parfum, les articles parfumés et les polymères parfumés, qui comprend l'étape consistant à mélanger intimement à ladite matière consommable une quantité renforçant ou augmentant l'arôme d'au moins un composé selon la revendication 16 ou 17.

19. Composition de blanchiment chlorée comprenant :
(a) une base de blanchiment au chlore ; et
(b) en mélange intime avec cette dernière, au moins un composé selon la revendication 16 ou 17.
